# EUROPEAN PATENT SPECIFICATION

(11) **EP 1 314 779 B1**
(45) Date of publication and mention of the grant of the patent: **03.10.2007**
(21) Application number: 01958544.7
(22) Date of filing: 28.08.2001
(51) Int. Cl.: C12N 9/76, C12N 15/57, C12Q 1/37, C12N 5/10, C12Q 1/68, C07K 16/40, G01N 33/50, G01N 33/15

(54) **AIRWAY-SPECIFIC TRYPSIN-LIKE ENZYMES AND METHOD OF USING THE SAME**
ATEMWEGSPEZIFISCHE, TRYPSIN-ÄHNLICHE ENZYME UND VERFAHREN ZU DEREN ANWENDUNG
ENZYMES DE TYPE TRYPSINE PROPRES AUX VOIES RESPIRATOIRES ET PROCEDE LES UTILISANT

(30) Priority: 28.08.2000 JP 2000257104; 05.03.2001 JP 2001059753
(43) Date of publication of application: 28.05.2003
(73) Proprietor: TEIJIN LIMITED, Osaka-shi Osaka 541-0054 (JP)
(72) Inventor: EGUCHI, Hiroshi, c/o Teijin Limited, Hino-shi, Tokyo 191-0065 (JP); CHOKKI, Manabu, c/o Teijin Limited, Hino-shi, Tokyo 191-0065 (JP); YAMAMURA, Satoshi, c/o Teijin Limited, Hino-shi, Tokyo 191-0065 (JP); MITA, Reiko, c/o Teijin Limited, Hino-shi, Tokyo 191-0065 (JP); MASEGI, Tsukio, c/o Teijin Limited, Hino-shi, Tokyo 191-0065 (JP)
(74) Representative: Hallybone, Huw George
(86) International application number: PCT/JP2001/007349
(87) International publication number: WO 2002/018562

(56) References cited:
- EP-A- 1 043 406
- EP-A- 1 152 057
- EP-A1- 0 699 763
- WO-A-00/60077
- WO-A2-99/38973
- LEYTUS ET AL: "A novel trypsin-like serine protease (hepsin) with a putative transmembrane domain expressed by human iver and hepatoma cells" BIOCHEMISTRY, AMERICAN CHEMICAL SOCIETY. EASTON, PA, US, vol. 27, 1988, pages 1067-1074, XP002080091 ISSN: 0006-2960
- KITAMOTO Y ET AL: "CDNA SEUQENCE AND CHROMOSOMAL LOCALIZATION OF HUMAN ENTEROKINASE, THE PROTEOLYTIC ACTIVATOR OF TRYPSINOGEN" BIOCHEMISTRY, AMERICAN CHEMICAL SOCIETY. EASTON, PA, US, vol. 34, 1995, pages 4562-4568, XP000917516 ISSN: 0006-2960
- CHOKKI MANABU ET AL: "Human airway trypsin-like protease increases mucin gene expression in airway epithelial cells" AMERICAN JOURNAL OF RESPIRATORY CELL AND MOLECULAR BIOLOGY, vol. 30, no. 4, April 2004 (2004-04), pages 470-478, XP009041531 ISSN: 1044-1549
- KAZUYOSHI YAMAOKA ET AL.: 'Cloning and characterization of cDNA for human airway trypsin-like protease' THE JOURNAL OF BIOLOGICAL CHEMISTRY vol. 273, no. 19, 08 May 1998, pages 11895 - 11901, XP002948288

## Description

### Technical Field

The present invention relates to a method for detecting the inhibitory activity of a compound or a polypeptide (including proteins and antibodies), using a system for detecting or assaying the mucus secretion-promoting action, inflammation-eliciting action, intracellular calcium inflow-eliciting action and protease activated receptor-activating action of said enzyme, involving airway-specific trypsin-like enzyme proteins whose structures are newly elucidated.

### Background Art

In recent years, a human airway trypsin-like enzyme (hereinafter referred to as "airway-specific trypsin-like enzyme" or "AST"(Airway Specific Trypsin-like Protease) ) has been purified from the sputum of a human chronic airway inflammation patient (JP-A 7-067640 (hereinafter, JP-A means "Japanese Unexamined Patent Publication"),Yasuoka S. et al., Am. J. Respir. Cell Mol. Biol., 16: p300-308, 1997), its amino acid sequence and cDNA sequence have been elucidated (JP-A 8-89246, US-5804410, EP-699763, Yamaoka K. et al., J. Biol. Chem., 273 (19); 11895-11901, 1998).

Several activities of the enzyme have been examined in vitro. It is estimated that the enzyme may participate in the disease states of airway inflammation, because the enzyme participates in mucous ciliary movements and further has an action to reinforce the production of cytokines, such as IL-8, GM-CSF, from human bronchial epithelial cell strains (Toshiko Terao, et al., 1998 The Japanese Respiratory Society). Further, since the enzyme has enzymatic activities such as a fibrinogen-degrading activity (Yoshinaga S. et al., J. Med. Invest., 45: 77-86, 1998), and a plasminogen activator (prourokinase)-activating action (Sumiko Yoshinaga, et al., 1998 Conference on Proteases and Inhibitors in Pathophysiology and Therapeutics), it is estimated that the enzyme may anti-inflammatorily act through the formation of fibrin on the basal surface of an airway, modify the morbit state of a chronic airway patient, and further participate in metastasis and the like.

On the other hand, a protease-activated receptor (Protease Activated Receptor, hereinafter referred to as "PAR") defined as a receptor whose signal transduction is promoted by the activity of the protease is a receptor mediating the signals by the protease, and a thrombin receptor (human PAR-1) existing in human platelets has been cloned in 1991 (Vu U K-H et al., Cell, 64: 1057-1068, 1991). Subsequently, mouse PAR-1 (Coughlin, S. R., Unpublished, Accession No. L03529, 1992), rat PAR-1 (Runge M. S. et al., J. Biol. Chem., 267: 16975-16979, 1992), mouse PAR-2 (Nystedt S. et al., Proc Natl Acad Sci USA, 91: 9208-9212, 1994), human PAR-2 (Bohm S. K., Biochem. J., 314: 1009-1016, 1996), rat PAR-2 (Saifeddine M. et al., Br. J. Pharmacol., 118(3): 521-530, 1996), human and mouse PAR-3 (Ishihara H. et al., Nature, 386: 502-506, 1997), mouse PAR-4 (Kahn M. L. et al., Nature, 394: 690-694, 1998), human PAR-4 (Xu W-F et al., Proc Natl Acad Sci USA, 95: 6642-6646, 1998), and the like, have been cloned. Many reports that PAR-1 and PAR-2 participate in various pathologic states such as inflammation have been made (Dery O. et al., Am. J. Physiol., 274: C1429-C1452, 1998).

In relation to the activation of the PAR-2, it has been reported that trypsin (Nystedt S. et al., Proc Natl Acad Sci USA, 91, 9208-9212, 1994) and tryptase (Schwartz, L. B. et al., J. Immunol., 126: 1290-1294, 1981) activate the PAR-2 (Molino M. et al., J. Biol. Chem., 272: 4043-4049, 1997).

There are a report that the PAR-2 participates in the regulation of pancreatic secretion in pancreas (Bohm S. K., Biochem. J., 314: 1009-1016, 1996), the activation of ion channels and the like (Nguyen T. D. et al., J. Clin. Invest., 103: 261-269, 1999), and a report that the PAR-2 further participates in the regulation of duodenal movements and the like (Kawabata A. et al., Br. J. Pharmacol., 128(4): 865-872, 1999).

On the other hand, reports have been made that the tryptase is released from mast cells, exhibits an enzymatic activity only as its tetramer (Schwartz, L. B. et al., J. Biol. Chem., 256: 11939-11943, 1981), and participates in skin inflammation (Steinhoff M. et al., Exp. Dermatol., 8: 282-294, 1999) and pulmonary inflammation (Am. J. Physiol. Lung Cell. Mol. Physiol., 278: L193-L201, 2000).

However, there are many still unknown points related to the actions of the tryptase and the activation of PAR2, and other PAR2-activating enzymes are entirely not elucidated. Further, there is a report suggesting the existence of the above-mentioned trypsinogen in the airway as an enzyme for activating PAR2 in the airway However, the activity and quantity of the trypsinogen are completely not known, and only the existence of a protein by immunohistochemistry has been shown (Cocks T. M. et al., Nature, 398: 156-160, 1999). It has still more not reported that an airway-specific enzyme activating PAR2 exists.

On the one hand, in chronic inflammatory airway diseases, the exacerbation of the pathologic states due to the hypersecretion of mucous is cited as a pathologic state becoming a problem together with continuous inflammation (Jeffery, P. K. et al., Am. J. Respir. Crit. Dare. Med., 150: S6-13, 1994). The effects of macrolide drugs, such as erythromycin, against DPB (diffuse panbronchiolitis) belonging to the above-described diseases are known (Nagai, H. et al., Respiration, 58(3-4): 145-149), but the macrolide drugs are preferably not used in Europe, USA, and the like, because of being antibiotics.

Further, various theories related to target molecules mainly causing excessive mucus production promotion and secretion cell proliferation have been reported (Christian, P. et at., J. Clin. Invest., 85: 682-689, 1990), but an effective therapeutic drug does still not exist among drugs having been developed on the basis of the theories. Thereby, a drug effective for controlling the excessive mucus secretive production promotion is globally desired for chronic airway inflammatory diseases represented by COPD (Chronic Obstructive Pulmonary Disease).

### Disclosure of Invention

The problem of the present invention is to construct a method for screening an agent for inhibiting the mucus production-promoting action, EGFR (Epidermal Growth Factor Receptor) pathway-activating action, inflammation-promoting action (due to cytokine production promotion or the like), intracellular calcium influx-eliciting action, and a PAR (Protease-Activated Receptor, defined as a receptor that signal tranduction is caused by the proteolytic activity)-activating action of the active AST having the true structure, and further an evaluation system for detecting the inhibitory activity.

Namely, it can be expected that the compound or polypeptide inhibiting the activities of the AST obtained by using the method of the present invention, or the compound or polypeptide inhibiting the mucus production-promoting action, EGFR pathway-activating action, inflammation-promoting action, intracellular calcium influx-eliciting action, and PAR-activating action of the AST, can be used for inhibiting or modifying the physiological actions of the AST, such as a secretion-promoting or mucus production-promoting action for the secretory cells, a congealing fibrinogenolysis system action, an airway remodeling action, an airway inflammation action, actions for the proliferation and suppression of fibroblasts · epitheliocytes · ciliated cells · smooth muscle cells · goblet cells, actions related to the proliferation and metastasis of cancer, actions for mucociliary movements, and an anti-virus infection action, and for improving and treating pathologic states.

Further, since the AST activates the PAR, it can be expected that the compound or polypeptide can be used for inhibiting or modifying the physiological actions of the PAR, such as secretion-promoting actions (the mucus production-promoting actions of mucous gland cells, serous gland cells, and goblet cells, secretion-promoting actions for nerve cells neuroendocrine cells, a chloride ion secretion-promoting action in airway epithelial cells, and the like) for secretory cells, the relaxation actions of blood vessels, airways, intestinal tracts, and the like via epithelial cells or endothelial cells, a smooth muscle-contracting action, the inflammatory cytokine production-inducing and reinforcing actions of fibroblasts, epithelial cells and the like, actions for the proliferation and suppression of cells such as fibroblasts epithelial cells (ciliated cells, goblet cells, basal cells) · smooth muscle cells · secretory gland cells, and hypersensitiveness-reinforcing action for nerve cells, and for improving and treating pathologic states.

Furthermore, since the AST activates the EGFR pathway, it can be expected that the compound or polypeptide can be used for inhibiting or modifying physiological actions caused by the activation of the EGFR, such as secretion product production-promoting actions and cytokine production-promoting actions for secretory cells (mucous gland cells, serous gland cells, goblet cells, squamous -cells, cancer cells, and the like), inflammatory cytokine production-inducing and reinforcing actions for various EGFR pathway-having cells such as fibroblasts, endothelial cells, epithelial cells, and smooth muscle cells, and cell-proliferating actions for various EGFR pathway-having cells such as fibroblasts, endothelial cells, epithelial cells, ciliated cells, goblet cells, basal cells, squamous cells, cancer cells, smooth muscle cells, and secretory gland cells, and for improving and treating pathologic states.

From the direct and indirect actions on the AST, the compound or polypeptide can be used as a diagnostic drug or for an evaluation system for screening new drugs for chronic airway inflammation (chronic obstructive pulmonary diseases (including chronic bronchitis, pulmonary emphysema, diffuse pan-bronchiolitis, and bronchiectasis), bronchitic asthma), sinobronchitis syndrome, pulmonary fibrosis, respiratory anaphilaxia, pulmonary hypertension, diseases caused by congealing fibrinogenolysis system disorders, broadly-defined airway tissue cancer, arthritis, and skin inflammatory diseases.

The inventors of the present invention took the above-described conditions into consideration and intensively researched methods for purifying the AST. As the result, a method for efficiently purifying the active AST from sputum or recombinant insect cells has been established. Consequently, it has been possible to obtain the AST in a larger amount. The obtained purified protein has been used to determine its amino acid sequence. In consequence, it has clarified for the first time that the structure of the purified active AST has a protein structure in which a propeptide moiety is linked to a trypsin-like protein moiety via a disulfide bond, in addition to the already reported amino acid structure similar to that of a trypsin-like protein.

Further, it has been found that the enzyme is specifically expressed and located in large amounts in bronchial epithelium and glandulae bronchiales, especially ciliated epithelial cells, basal cells. It has also been found that the AST having said structure has an ability to activate the PAR. In addition, it has been found that the AST promotes the production of mucus in a mucus-secreting ability-having cell strain originated from a respiratory organ. The inventors of the present invention carried out research on the basis of the findings and consequently reached the present invention.

The inventors of the present invention have prepared a recombinant baculovirus vector on the basis of the cDNA sequence of HAST (namely, human AST) in insect cells (JP-A 8-89246, US-5804410, EP-699763, and Yamaoka K. et al., J. Biol. Chem., 273 (19): 11895-11901, 1998), and expressed the recombinant protein. The active protein has been purified from the recombinant protein by the purification method shown in Example 1, and the primary structure of the active protein has been determined. Consequently, it has been found that the active protein contains a protein having a structure in which a propeptide partial moiety sequence comprising an amino acid sequence between methionine (Met) at the 1^{st} position and arginine (Arg) at the 186^{th} position (concretely, a propeptide moiety started from asparagine (Asn) at the 145^{th} position or aspartic acid (Asp) at the 162^{nd} position) is bound to a trypsin-like protein moiety via a disulfide bond, in addition to the already reported trypsin-like structure protein (JP-A 7-067640, S. Yasuoka et al., Am. J. Respir. Cell Mol. Biol., 16: p300-308, 1997, a trypsin-like protein moiety starting from isoleucine (Ile) at the 187^{th} position). And, it has been discovered that the protein having the structure mentioned above is the main component of the active AST.

Further, natural HAST has been purified from sputum, and the amino acid sequence of the natural HAST has similarly been determined. Consequently, it has again been found that there is a protein having a structure in which a propeptide partial moiety sequence (concretely, a propeptide moiety starting from isoleucine (Ile) at the 173^{rd} position) is bound to a trypsin-like protein moiety starting from isoleucine (Ile) at the 187^{th} position via a disulfide bond. And, it has been found that the protein having the structure is the main component of the active AST.

Meanwhile, the. HAST has been considered to specifically exist in human being, and has been named HAT (Human Airway Trypsin-like _ Protease) (JP-A 7-067640, S. Yasuoka et al., Am. J. Respir. Cell Mol. Biol., 16: p300-308, 1997). However, the inventors of the present invention have carried out a PCR (Polymerase Chain Reaction) using a degenerate primer prepared on the basis of the homology of several serine proteases and further using a cDNA prepared from a mouse airway as a template, and determined the sequence of the obtained DNA fragment. Consequently, it has been found for the first time that a HAST-like protein exists also in an animal, such as mouse, not carrying out standing bipedal locomotion. In addition, the full length cDNA encoding the AST-like protein of the mouse has been obtained by executing 5'-RACE (Rapid Amplification of cDNA Ends), 3'-RACE. The inventors of the present invention have examined the tissue specificity of the gene expression, and also observed an airway-specific expression in the mouse. Further, the obtained mouse AST gene has been expressed in insect cells as a recombinant, and the primary structure of the protein has been determined. In consequence, it has been clarified that ,similarly to the human AST (HAST), the mouse AST is also an enzyme having a structure in which the partial sequence of a propeptide comprising an amino acid sequence between methionine (Met) at the 1^{st} position and arginine (Arg) at the 186^{th} position is bound to a trypsin-like protein moiety starting from isoleucine (Ile) at the 187^{th} position via a disulfide bond. By the similar method, the cloning of hamster AST and the cloning of guinea pig AST have also been succeeded.

Additionally, by methods similar to the above-described methods, it has been found for the first time that HAST-like proteins exist also in higher mammals such as monkey, pig, dog, rabbit, and bovine. Concretely, a degenerate primer designed on the basis of cDNA sequences of human and rodent ASTs has been prepared, and a PCR using a cDNA synthesized from a total RNA extracted from the airway of a higher mammal as a template has been carried out to amplify the partial cDNA sequence of the AST of the higher mammal, followed by determining the DNA sequence. A primer for RACE has been prepared on the basis of the determined DNA sequence, and 5'-RACE, 3'-RACE are performed to obtain the full length cDNA encoding the HAST-like protein. The mammalian AST gene thus obtained has transiently been expressed in human cultured cells, and it has consequently been found that a trypsin-like activity similar to that of the human AST (HAST) can be expressed. Further, a simian AST gene has been expressed in insect cells as a recombinant, and the primary structure of the protein has been determined. Consequently, the N-terminal amino acid sequence of a trypsin-like protein moiety starting from IIGG and the partial amino acid sequence of a propeptide starting from DQAA have been detected in equimolar amounts, respectively. And, it has been clarified that, similarly to the human AST, the simian AST is also an enzyme having a structure in which a propeptide partial sequence comprising an amino acid sequence between methionine (Met) at the 1^{st} position and arginine (Arg) at the 186^{th} position is bound to a trypsin-like protein moiety starting from isoleucine (Ile) at the 187^{th} position via a disulfide bond.

The above-described discovery of the existence of the animal AST enables the evaluation of the expression of the animal AST at a gene level or at a protein level, and the evaluation in the expression and activity of the animal AST in an animal disease model is greatly useful for clarifying the participation of the AST in said pathological state. Additionally, the participation of the AST in a pathologic model animal can be examined by inhibiting the expression of the AST with an anti-sense oligonucleotide or transducing an expression vector to increase the expression of the AST. Further provided is information which is important on the evaluation of the medicinal efficacies of an AST inhibitor or an inhibitory polypeptide (including proteins and antibodies) in an animal model and also important on the examination and evaluation of a species difference between animals.

Next, a recombinant AST having the above-described structure has been allowed to act on a recombinant PAR-expressing insect cell, and the activation of the PAR has been evaluated, while using calcium influx as an indicator. Consequently, it has been found for the first time that AST activates PAR and the calcium influx is caused in the insect cell in which PAR1 and PAR2 are expressed.

Further, they have been found for the first time that the AST activates the PAR in normal human airway epithelial cells and airway epithelial cell strain to cause calcium influx and that the AST having said structure causes IL-8 production promotion and cell proliferation with a signal transduction system mediating the PAR, and it has been clarified that the AST modifies the morbid state of an airway disease with the signal transduction system mediating the PAR. And, a screening system based on the enzymatic activity or PAR activation action of a recombinant AST protein having said structure has been constructed on the basis of the results.

In addition, it has been found for the first time that, in a human mucus-secreting cell strain, the AST having said structure promotes mucus production, causes intracellular calcium influx, activates an EGFR pathway, and the like to cause IL-8 production promotion and cell proliferation. And it has been clarified that, in a chronic airway disease in which the mucus production is promoted, the AST promotes the mucus production, causes the intracellular calcium influx, activates the PAR, the EGFR pathway, and the like to cause the IL-8 production promotion and the cell proliferation, thus modifying the pathologic state.

Furthermore, it has thus been found that a screening system can be constructed on the basis of the enzymatic activity or mucus production-promoting activity, PAR-activating action, intracellular calcium influx-eliciting action, EGFR pathway-activating action and IL-8 production-reinforcing action of the recombinant AST protein having the above-described structure, and the present invention has thus been completed.

Namely, the present invention is based on the AST having the structure in which the propeptide moiety is bound to the trypsin-like protein moiety via the disulfide bond.

Specifically, the present invention is the inhibitor or inhibitory polypeptide-screening system using the mucus production-promoting action, PAR-activating action, intracellular calcium influx-eliciting action, EGFR pathway-activating action and IL-8 production-reinforcing action or cell proliferation-promoting action of the enzyme having said structure as an indicator.

The present invention will more concretely be described as follows.
1. A method for screening for a compound or a polypeptide capable of inhibiting AST by mixing:
   i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
   ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
   iii) a cell expressing said enzyme, or
   iv) a tissue expressing said enzyme,
   the compound or the polypeptide to be assayed, and further a PAR-expressing cell, and then using the PAR activation as an indicator.
2. The detection method according to claim 1, wherein the protease-activated receptor is PAR2.
3. A method for screening for a compound or a polypeptide capable of inhibiting AST, by mixing:
   i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
   ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
   iii) a cell expressing said enzyme, or
   iv) a tissue expressing said enzyme,
   the compound or the polypeptide to be assayed, and further a cell having an intracellular calcium influx ability, and then using the intracellular calcium influx as an indicator.
4. A method for screening for a compound or a polypeptide capable of inhibiting AST, by mixing:
   i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
   ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
   iii) a cell expressing said enzyme, or
   iv) a tissue expressing said enzyme,
   the compound or the polypeptide to be assayed, and further a cell having a mucus-producing ability, and then using the secretion product as an indicator.
5. The detection method according to claim 4, wherein a method for assaying the mucus-producing ability is an AB-PAS staining method.
6. The detection method according to claim 4, wherein the indicator of the mucus-producing ability is the quantity of expressed Muc5AC.
7. The detection method according to claim 4, wherein the indicator of the mucus-producing ability is the quantity of released or produced EGF, HB-EGF, TGF-α, Amphiregulin or Betacellulin.
8. The detection method according to claim 4, wherein the indicator of the mucus-producing ability is the activation of an EGF-R signal transduction pathway.
9. A method for screening for a compound or a polypeptide capable of inhibiting EGF, HB-EGF, TGF-α, Amphiregulin or Betacellulin, by mixing:
   i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
   ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
   iii) a cell expressing said enzyme, or
   iv) a tissue expressing said enzyme,
   the compound or the polypeptide to be assayed, and further a cell having an EGF, HB-EGF, TGF-α, Amphiregulin or Betacellulin, and then using the release quantity of the EGF, HB-EGF, TGF-α, Amphiregulin or Betacellulin, as an indicator.
10. A method for screening for a compound or polypeptide capable of inhibiting AST, by mixing:
   i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
   ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
   iii) a cell expressing said enzyme, or
   iv) a tissue expressing said enzyme,
   the compound or the polypeptide to be assayed, and further a cell having an EGFR signal transduction pathway, and then using the activation of the EGFR signal transduction pathway as an indicator.
11. A method for screening for a compound or a polypeptide capable of inhibiting AST, by mixing:
   i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
   ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
   iii) a cell expressing said enzyme, or
   iv) a tissue expressing said enzyme,
   the compound or the polypeptide to be assayed, and further a cell and using the release quantity or expression quantity of II-8 or the transcription activity of the II-8 promoter as an indicator.

### Brief Description of Drawings

Figure 1 shows the results of AB-PAS staining in a mucus-secreting cell strain NCI-H292 by the addition of EGF-L.
Figure 2 shows the results of AB-PAS staining in a mucus-secreting cell strain NCI-H292 by the addition of the HAST of the present invention or EGF·L.
Figure 3 shows the results of AB-PAS staining in a mucus-secreting cell strain NCI-H292 by the addition of the HAST of the present invention and a serine protease inhibitor.
Figure 4 shows the results of AB-PAS staining in a mucus-secreting cell strain NCI-H292-by the addition of the HAST of the present invention or thermally treated HAST.
Figure 5 shows the assay results of the mRNA quantities ofMuc5AC, when a protease inhibitor was simultaneously added to a mucus-secreting cell strain NCI-H292 to which the HAST of the present invention was added.
Figure 6 shows the assay results of the mRNA quantities of Muc5AC, when an EGFR neutralizing antibody or a protease inhibitor was simultaneously added to a mucus-secreting cell strain NCI-H292 to which the HAST of the present invention was added.
Figure 7 shows the assay results of the mRNA quantities of Muc5AC in a mucus-secreting cell strain NCI-H292 to which the AST of the present invention, trypsin, tryptase, or elastase was added.
Figure 8 shows assay results that the IL-8 release promotion from an airway epithelial cell strain BEAS-2B by the addition of AST of the present invention is inhibited with protease inhibitors.
Figure 9 shows that the IL-8 release promotion from an airway epithelial cell strain BEAS-2B by the addition of AST of the present invention occurs at a transcription level.
Figure 10 shows that the AST of the present invention promotes the tyrosine phosphorylation of EGFR in mucus-secreting cell strain NCI-H292.
Figure 11 is a figure showing the homology of the amino acid sequence of human AST and mouse AST.
Figure 12 shows calcium influx in PAR-expressing insect cells due to the AST of the present invention.
Figure 13 shows calcium influx due to the AST of the present invention with the normal human airway epithelial cells.
Figure 14 shows calcium influx due to the AST of the present invention with the airway epithelial cell strain.
Figure 15 shows the evaluation results of the inhibitory activities of the anti-AST antibodies of the present invention.
Figure 16 shows the standard curve of an assay system using the anti-AST polyclonal antibody of the present invention against a recombinant AST.
Figure 17 shows the assay results of AST in sputum by an assay system using the anti-AST polyclonal antibody of the present invention.
Figure 18 shows results obtained by examining the liquid phase antigenic reactivity of the anti-AST monoclonal antibody and anti-AST polyclonal antibody of the present invention with the recombinant AST.
Figure 19 shows the standard curve of an assay system using the anti-AST monoclonal antibody and anti-AST polyclonal antibody of the present invention against the recombinant AST.

### Best Mode for Carrying Out the Invention

In the airway-specific trypsin-like enzyme referred to in the present invention comprising the whole amino acid sequence represented by SEQ ID NO: 1, the propeptide moiety is bound to the trypsin-like protein moiety via the disulfide bond of Cys at the 173^{rd} position to Cys at the 292^{nd} position. In the mammalian airway-specific trypsin-like enzyme referred to in the present invention having the 66% or more homology with the amino acid sequence represented by SEQ ID NO: 1, a moiety corresponding to the propeptide moiety is bound to a moiety corresponding to the trypsin-like protein moiety via the disulfide bond of a cysteine moiety corresponding to Cys at the 173^{rd} position to a cysteine moiety corresponding to Cys at the 292^{nd} position in the AST represented by SEQ ID NO: 1.

In a case of the human AST, the propeptide moiety is a propeptide moiety whose N-terminal (amino terminal) amino acid is an amino acid between Asn at the 145^{th} position and Ile at the 170^{th} position. A propeptide moiety ranged from either of Asn at the 145^{th} position, Asp at the 162^{nd} position and Ile at the 170^{th} position to Arg at the 186^{th} position as the C-terminal (carboxy terminal) is especially preferable. A propeptide moiety ranged from either of Asp at the 162^{nd} position and Ile at the 170^{th} position to Arg at the 186^{th} position is most preferable.

The AST or AST-expressing cell used in the method for screening the AST-inhibiting activity of the compound or polypeptide to be assayed, is generally the structure-having AST recited above or a cell producing said AST, preferably an AST-expressing cell or organ, tissue originated from an oral tissue, nasal tissue, paranasal sinus tissue, pharyngeal tissue, laryngeal tissue, trachea, bronchi or pulmonary tissue, a cell originated from the tissue or a cell strain thereof. The cell or cell strain originated from the tissue includes cancer cells and cancer cell strains. Especially, epithelial cells, in particular, ciliated epithelium cells or basal cells or cell strains originated from the cells are preferable. The purified AST having the above-described structure is most preferable for being supplied to evaluation.

In the method for screening the inhibitory activity of the compound or polypeptide to be assayed against the mucus production-promoting action due to the AST, the dyeing quantity of the mucus glycoprotein by an AB-PAS staining method, the release quantity of a sulfuric acid-labeled polymer, the production quantity of mucin, the mRNA expression quantity of mucin (especially Muc5AC), the promoter transcription activity of the mucin (especially Muc5AC), the ligand-producing or releasing quantity of an EGF receptor, the phosphorylated tyrosine quantity of a protein participated in an EGF receptor signal transduction pathway, or the quantity of a phosphorylated MAPK (MAP kinase) is preferably cited as the indicator of the mucus production-promoting action.

In a method for screening the inhibitory activity of the compound or polypeptide to be assayed against the EGFR pathway-activating action due to the AST, the cutting or release quantity of the EGFR-L (EGFR-Ligand), the assay target of the already reported EGFR signal transduction system (Wells A., Int. J. of Biochem. & Cell Biology, 31: 637-643, 1999), a phenomenon caused by the signal transduction, or the like, can be used as the indicator of the EGFR pathway activation to assay the EGFR signal transduction system activation due to the AST.

The indicator of the EGFR pathway activation includes intracellular calcium influx, a cell proliferation assay, the assay of the transcription activity of a gene (IL-8, IL-6, GM-CSF or the like) containing a transcription element such as NFkB or AP-1, the assay of a produced protein, the assay of an inositol phosphate-degrading activity in cells, the assay of prostaglandin E₂ production, the assay of thromboxane A₂, the assay of cAMP (cyclic AMP), the assay of a mucus production quantity, the production quantity of mucin, the mRNA expression quantity of mucin (especially Muc5AC), the promoter transcription activity of the mucin (especially Muc5AC), and the phosphorylated tyrosine quantity of a protein (the phosphorylated tyrosine quantity of EGFR, MAPK (MAP kinase) or the like) participated in an EGF receptor signal transduction pathway.

In a method for screening the inhibitory activity of the compound or polypeptide to be assayed against the PAR-activating action due to the AST, the PAR signal transduction system activation due to the AST can be assayed with the already reported assay target as the indicator of the PAR activation signal transduction.

The indicator of the PAR activation signal transduction includes an intracellular calcium influx, a cell proliferation assay, the assay of the transcription activity or produced protein of a gene (IL-8, IL-6, GM-CSF or the like) containing a transcription element such as NFkB or AP-1, the assay of an inositol phosphate-degrading activity in cells, the assay of prostaglandin E₂ production, the assay of thromboxane A₂, the assay of cAMP (cyclic AMP), a method for directly or indirectly assaying the production of NO (Nitric Oxide), the assay of calcium release or electric current with Xenopus Oocyte, the assay of a chloride ion transport quantity via a chloride channel, and the assay of a secreted mucus quantity.

Additionally, the AST-expressing cell is preferably a recombinant cell expressing said enzyme. Further, the AST-expressing cell or a tissue expressing said enzyme is preferably originated from an oral tissue, nasal tissue, paranasal sinus tissue, pharyngeal tissue, laryngeal tissue, trachea, bronchi or pulmonary tissue, or is preferably a cell originated from the tissue, an organ or the tissue, or a cell strain thereof. The cell or cell strain originated from the tissue includes cancer cells and cancer cell strains. Especially, epithelial cells, particularly, airway epithelial cells, more particularly, ciliated epithelium cells or basal cells or cell strains originated from the cells are preferable. In addition, the AST-expressing cell includes goblet cells or a cell strain originated from the goblet cells, mucous gland cells or a cell strain originated from the mucous gland cells, and serous gland cells or a cell strain originated from the serous gland cells.

The cells for evaluating the secretion include a mucus-secreting cell strain, NCI-H292, and preferably further include secretory ability-having cells, organ or tissue existing in an oral tissue, nasal tissue, paranasal sinus tissue, pharyngeal tissue, laryngeal tissue, trachea, bronchi or pulmonary tissue, preferably airway epithelial cells, particularly ciliated epithelium cells or basal cells or cell strains originated from the cells. The cells or cell strain originated from the tissue include cancer cells and cancer cell strains. In addition, the secretory ability-having cells preferably include goblet cells or a cell strain originated from the goblet cells, mucous gland cells or a cell strain originated from the mucous gland cells, and serous gland cells or a cell strain originated from the serous gland cells. NCI-H 292 cells, A549 cells and the like established as cell strains are most preferably supplied for the evaluation.

The PAR-expressing cells include PAR-expressing recombinant insect cells, and preferably further include PAR-expressing cells, organ or tissue existing in an oral tissue, nasal tissue, paranasal sinus tissue, pharyngeal tissue, laryngeal tissue, trachea, bronchi or pulmonary tissue, especially preferably airway epithelial cells, particularly ciliated epithelium cells or basal cells or cell strains originated from the cells. In addition, the PAR-expressing cells preferably include goblet cells or a cell strain originated from the goblet cells, mucous gland cells or a cell strain originated from the mucous gland cells, serous gland cells or a cell strain originated from the serous gland cells, airway smooth muscle cells, pulmonary fibroblasts, and nerve cells. BEAS-2B established as a cell strain is most preferably supplied for the evaluation.

The concrete examples of the PAR are protease-activating receptors to be activated with the AST, preferably PAR1 and PAR2 among known PAR1, PAR2, PAR3, and PAR4, most preferably PAR2 among the four PARs.

The EGFR-L-expressing cells include recombinant EGFR-L-expressing animal cells, and preferably further include cells, organs or tissues existing in an oral tissue, nasal tissue, paranasal sinus tissue, pharyngeal tissue, laryngeal tissue, trachea, bronchi or pulmonary tissue, especially preferably airway epithelial cells, particularly ciliated epithelium cells or basal cells or cell strains originated from the cells. In addition, the EGFR-L-expressing cells preferably include goblet cells or a cell strain originated from the goblet cells, mucous gland cells or a cell strain originated from the mucous gland cells, serous gland cells or a cell strain originated from the serous gland cells, airway smooth muscle cells, pulmonary fibroblasts, and nerve cells.

Concrete examples include HCI-H292, A549, and the like established as cell strains. Recombinant EGFR-L-expressing insect cells or animal cells are preferably supplied for evaluation.

The concrete example of the EGFR-L is generally EGFR-L to be released with the AST, and includes known EGF, HB-EGF, TGF- α, Amphiregulin, and Betacellulin. EGF and HB-EGF are preferable among the known substances, and EGF is most preferable.

The EGFR-expressing cells include NCI-H292 cell strain, and preferably further include cells, organs or tissues existing in an oral tissue, nasal tissue, paranasal sinus tissue, pharyngeal tissue, laryngeal tissue, trachea, bronchi or pulmonary tissue, especially preferably airway epithelial cells, particularly ciliated epithelium cells, squamous cells, basal cells, or cancer cells or cell strains originated from the cells. In addition, the EGFR-L-expressing cells preferably include goblet cells or a cell strain originated from the goblet cells, mucous gland cells or a cell strain originated from the mucous gland cells, serous gland cells or a cell strain originated from the serous gland cells, airway smooth muscle cells, pulmonary fibroblasts, and nerve cells. The EGFR-L-expressing cells are most preferably HCI-H292, A549 and the like, established as cell strains.

The concrete example of the EGFR-L is generally EGFR-L to be released with the AST, and includes known EGF, HB-EGF, TGF- α, Amphiregulin, and Betacellulin. EGF and HB-EGF are preferable among the known substances, and EGF is most preferable.

Variants of the mammalian AST gene described in the present invention include that of mouse, encoded by a mRNA or cDNA represented by SEQ ID NO: 5 or SEQ ID NO: 6. Similarly, further variants of the mammalian AST described in the present invention include those of monkey, pig, dog, rabbit, cattle, hamster, and guinea pig, the mRNA or cDNA encoding those ASTs represented by SEQ ID NO: 27, 29, 31, 33, 35, 37,or 39.

Expression vectors encoding the AST recital in the present invention and prepared with the whole or partial information of the nucleic acid sequences may be produced as well as; a cell transfected or transformed with the expression vector; a method for producing the AST, comprising culturing the cell and then recovering the structure-having active AST from the culture solution or cultured cells; and the AST obtained by the production method. Further, the natural mammalian AST may be purified from the body fluid, cells or cell strains of a mammal in which the natural mammalian AST exists.

Compound identified by the methods of the invention may be used to bent a mammal having a disease characterized by the activation excess or up-regulate of the above-described AST.

The disease characterized by the activation excess or up-regulate of the above-described AST includes chronic airway inflammation, chronic obstructive pulmonary diseases, chronic bronchitis, pulmonary emphysema, diffuse panbronchiolitis, bronchiectasis, asthma, sinobronchitis syndrome, fibrosis, nerve oversensitivity, diseases due to congealing fibrinogenolysis system disorders, cancer, arthritis, and skin-inflammatory diseases, and especially includes inflammatory diseases in respiratory systems and diseases whose pathologic conditions are caused by secretion disorders in oral tissues, nasal tissues, paranasal sinus tissues, pharyngeal tissues, laryngeal tissues, tracheae, bronchi, or pulmonary tissues, extracellular substrate synthesis disorders, cell amplification disorders, cell differentiation disorders, nerve system disorders, immune system disorders, mucociliary transport system disorders or congealing fibrinogenolysis system disorders.

The disease characterized by the mucus production promotion, inflammation promotion, intracellular calcium influx promotion, EGFR pathway activation, or PAR activation due to the AST includes chronic airway inflammation, chronic obstructive pulmonary diseases, chronic bronchitis, pulmonary emphysema, diffuse panbronchiolitis, bronchiectasis, asthma, sinobronchitis syndrome, fibrosis, nerve oversensitivity, diseases due to congealing fibrinogenolysis system disorders, cancer, arthritis, and skin-inflammatory diseases, and especially includes inflammatory diseases in respiratory systems and diseases whose pathologic conditions are caused by secretion disorders in oral tissues, nasal tissues, paranasal sinus tissues, pharyngeal tissues, laryngeal tissues, tracheae, bronchi, or pulmonary tissues, extracellular substrate synthesis disorders, cell amplification disorders, cell differentiation disorders, nerve system disorders, immune system disorders, mucociliary transport system disorders or congealing fibrinogenolysis system disorders.

In Examples 13, 14 of the present invention, the mucus production-promoting action of the AST is evaluated with the NCI-H292 cell strain, and the cell used in the system for screening the mucus production promotion inhibition of the AST inhibitors is preferably a cell having a secreting ability, more preferably a cell, organ or tissue having the secreting ability in a respiratory system. The NCI-H292 cell, A549 cell or the like established as a cell strain is most preferable to supply for the evaluation.

The secretion product used as the indicator is an organic substance or an inorganic substance, and the organic substance is preferably a glycoprotein, more preferably a mucus. Mucin which is a component of the mucus is most preferable as a secretion indicator from relations with pathologic states (Tsuda, T. et al., "Molecular Biology of Respiratory Diseases"; 87-92, 1998). Further, Muc5AC (Meerzaman, D. et al., J. Biol. Chem., 269: 12932-12939, 1994), Muc2 (Gum, J. R. Jr. et al., J. Biol. Chem., 269 (4): 2440-2446, 1994), and the like, to which attentions are paid in the pathologic states of hypersecretion such as COPD are especially preferable among the mucin.

The execution form of the assay indicator in the HAST inhibitor-screening system based on the mucus production-promoting action preferably includes (1) an enzyme immune assay using a monoclonal antibody 17Q2 (human airway mucin is used as an antigen) which is an antibody against the mucin antigen (Thomas, E. et al., Am. J. Respir. Cell Mol. Biol., 14: 104-112, 1996) or the like, the mucus-dyeing method (AB-PAS dyeing) shown in Example 13, and a method for assaying the release quantity of a sulfuric acid-labeled polymer, at the level of glycoproteins, and (2) a real time PCR method disclosed in Example 14 as a method for assaying the mRNA quantity of the mucin (preferably Muc5AC, Muc2), and a reporter gene assay system using the promoter domain of the mucin as a method for evaluating the transcription activity of the gene, at the level of genes.

In addition, since the Mucr5AC mRNA increase based on the activity of the HAST is blocked with the EGF-R neutralizing antibody as shown in Example 14, it can be estimated that the signal transduction of the EGF-R pathway can become one of the main pathways of the mucus production promotion due to the AST (Takeyama, K. et al., Proc. Natl. Acad. Sci. USA, 96: 3081-3086, 1999). The effect of the AST inhibitor can be assayed by quantifying the reaction participating in the EGF-R signal transduction pathway (Louis, M. L. et al., Current Opinion in Cell Biology, 11: 177-183, 1999). If the reactions can be quantified in the above-described related pathway, all of the reactions will be able to be used as assay indicators. For example, the phosphorylation of the tyrosine residue of the EGF-R and the phosphorylation of MAPK (Mitogen-Activated Protein Kinase) in target cells can be used as indicators in an immune assay.

The execution form of the AST inhibitor-screening system based on the mucus production-promoting action includes a mucus production-inhibiting effect assay which comprises adding the AST to a tissue, organ or cultured cell having a mucus-secreting ability, adding a compound or the like, and then assaying the quantity of the secretion product, the quantity of the mRNA, the transcription activity of said gene, or the like. Further, as described above, the inhibition effect can be judged by a method for directly assaying the content of the EGFR-L in the organ or the cell culture supernatant, a method for adding to any other cell system and then indirectly assaying the content of the EGFR-L, or the like. The EGFR-L to be assayed preferably includes EGF (Epidermal Growth Factor, Carpenter, G. et al., J. Biol. Chem., 265: 7709-7712, 1990), HB-EGF (Heparin-binding EGF-like Growth Factor, Abraham, J. A. et al., Biochem. Biophys. Res. Commun., 190: 125-133, 1993), TGF- α (Transforming Growth Factor-α , Lee, D. C. et al., Pharm. Rev., 47: 51-85, 1995), Amphiregulin (Plowman, G. D. et al., Mol. Cell. Biol., 10: 1969-1981, 1990), and Betacellulin (Shing, Y. et al., Science, 259: 1604-1607, 1993) (Gerhard, R. et al., Biochim. Biophys. Acta, 1333: F179-F199, 1997). EGF, HB-EGF are especially preferable.

### Examples

The present invention will be explained in more detail hereafter with examples, but the present invention is not limited to the examples.

### [Example 1] Preparation of AST

The purification of an active natural AST from a sputum was carried out by the same method as the below-described method for purifying a recombinant AST.

The purification of a recombinant HAT was carried out by preparing a recombinant baculovirus vector on the basis of a cDNA sequence disclosed in JP-A 8-89246, US-5804410, EP-699763, and Yamaoka K. et al., J. Biol. Chem., 273(19): 11895-11901, 1998), infecting insect cells with the recombinant baculovirus vector to produce the recombinant AST (rAST), and then purifying the rAST from the cell fraction of the product. Namely, the insect cells (Tn-5 cells) were allowed to grow up to a density of 5 × 10⁶ cells / mol in a single layer, and the culture medium was then removed. A serum-free culture medium containing the recombinant AST-expressing baculovirus was added to the grown insect cells in an MOI (Multiplicity of Infection) of 0.2 to 0.5 per cell to infect the insect cells with the recombinant AST-expressing baculovirus, and the infected insect cells were cultured for three days to express the AST. The identification of the expressed protein was carried out by a western blot method using SDS-PAGE and an anti-AST peptide antibody (Anal. Biochem., 112, 195-203, 1981).

The culture product was centrifuged (about 500 × g) to separate the cells from the supernatant. The cells corresponding to 450 mL were suspended in 100 mL of M-PER (Mammalian Protein Extraction Reagent, produced by PIERCE Corp.), incubated at room temperature for 30 minutes, and further subjected to an ultrasonic crushing treatment on ice for 15 minutes to lyse the cells. The obtained lysate was centrifuged at 10,000 rpm at 4°C for 30 minutes, and the supernatant was recovered. The recovered supernatant was dialyzed with 1 liter of 50 mM sodium acetate (pH 4) / 0.01% PEG 6000 at room temperature for two hours with stirring, and then centrifuged at 10,000 rpm at 4°C for 30 minutes to recover the supernatant.

Subsequently, the recovered sample was mixed with BSA (final concentration: 100 µg/mL), twice analyzed with 2-liters of 50 mM Tris-HCl (pH 8) / 0.5M NaCl / 0.01% PEG 6000, and then centrifuged at 10,000 rpm at 4°C for 30 minutes to recover the supernatant. The recovered supernatant was put on benzamidine sepharose 6B (10 mL bed volume), washed with 100 mL of 50 mM, Tris-HCl (pH 8) / 0.5M NaCl / 0.01% PEG 6000 in a column, further washed with 80 mL of a 10 mM sodium phosphate buffer solution (pH 7.5) in the column, and then subjected to the elution of the bound protein with 10 mM HCl (pH 2). After the elution, the absorbance (280 nm) of each fraction was assayed, and the main peak fractions were collected. The collected main peak fractions were subjected to an SDS polyacrylamide gel electrophoresis (SDS-PAGE) to detect and identify the protein having a molecular weight of about 30 kD, which was frozen and stored as the purified rAST sample.

### [Example 2] Activity assay of AST

50 µ L of an AST-containing solution was added to 1.0 mL of a 50 mM Tris-HCl buffer solution (pH 8.6) containing 100 *µ*M of a synthetic substrate Boc-Phe-Ser-Arg-MCA (MCA: methylcoumarinamide) for trypsin and 0.01% of BSA and then incubated at 37°C for one hour. Subsequently, 1 mL of 30% acetic acid was added, and the produced 7-amino-4-methylcoumarin (AMC) was assayed by fluorometry (fluorescent light: 460 nm, exciting light: 380 nm). The enzymatic activity was calculated. The activity for producing 1 pM of AMC for one minute was defined as one unit.

### [Example 3] Determination of amino acid sequences of recombinant AST and natural AST

The purified protein obtained in Example 1 was subjected to SDS-PAGE under reducing and non-reducing conditions, respectively. After each electrophoresis, the protein in the gel was transferred to a PVDF (polyvinylidene disulfide) membrane (Immobilon-P-transfer membrane, Millipore Corp.), and then dyed in a Coomassie Blue R-250 solution (0.1% of a 50% methanol solution). The band of the AST protein at a place near to about 30 kD was cut out, and the amino acid sequence of the AST protein was determined.

Consequently, with respect to the recombinant AST expressed in the insect cells, an amino acid sequence starting from I-L-G-G was identified under the reducing condition. Three kinds of bands were identified under the non-reducing condition. When the three kinds of the proteins were named as (a); (b) and (c) sequentially from the protein having the largest molecular weight, the contents of (a), (b) and (c) were 8%, 87% and 5%, respectively, which were estimated by the dyeing of silver. The bands were cut out, and then the amino acid sequences of the proteins were determined. The determined amino acid sequences were (a) an amino acid sequence starting from N-S-G-N-L-E-I-N and I-L-G-G-T-E-A-E in equivalent moles, respectively, (b) an amino acid sequence starting from D-Q-A-A-A-N-W-L and I-L-G-G-T-E-A-E in equivalent moles, respectively, and (c) an amino acid sequence starting from I-L-G-G-T-E-A-E. The previously reported AST is the protein having the structure (c).

From the above-described results, it was found that the main band (87%) of the purified rAST had the structure (b) wherein a propeptide moiety starting from the D-Q-A-A-A₋N-W-L was bound to a trypsin-like protein moiety starting from the I-L-G-G-PE-A-E via a disulfide bond. The structure has the activity.

On the other hand, with respect to the AST purified from the sputum, an amino acid sequence starting from the already reported I-L-G-G was identified under the reducing condition. Meanwhile, the peaks of amino acids could be identified, respectively, in addition to the I-L-G-G under the non-reducing condition. The sequence was read as I-N-E-Blank-G-A-G-P (the first I was the same as that of the main peak). The sequence coincided with the amino acid sequence of the human AST, starting from I at the 170^{th} position of the propeptide. Thereby, it was found for the first time that the natural AST originated from the sputum mainly had a structure wherein the propeptide was bound via a disulfide bond, similarly to the recombinant AST. It was clarified that the natural AST had a structure in which the cysteine of the disulfide bond on the propeptide (light chain) side was cysteine at the 173r^{d} position and in which the trypsin-like enzyme moiety (heavy chain) was bound to the cysteine moiety corresponding to cysteine at the 292^{nd} position by a structure homology comparison with the other trypsin substance via the disulfide bond.

### [Example 4] Preparation of various recombinant human PAR-expressing cells

### (1) Acquisition of PAR cDNA and preparation of PAR cDNA-containing transfer vector

RNA was extracted from normal human airway epithelial cells, and cDNA was synthesized using a cDNA synthesis kit (made by Amersham Pharmacia Biotech. Corp.). PAR-1 was amplified from primers PAR1F (SEQ ID NO: 7) and PAR1R (SEQ ID NO: 8) by a PCR, and PAR-2 was amplified from primers PAR2F (SEQ ID NO: 9) and PAR2R (SEQ ID NO: 10) by a PCR. PAR-3 was also amplified from primers PAR3F (SEQ ID NO: 11) and PAR3R (SEQ ID NO: 12) by a PCR. PAR-4 was further amplified from primers PAR4F (SEQ ID NO: 13) and PAR4R (SEQ ID NO: 14) with a 1/100 amount of a human prostate gland / originated cDNA (purchased from Clontech) by a PCR. The PCR reaction solution was subjected to an agarose gel electrophoresis. Consequently, the band of the cDNA fragment of PAR-1, PAR-2, PAR-3 or PAR-4 was recognized. The DNA fragment was extracted and purified from the gel with a DNA purification kit (using QIAE XII made by QIAGEN Corp.). The purified DNA fragment was cleaved with EcoRI, BamHI (Bgl II with respect to PAR-4), and then ligated to a fragment obtained by cleaving a baculovirus transfer vector pVL 1393 (Invitrogen Corp.) with restriction enzymes EcoRI and BamHI. The obtained reaction solution was used to transform Escherichia coli JM 109, and plasmids were extracted from several transformant colonies. Several clones of the extracted plasmids were purified (using a plasmid extraction kit made by QIAGEN Corp.). The DNA sequences were determined using the purified plasmids with a fluorescence automatic sequencer made by ABI Corp. Consequently, it was found that the DNA fragments encoded the PAR.

### (2). Preparation of recombinant baculovirus for expressing PAR

BaculoGold^{™} transfection kit made by PharMingen Corp. was used for preparing a recombinant baculovirus. Insect cells Sf9 were sowed on a 35 mm dish in a density of 1 × 10⁶ cells / mL and adhered at room temperature for 30 minutes. On the other hand, 0.5 mg of BaculoGold^{™} DNA and 2 µg of pVL-PAR plasmid DNA were mixed with each other, left at room temperature for 5 minutes, and then well mixed with 0.5 mL of a transfection buffer B (25 mM HEPES, pH 7.1, 125 mM CaCl₂, 140 mM NaCl). Then, the culture medium of the sowed Sf9 cells was taken out and then mixed with 0.5 mL of a transfection buffer A (Grace culture medium, containing 10% bovine serum). A solution of DNA in the transfection buffer B was gradually dropped on the mixture to carry out the cotransfection, and incubated at 27°C for 4 hours. Then, the culture medium was replaced by a new Grace culture medium (containing 10% bovine serum). Five days later, the culture supernatant containing the recombinant viruses was recovered. 100 µL of the culture supernatant and 200 µm of EX-CELL 405 culture medium were prepared, and then used to infect the Sf9 cells (1 × 10⁶ cells / dish) at room temperature for one hour. The supernatant was taken out, dropwisely mixed with 2 mL of a 1% SeaPlaque agarose-containing Grace culture medium (42°C), and left at room temperature for 10 minutes until to solidify the agarose. Then, the mixture was cultured at 27°C for 5 days. On the fifth day a neutral red / PBS (Phosphate Buffered Saline) solution (0.1 g/L) was added in a volume of 1 mL / dish, and on the sixth day the virus plaques were observed and identified. Several single virus plaques were taken out together with the agarose with a Pasteur pipette, and then mixed with Grace culture medium to diffuse the viruses in the culture medium. The single plaque-originated virus liquid was used to infect the Sf9 cells (cultured in a 25 cm² flask) cultured in the single layer. The infected cells were cultured at 27°C for 5 days, and the virus liquid was then recovered. 1 mL of the virus liquid was placed in a 1.5 mL Eppendorf tube and then centrifuged at 15,000 rpm at 4°C for 30 minutes to precipitate the virus particles. The precipitates were suspended in 200 µL of a TE (10 mM Tris, 0.1 mM EDTA) buffer solution, mixed with 50 µL of Lysis buffer solution (10% SDS, 1 mM EDTA), and incubated at 60°C for 20 minutes. Further, phenol / chloroform extraction and ethanol precipitation were performed to recover the virus DNA in the reaction solution. One-tenth of the recovered virus DNA was used to carry out a PCR amplification with primers BacF (SEQ ID NO: 15) and -BacR (SEQ ID NO: 16). Thus, it was identified that PARcDNA was contained in the DNA. The recombinant baculovirus for expressing the PAR was named VL-PAR. The virus liquid was properly diluted and then subjected to a plaque assay and the check of the titer. Further, the virus liquid was used to infect the Sf9 cells to obtain the virus liquid having a high concentration of about 1-5 × 10⁷ pfu (plaque forming unit) / mL. The virus liquid was stored at 4°C and -8°C.

### (3). Expression of PAR in insect cells with recombinant baculovirus

The above-obtained recombinant baculovirus VL-PAR for expressing the PAR was used to infect 1.0 × 10⁷Sf9 cells sowed on a 75 cm² flask at an MOI=2, and was then left and stationally cultured at 27°C for 40 hours. After the culture, the infected cells were recovered and then subjected to a calcium influx experiment to confirm the function of the PAR.

### [Example 5] Evaluation of calcium influx due to human AST with PAR-expressing insect cells

The recovered infected cells (about 5 × 10⁶ cells) were once washed with HEPES-Tyrode buffer solution-Ca²⁺ (-) pH 7.4, and then suspended in 5 mL of HEPES-Tyrode buffer solution (containing Fura2-AM, 1 µg / mL) to load the Fura2-AM on the cells at 27°C for 30 minutes. The suspension was centrifuged at 800 rpm at room temperature for 5 minutes to recover the cells. The cells were twice washed with HEPES-Tyrode buffer solution-Ca²⁺ (-) pH 7.4 and then resuspended in 5 mL of HEPES-Tyrode buffer solution-Ca²⁺ (-) pH 7.4. The cell suspension was mixed with 5 µL of 1M CaCl₂ to give a final concentration of 1 mM CaCl₂, and then incubated in a shaded state at room temperature for 5 minutes. The prepared Fura2-AM-loaded infected cells were dispensed into a 96 well plate in a volume of 100 µL / well, further subjected to the dispensation of HEPES-Tyrode buffer solution-C²⁺ (+) pH 7.4 in a volume of 80 µL / well, and then assayed with a Fluorescence Drug Screening System (made by Hamamatsu Photonics Co.). Namely, [intensity of 500 nm fluorescent light due to 340 nm exciting light] / [intensity of 500 nm fluorescent light due to 380 nm exciting light] (reflecting the concentration of calcium in the cells) was assayed.

AST (0.2 to 2 units / mL), trypsin (originated from bovine pancreas, 0.1 to 10 units / mL), human thrombin (0.1 to 10 units / mL), or SFLLRNamide (agonist peptide for PAR-1, 2), or SLIGRL (agonist peptide for PAR-2, 10 to 100 µM) was added to the cells to stimulate the cells, and the calcium concentrations in the cells were assayed with the passage of time. Consequently, the specific calcium influx was observed in the insect cells infected with the recombinant baculovirus for expressing the PAR. From the result, it was proved that the function-having PAR was expressed in the insect cells.

The Ca²⁺ influxes of PAR-1 to PAR-4 are shown below, respectively.

For PAR-1, the Ca²⁺ influx was observed at AST (2 units / mL), trypsin (0.1 to 10 units / mL), thrombin (0.1 to 10 units / mL), and SFLLRNamide (10 to 100 *µ*M).

For PAR-2, the Ca²⁺ influx was observed at AST (2 units / mL), trypsin (0.1 to 10 units / mL), thrombin (0.1 to 10 units / mL), SFLLRNamide (10 to 100 *µ*M), and SLIGRL (10 to 100*µ*M).

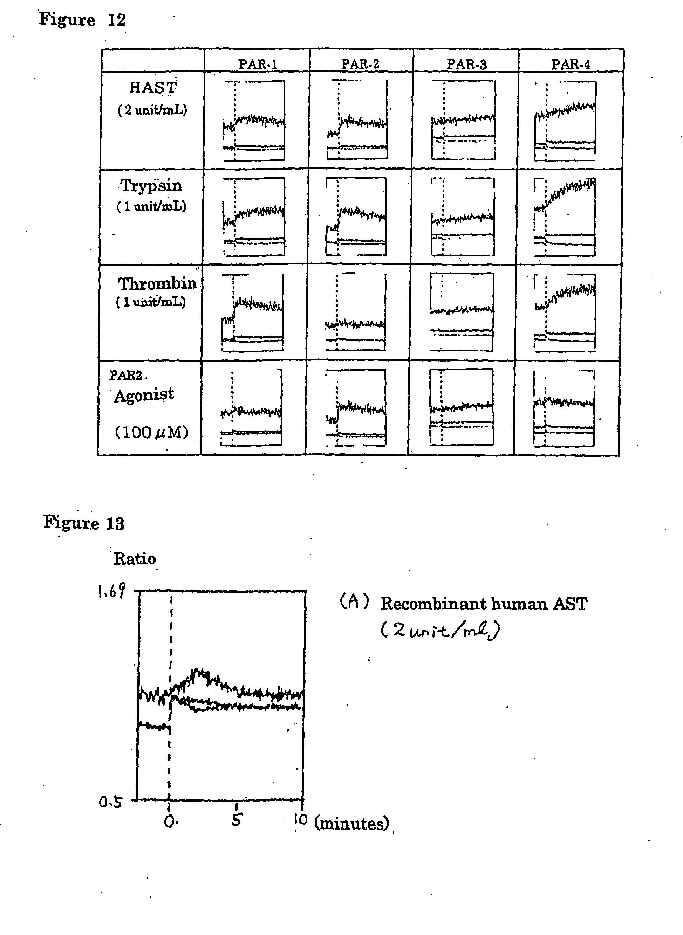

For PAR-4, the Ca²⁺ influx was observed at AST (2 units / ml), trypsin (0.1 to 10 units / mL), and thrombin (0.1 to 10 units / mL) (see Figure 12).

### [Example 6] Experiment for calcium influx due to AST with airway epithelial cell

### (1) Experiment for calcium influx due to AST with normal human airway epithelial cell

The Ca²⁺ influx due to the AST was examined with confluent normal epithelial cells adhered to three 10 cm dishes (purchased from BioWhittaker Corp.). The cells were peeled with a 10 mM EDTA / HEPES-Tyrode buffer solution for 10 minutes, recovered by a centrifugal treatment at 800 rpm at room temperature for 5 minutes, and then resuspended in 5 mL of HEPES-Tyrode buffer solution-Ca²⁺ (-) pH 7.4. The recovered cells (about 1.2 × 10⁶ cells) were suspended in 5 mL of HEPES-Tyrode buffer solution (1 µg / mL, containing Fura2-AM) to load the Fura2-AM to the cells at 37°C for one hour. The cells were recovered by a centrifugal treatment at 800 rpm at room temperature for 5 minutes, twice washed with HEPES-Tyrode buffer solution-Ca²⁺ (-) pH 7.4, and then resuspended in 1.3 mL of HEPES-Tyrode buffer solution-Ca²+ (-) pH 7.4. The cell suspension was mixed with 5 µL of 1M CaCl₂ to give a final concentration of 1 mM CaCl₂, and then incubated in a shaded state at room temperature for 5 minutes. The prepared Fura2-AM-loaded infected cells were dispensed into a 96 well plate in a volume of 100 µL / well, further subjected to the dispensation of HEPES-Tyrode buffer solution-Ca²⁺ (+) pH 7.4 in a volume of 80 µL / well, and then assayed with Fluorescence Drug Screening System (Hamamatsu Photonics Co.). Namely, [intensity of 500 nm fluorescent light due to 340 nm exciting light] / [intensity of 500 nm fluorescent light due to 380 nm exciting light] (reflecting the concentration of calcium in the cells) was assayed.

AST (2 units / mL), bovine pancreas-originated trypsin (1 to 100 units / mL), human thrombin (1 to 100 units / mL), SFLLRNamide, or SLIGRL (10 to 100 *µ*g) were added to stimulate the cells, and calcium concentrations in the cells were then assayed with the passage of time.

Consequently, a Ca²⁺ influx was observed at AST (2 units / mL), trypsin (10 to 100 units / mL), and SFLLRNamide (100 µ M) (see Figure 13).

### (2) Experiment for calcium influx due to AST with airway epithelial cell strain

The Ca²+ influx due to the AST was examined with respect to an airway epithelial cell strain BEAS·2B. The confluent cells adhered to six 10 cm dishes were used. The cells were peeled with a 10 mM EDTA / HEPES-Tyrode buffer solution for 10 minutes, recovered by a centrifugal treatment at 1,500 rpm at room temperature for 5 minutes, and then resuspended in 10 mL of HEPES-Tyrode buffer solution-Ca²+ (-) pH 7.4. The recovered cells (about 1.1 × 10⁷ cells) were suspended in 11 mL of HEPES-Tyrode buffer solution (1µg / mL, containing Fura2-AM) to load the Fura2-AM at 37°C for 30 minutes. The cells were recovered by a centrifugal treatment at 1,500 rpm at room temperature for 5 minutes, twice washed with HEPES-Tyrode buffer solution-Ca²+ (-) pH 7.4, and then resuspended in 11 mL of HEPES-Tyrode buffer solution-Ca²+ (-) pH 7.4. The cell suspension was mixed with 11 mL of 1M CaCl₂ to give a final concentration of 1 mM CaCl₂, and then incubated in a shaded state at room temperature for 5 minutes. The prepared Fura2-AM-loaded infected cells were dispensed into a 96 well plate in a volume of 100 u L / well, further subjected to the dispensation of HEPES-Tyrode buffer solution-Ca²⁺ (+) pH 7.4 in a volume of 80 µ L / well, and then assayed with Fluorescence Drug Screening System (Hamamatsu Photonics Co.). Namely, [intensity of 500 nm fluorescent light due to 340 nm exciting light] / [intensity of 500 nm fluorescent light due to 380 nm exciting light] (reflecting the concentration of calcium in the cells) was assayed.

AST (0.2 to 2 units / mL), bovine pancreas-originated trypsin (0.1 to 100 units / mL), human thrombin (0.1 to 100 units / mL), or SFLLRNamide, or SLIGRL (PAR against peptide, 10 to 100 µ M) were added to stimulate the cells, and calcium concentrations in the cells were then assayed with the passage of time.

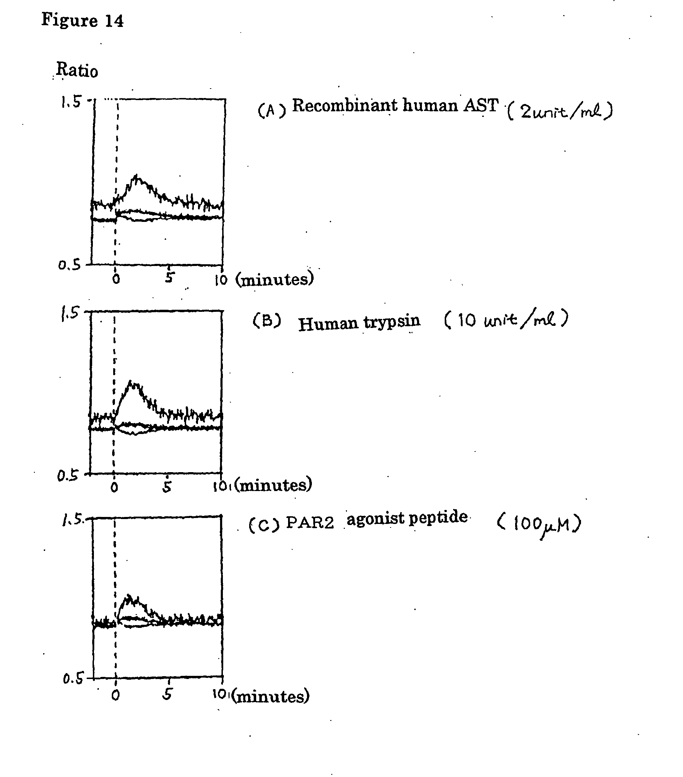

Consequently, the Ca²⁺ influx was observed at AST (2 units / mL), trypsin (10 to 100 units / mL), and SFLLRNamide (100 µ M) (see Figure 14).

### [Example 7] IL-8 release from airway epithelial cell strain (BEAS-2B) due to HAST

### 1). Cells

Tests were carried out with BEAS-2B (airway epithelial cell strain, purchased from ATCC). LHC-9 (produced by Biofluid Corp.) was used for usual cultures. When the cells were used for the test, the cells were suspended in LHC-8 (not containing hydrocortisone, produced by Biofluid Corp.), sowed on a 96 well plate (made by Falcon Corp.) coated with collagen (produced by Collagen Corp.) / BSA (produced by Boehringer Mannheim GmbH) / fibronectin (Sigma Corp.) in a density of 5.0 × 10⁴ cells / cm², and then cultured for 24 hours. After the normal take of the cells was identified, the cells were treated with a drug.

### 2) Drug treatment

HAST (100 mU / mL) and TNF- α (produced by R & D) (1 mg / mL) were reacted with a prescribed concentration of benzamidine or leupeptin in LHC-8 (not containing hydrocortisone) on ice for 30 minutes, and then heated at 37°C. The heated reaction solution was wholly exchanged by the cell culture solution in the wells to start the drug treatment. After the culture for 24 hours, the culture supernatant was recovered and stored at -80°C, until the assay of IL-8 production was carried out.

### 3) IL-8 concentration assay

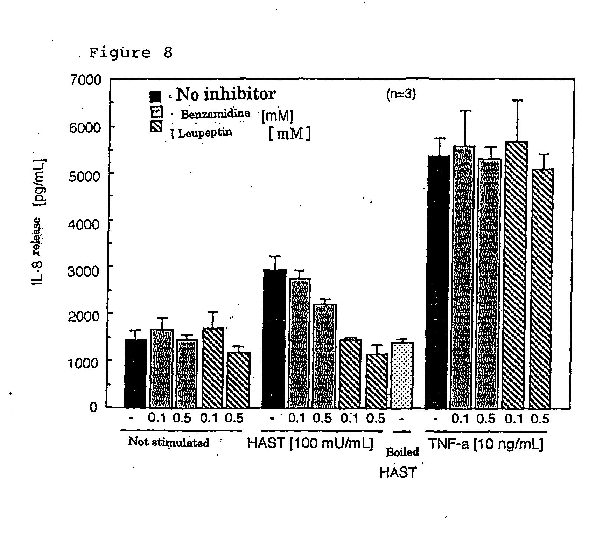

The concentration of IL-8 in the culture supernatant was assayed by a sandwich ELISA method using a commercially available antibody. Mouse MAb Anti-Human IL-8 Capture (produced by Biosource Corp.) adjusted with PBS in a concentration of 1 µg / mL was added to a 96 well EIA plate (made by Maxi Sorp. Nunc Corp.) in a volume of 50 µ L / well, and then allowed to stand at 4°C overnight to immobilize the antibody. The immobilized antibody was washed with 250 µ L / well of a washing buffer solution (PBS containing 0.05 % Tween 20 (produced by BioRad Corp.)) five times, mixed with 250 µL / well of a blocking buffer solution (PBS containing 1% BSA (produced by Boehringer Mannheim GmbH) and 0.05% sodium azide), and then allowed to stand at 4°C overnight to carry out the blocking treatment. The blocked product was washed with 250 µ L / well of a washing buffer solution five times, mixed with 50 µ L / well of the culture supernatant diluted with the blocking buffer solution 50 times or with IL-8 standard prepared with the blocking buffer solution, and reacted at room temperature for one hour. The reaction product was washed with 250 *µ*L / well of the washing buffer solution five times, mixed with 50 *µ*L / well of Mouse MAb Anti-Human IL-8 biotin (produced by Biosource Corp.) prepared with a diluting buffer solution (PBA containing 1% BSA and 0.05% of Tween 20) in a concentration of 0.05 *µ*g / mL, and reacted at room temperature for one hour. The reaction product was washed with 250 *µ*L / well of the washing buffer solution five times, mixed with 50 *µ*L / well of HRP-streptavidin (produced by Genzyme Corp.) diluted with the diluting buffer solution 6,000 times, and reacted for 15 minutes. The reaction product was washed with 250 µL/ well of the washing buffer solution five times, and then treated with 100 µL/ well of TMB Microwell peroxidase substrate (produced by KPL Corp.) for about 15 minutes to develop the color. 100 *µ*L / well of 1M sulfuric acid was added to stop the color-developing reaction, and then the absorbance of the product at 450 nm was immediately assayed with a ThermoMax microplate reader (made by Molecular Devices Corp.). The absorbance of each sample was regressed to a standard curve made from the absorbances of standards to calculate the concentration of IL-8 in the sample. The results are shown in Figure 8. Consequently, it was shown that the release of the IL-8 from the airway epithelial cells caused by the HAST was inhibited by inhibiting the activity of the HAST with the protease inhibitors.

### [Example 8] Transcriptional activation of IL-8 promoter by HAST

### 1) Cloning of IL-8 promoter domain

About 1.5 Kb and 162 bp of the promoter domains (Mukaida N. et al., J. Immunol., 143: 1366-1371, 1989) of IL-8 were cloned with LA-PCR (produced by Takara Shuzo Co.) using human genome DNA as a template, a forward primer: IL8-1481; CCCAGATCTGAATTCAGTAACCCAGGCATTATTTTATC, a forward primer: IL8-162; AACTTTGGATCCACTCCGTATTTGATAAGG, a reverse primer: IL8LUC-R; CATGTTTACACACAGTGAGAATGGTTCCTTCC. The PCR was carried out at 98°C for 20 sec, at 58°C for one minute and at 68°C for 5 minutes in 25 cycles. The obtained DNA fragments of the promoter domains of about 1.5 Kb and about 0.25 Kb were named IL8-1481 and IL8-162, respectively.

### 2) Construction of IL-8 reporter plasmid

A DNA fragment containing from ATG (translation-starting codon) to NarI site in the luciferase gene of pGL3-Basic (produced by Promega Corp.) was amplified by a PCR method using a forward primer: IL8LUC-F; CTGTGTGTAAACATGGAAGACGCCAAAAACATAAA and a reverse primer: LUC2087R; CGGGAGGTAGATGAGATGTG. This luciferase gene N-terminal DNA fragment was ligated to the IL8-1481 and the IL8-162 by a PCR method to obtain the PCR fragments IL8-1481-Luc (about 2 Kb) and IL8-162-Luc (about 800 bp). The PCR fragments were cleaved with BamHI and NarI and then inserted into the upper stream of the luciferase gene with the BglII and NarI of the pGL3-Basic to construct the reporter plasmids pGL-IL8-1418, pGL3-IL8-162.

### 3) Cells

Tests were performed with BEAS-2B (airway epithelial cell strain, purchased from ATCC). An ordinary culture was carried out with LHC-9 (produced by Biofluid Corp.). When the cell strain was used for the test, the cell strain was suspended in LHC-8 (not containing hydrocortisone, produced by Biofluid Corp.), sowed on a 96 well plate (produced by Falcon Corp.) coated collagen (produced by Collagen Corp.) / BSA (produced by Boehringer Mannheim Corp.) / fibronectin (produced by Sigma Corp.) in a density of 2.0 × 10⁴ cells / cm², and then cultured 24 hours. After it was identified that the cells normally took, the transient gene introduction was carried out.

### 4) Transient gene introduction

Transient gene introduction was carried out with LipofectAmine PLUS reagent (produced by Gibco Corp.). The culture solution in the 96 well plate was replaced by 50 µ L of fresh LHC-8 (not containing hydrocortisone, produced by Biofluid Corp.). A transfection culture medium containing 0.01 µ g of a control plasmid, 0.09 µ g of a reporter plasmid (pGL3-Basic or pGL3-IL8-1481 or pGL3-IL8-162), 0.5 µ L of LipofectAmine reagent (produced by Gibco Corp.) and 0.5 µ L of PLUS reagent (produced by Gibco Corp.) in 20 µ L of LHC-8 (not containing hydrocortisone, produced by Biofluid Corp.) was added in a volume of 20 µ L / well to start the gene introduction. Then, the culture was carried out in a CO₂ incubator for 48 hours.

### 5) Drug treatment

48 Hours later from the gene introduction, a drug treatment was carried out. The culture solution in the wells was replaced by LHC-8 (not containing hydrocortisone, produced by Biofluid Corp.) containing HAST (300 mU / mL) or TNF- α (produced by R&D Corp.)(1 ng / mL), and the culture was performed for 3 hours. Subsequently, the culture solution was removed, and the left cells were once washed with PBS and then supplied for the assay of luciferase activity.

### 6) Assay of luciferase activity

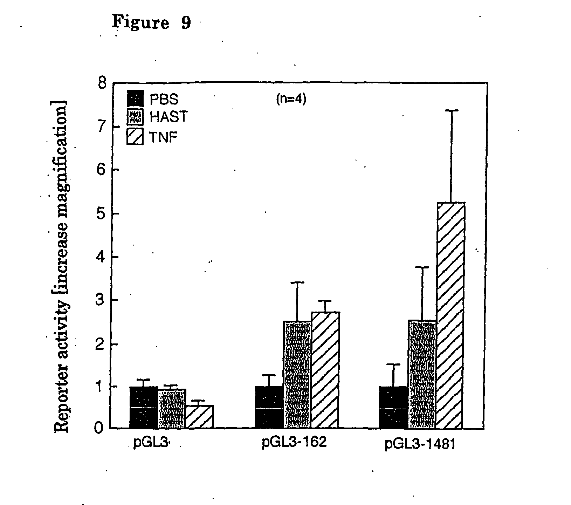

The luciferase activity was assayed with Pica Gene Dual · Renilla reniformis (produced by Toyo Ink Co.,). The cells were treated with HAST or TNF- α for three hours, subjected to the removal of the culture solution, once washed with PBS, and then lysed with 20 µL of a cell-lysing agent at room temperature for 15 minutes. The cell-lysed solution was recovered with a 1.5 mL micro tube, and then centrifuged at 15,000 rpm for 10 minutes. The supernatant was collected as a sample, and 20 µ L of the sample was added to a black 96 well plate (made by Sumitomo Bakelite Co.). First, 100 µ L of Pica Gene luminescent reagent II was added, and the luminescence due to the firefly luciferase was assayed with a luminometer (LUMINOUS CT-9000D (produced by DIA-IATRON Corp.)). Subsequently, 100 *µ*L of Renilla reniformis luminescent reagent was added, and the luminescence due to the Renilla reniformis luciferase was assayed with the luminometer (LUMINOUS CT-9000D (produced by DIA-IATRON Corp.)). A correction comprising dividing the obtained firefly luciferase activity by the Renilla reniformis luciferase activity was carried out to obtain the reporter activity (Relative Light Unit (R. L. U.)). Further, the reporter activity in each sample was subjected to a data analysis as an increased magnification based on an average value in a non-stimulated group. The results are shown in Figure 9. From the results, it was clarified that the HAST promoted the production of the IL-8 at a transcription level.

### [Example 9] Acquisition of mouse AST counter part cDNA

### (1). Acquisition of mouse airway cDNA

The airways of mice (C57 Black) were collected, and then homogenized in 1 mL / airway of ISOGEN. The obtained homogenate was mixed with 0.2 mL of chloroform in a vortex state, allowed to stand at room temperature for 2 to 3 minutes, and then centrifuged at a 12,000 rpm at 4°C for 10 minutes. The upper layer was transferred into a new tube, mixed with 0.5 mL of isopropyl alcohol, and then allowed to stand at room temperature for 10 minutes. The mixture was centrifuged at 15,000 rpm at 4°C for 15 minutes to precipitate the total RNA. The obtained pellet was well mixed with 1 mL of 75% ethanol and then centrifuged at 10,000 rpm at 4°C for 5 minutes. The obtained pellet was dried with air, dissolved in water free from DNase / RNase, and then preserved at -80°C.

The cDNA was synthesized with oligo (dT)₁₂₋₁₈ and 2.5 µ g of the obtained whole RNA originated from the mouse airways by the use of SuperScript^{™} Preamplification System for First Strand cDNA Synthesis Kit (made by GIBCO-BRL Corp.) according to an appended protocol.

### (2) Acquisition of AST PCR fragment from mouse airway cDNA

A homology comparison was carried out between the base sequences of human AST and mouse hepsin, and a forward primer and a reverse primer represented by SEQ ID NO: 17 and SEQ ID NO: 18, respectively, were produced. A PCR reaction was performed using the primers and the cDNA originated from the mouse airways as a template by the use of TaKaRa Taq^{™} polymerase (produced by Takara Shuzo Co.) according to an appended protocol. The PCR product was subjected to an agarose gel electrolysis. Consequently, an amplified band was recognized near to a target 450 bp. The amplified DNA fragments were cut out from the agarose gel, and then inserted into TA cloning vector. Escherichia coli was transformed with the vector to obtain the clone. A plasmid was prepared by an established method, and the base sequence of the inserted DNA fragment was determined. Consequently, it was clarified that not only the mouse Hepsin but also a gene exhibiting the homology with the human AST were cloned.

### (3) Acquisition of full-length mouse AST cDNA by 5'-RACE, 3'-RACE

Primers for 5'-RACE (Rapid Amplification of cDNA Ends) and 3'-RACE were synthesized on the partial base sequence obtained in (2). The primer sequences for the 1^{st} RACE are represented by SEQ ID NO: 19 and SEQ ID NO: 20, and the primer sequences for the 2^{nd} RACE are represented by SEQ ID NO: 21 and SEQ ID NO: 22. The 5'-RACE and the 3'-RACE were performed on the sequences with Mouse 15-day Embryo Marathon-Ready^{™} cDNA (produced by Clontech Corp., Swiss-Webster/NIH embryo). A PCR reaction was carried out with Advantage 2 Polymerase Mix (produced by Clontech Corp.) according to an appended protocol. The PCR product was subjected to an agarose gel electrophoresis, and the about 1.1 bp amplified fragment due to the 5'-RACE and the about 0.6 kbp amplified fragment due to the 3'-RACE were thus obtained. The amplified DNA fragments were cut out from the agarose gel, inserted into TA cloning vectors, which were used to transform Escherichia coli, thus obtaining several clones of the transformants, respectively. Plasmids were prepared from the transformants by an established method, and then the base sequences of the inserted DNA fragments were determined. As the result, it was identified that the base sequence starting from a sequence coincident with the partial base sequence obtained in (2) and having homology with the human AST in the 5'-direction and in the 3'-direction was extended. The determined perfect length mouse AST cDNA sequence and amino acid sequence are represented by SEQ ID NO: 4.

Further, in order to confirm whether mouse AST practically existing in a mouse airway has the same amino acid sequence or not, PCR primers (SEQ ID NO: 23 and SEQ ID NO: 24) recognizing the outside of a gene encoding the amino acid were synthesized. Advantage 2 Polymerase (produced by Clontech Corp.) was used to perform the PCR reaction of a mouse airway cDNA, and the base sequence of the amplified DNA fragment was determined. Consequently, it was found that the amino acid sequence coincided, and the cloning of the airway-originated mouse AST cDNA (SEQ ID NO: 6) was succeeded.

### (4) Comparison of homology with human AST

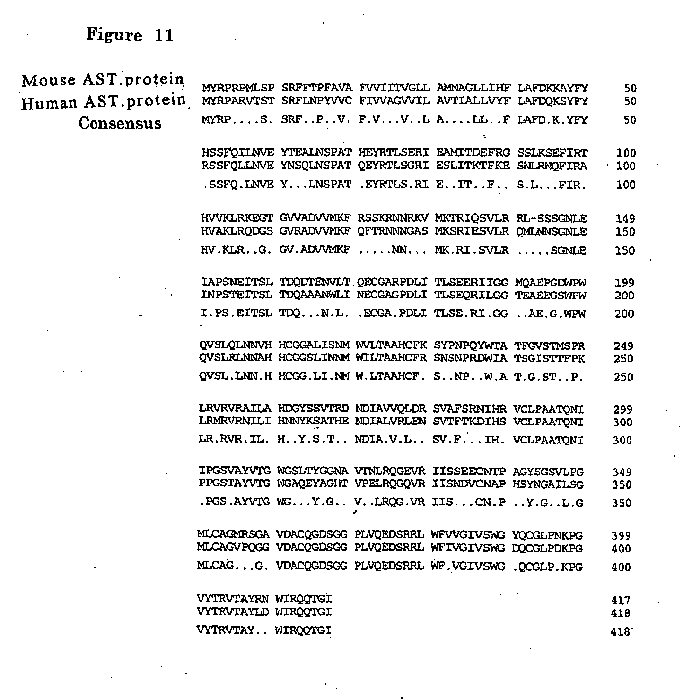

The amino acid homology comparison result between the human AST and the mouse AST is shown in Figure 11. The homology comparison was carried out with a gene-analyzing software (Gene Works; Teijin System Technology Co.). 66 % of the amino acid sequences were totally coincident with each other.

### [Example 10] Acquisition of rodent AST counter part cDNA

### (1). Synthesis of rodent airway cDNA

The airway of a hamster or guinea pig was picked, and then homogenized in about 5 mL of ISOGEN per 0.5 gram of the airway. The obtained homogenate was centrifuged at 3,000 rpm for 10 minutes, and 1 mL of the supernatant was collected. The supernatant was mixed with 0.2 mL of chloroform in a vortex state, allowed to stand at room temperature for 2 to 3 minutes, and then centrifuged at a 12,000 rpm at 4°C for 10 minutes. The upper layer was transferred to a new tube, mixed with 0.5 mL of isopropyl alcohol, and then allowed to stand at room temperature for 10 minutes. The mixture was centrifuged at 15,000 rpm at 4°C for 15 minutes to precipitate the total RNA. The obtained pellet was well mixed with 1 mL of 75% ethanol and then centrifuged at. 10,000 rpm at 4°C for 5 minutes. The obtained pellet was dried with air, dissolved in water free from DNase and RNase, and then preserved at -80°C.

The cDNA was synthesized using oligo (dT)₁₂₋₁₈ and 2.5 *µ*g of the obtained total RNA originated from the airways by the use of SuperScript^{™} Preamplification System for First Strand cDNA Synthesis Kit (made by GIBCO-BRL Corp.) according to an appended protocol.

### (2) Acquisition of AST PCR fragment from cDNA

A homology comparison was carried out between the base sequences of human AST and mouse hepsin, and a forward primer and a reverse primer represented by SEQ ID NO: 17 and SEQ ID NO: 18, respectively, were produced. A PCR reaction was performed using the primers and the cDNA originated from the airways as a template by the use of TaKaRa Taq^{™} polymerase (produced by Takara Shuzo Co.) according to an appended protocol. The PCR product was subjected to an agarose gel electrolysis. Consequently, an amplified band was recognized near to a target 450 bp. The amplified DNA fragments were cut out from the agarose gel, and then inserted into TA cloning vector. Escherichia coli was transformed with the vector to obtain the clone. A plasmid was prepared by an established method, and the base sequence of the inserted DNA fragment was determined. Consequently, it was clarified that a gene exhibiting the homology with the human AST were cloned.

### (3) Acquisition of full-length rodent AST cDNA by 5'-RACE, 3'-RACE

Primers for 5'-RACE and 3'-RACE were synthesized on the partial base sequence obtained in (2). On the other hand, the cDNA for the 5'-RACE and the 3'-RACE was synthesized with 1 *µ*g of the total RNA collected from the airways by the use of SMART-RACE Kit (made by Clontech Corp.). The 5'-RACE and the 3'-RACE were performed. A PCR reaction was carried out with Advantage. 2 Polymerase Mix (produced by Clontech Corp.) according to an appended protocol. The PCR products obtained by the 5'-RACE and the 3'-RACE were subjected to an agarose gel electrophoresis. The main amplified DNA fragments were cut out from the agarose gel, inserted into TA cloning vectors (produced by Invitrogen Corp.), which were used to transform Escherichia coli thus obtaining several clones of the transformants, respectively. Plasmids were prepared from the transformants by an established method, and then the base sequences of the inserted DNA fragments were determined. As the result, it was identified that the base sequence starting from a sequence coincident with the partial base sequence obtained in (2) and having homology with the human AST in the 5'-direction and in the 3'-direction was extended. The determined perfect length rodent AST cDNA sequence and amino acid sequence are shown. The guinea pig: SEQ ID NO: 36 and 37, the hamster: SEQ ID NO: 38 and 39.

### [Example 11] Acquisition of mammalian AST counter part cDNA

### (1). Acquisition of mammalian airway cDNA

The airway of rabbit, dog, pig, cattle, or Macaca irus was picked, and then homogenized in about 5 mL of ISOGEN per 0.5 g of the airways. The obtained homogenate was centrifuged at 3,000 rpm for 10 minutes, and 1 mL of the supernatant was collected. The supernatant was mixed with 0.2 mL of chloroform in a vortex state, allowed to stand at room temperature for 2 to 3 minutes, and then centrifuged at a 12,000 rpm at 4°C for 10 minutes. The upper layer was transferred into a new tube, mixed with 0.5 mL of isopropyl alcohol, and then allowed to stand at room temperature for 10 minutes. The mixture was centrifuged at 15,000 rpm at 4°C for 15 minutes to precipitate the total RNA. The obtained pellet was well mixed with 1 mL of 75% ethanol and then centrifuged at 10,000 rpm at 4°C for 5 minutes. The obtained pellet was dried with air, dissolved in water free from DNase and RNase, and then preserved at -80°C.

The cDNA was synthesized with oligo (dT)₁₂₋₁₈ and 2.5 µ g of the obtained total RNA originated from the airways by the use of SuperScript^{™} Preamplification System for First Strand cDNA Synthesis Kit (made by GIBCO-BRL Corp.) according to an appended protocol.

### (2) Acquisition of AST PCR fragment from airway cDNA

A homology comparison was carried out between the base sequences of human AST and rodent AST, and a forward primer represented by ASTS652F (SEQ ID NO: 40) and ASTS721F (SEQ ID NO: 41) and a reverse primer represented by ASTS1166 (SEQ ID NO: 42) and ASTS1211 (SEQ ID NO: 43) were produced. A PCR reaction was performed with the primers and the cDNA originated from the mammalian airways as a template by the use of Pyrobest DNA polymerase (produced by Takara Shuzo Co.) according to an appended protocol. The PCR product was subjected to an agarose gel electrolysis. Consequently, an amplified band was recognized from about 475 bp to about 590 bp. The amplified DNA fragment was cut out from the agarose gel, and then inserted into TA cloning vector. Escherichia coli was transformed with the vector to obtain the clone. A plasmid was prepared by an established method, and the base sequence of the inserted DNA fragment was determined. Consequently, it was clarified that a gene fragment exhibiting the homology with the human AST were cloned.

### (3) Acquisition of full-length mammalian AST cDNA by 5'-RACE, 3'-RACE

With respect the rabbit AST, primers for 5'-RACE and 3'-RACE were synthesized on the partial base sequence obtained in (2). With respect to the simian, dog, swine or bovine AST, SEQ ID NO: 40 for 3'-RACE and SEQ ID NO: 43 for 5'-RACE were used as primer sequences for the 1^{st} RACE, and SEQ ID NO: 41 for 3'-RACE and SEQ ID NO: 42 for 5'-RACE were used as primer sequences for the 2nd RACE. On the other hand, the cDNA for the 5'-RACE and the 3'-RACE was synthesized with 1 µ g of the total RNA. collected from each mammalian airway by the use of SMART-RACE Kit (made by Clontech Corp.). The 5'-RACE and the 3'-RACE were performed. A PCR reaction was carried out with Advantage 2 Polymerase Mix (produced by Clontech Corp.) according to an appended protocol. The PCR products obtained by the 5'-RACE and the 3'-RACE were subjected to an agarose gel electrophoresis. The main amplified DNA fragments were cut out from the agarose gel, inserted into TA cloning vectors (produced by Invitrogen Corp.), which were used to transform Escherichia coli, thus obtaining several clones of the transformants, respectively. Plasmids were prepared from the transformants by an established method, and then the base sequences of the inserted DNA fragments were determined. As the result, it was identified that the base sequence starting from a sequence coincident with the partial base sequence obtained in (2) and having homology with the human AST in the 5'-direction and in the 3'-direction was extended. Each determined perfect length mammalian AST cDNA sequence and amino acid sequence are shown. The pig: SEQ ID NO: 26 and 27, monkey: SEQ ID NO: 28 and 29, dog: SEQ ID NO: 30 and 31, cattle: SEQ ID NO: 32 and 33, rabbit: SEQ ID NO: 34 and 35.

### [Example 12] Assay of enzymatic activities of various mammalian ASTs (1) Preparation of plasmids for expressing various mammalian AST animal cells

A forward primer and a reverse primer were prepared on gene sequences encoding the N-terminal and C-terminal of each mammalian AST, and a cDNA encoding the full-length AST was prepared by a PCR amplification using the forward primer, the reverse primer, Pyrobest DNA polymerase (produced by Takara Shuzo Co.), and an airway cDNA as a template. On the other hand, a product obtained by cleaving pcDNA3 (produced by Invitrogen Corp.) with restriction enzymes NruI and KpnI, and the promoter domain of Elongation Factor-1α (EF-1α) from human genome were amplified by a PCR using a forward primer: GACTTCGCGACGTGAGGCTCCGGTGCCCGTC and a reverse primer: GACTGGTACCAAGCTTTTCACGACACCTGAAATGGAAG. The amplified fragment was cleaved with restriction enzymes NruI and KpnI, and then inserted into the above-described plasmid vector to construct the pEF9 expression vector having the promoter of the human EF-1 α . The PCR-amplified ATS fragment was inserted into a multi-cloning site in the downstream of the human EF-1α promoter of the above-described pEF9 expression vector to prepare each animal AST expression plasmid. The base sequences were identified with an automatic sequencer.

### (2) Enzymatic activity evaluation by transient expression in animal cultured cell

Human fetal kidney-originated 293 EBNA cells (Invitrogen Corp.) were cultured in a 10 cm schale, and the about 5.8 × 10⁶ cells were transfected with 28 µ g of each expression plasmid prepared in (1) in 56 µ L of LipofectAMINE 2000 (produced by GibcoBRL Corp.) according to an appended manual. Three days later, the cells were lysed and recovered in 5 mL of M-PER (produced by PIERCE Corp.) and then mixed with 5 mL of a buffer solution (50 mM Tris-HCl, 0.01% BSA, pH=8.6) for AST activity assay to give the cell lysate. The cell lysate was further diluted with the buffer solution for the AST activity assay ten times, and then supplied for the activity assay. 40 *µ*L of the above-described assay sample was mixed with 20 *µ*L of a synthetic substrate solution (a solution obtained by diluting a 10 mM Boc-Phe-Ser-Arg-MCA DMSO (dimethyl sulfoxide) solution with the buffer solution for the AST activity assay 50 times), and the increase of the fluorescence intensity per minute was assayed (fluorescence wavelength: 460 nm, excitation wavelength: 380 nm) to determine the enzymatic activity. The results are shown in the next table.

| | Cell lysate Fluorescence intensity ratio (Human : 100) | Sup Fluorescence intensity ratio (Human : 100) |
|---|---|---|
| Human | 100 | 100 |
| Monkey | 250 | 150 |
| Pig | 92 | |
| Dog | 40 | |
| Cattle | 16 | |
| Rabbit | 30 | |

### (3) Preparation of recombinant baculovirus for insect cell expression of mammalian AST

With respect to mouse AST, dog AST, or simian AST, Bac-to-Bac System (produced by GIBCO-BRL Corp.) was used according to an appended protocol to prepare a recombinant Bacmid for insect cell expression, and the Sf9 cell strain was transfected with the recombinant Bacmid to obtain the recombinant baculovirus.

### (4) Massive expression of mammalian AST by insect cell expression and enzyme activity assay

An insect cell strain Tn5 was massively cultured, and then infected with the above-described baculovirus. The infected insect cell strain was cultured for three days to express the recombinant mammalian AST. The expressed cell pellet was subjected to the same operations as in Example 1 to obtain the purified recombinant mammalian AST. The cleavage activity of the obtained recombinant mammalian AST was evaluated with three kinds of trypsin-like synthetic substrates. Consequently, it was found that the recombinant mammalian AST had an activity to cleave the synthetic substrates: Boc-Phe-Ser-Arg-pNA, Boc-Ser-Glu-Gln-Arg-pNA, and Boc-Ser-Lys-Gly-Arg-pNA similarly to that of HAST and thereby had the same trypsin-like activity as that of the HAST.

### [Example 13] Analysis of mucus glycoprotein synthesis by addition of AST (1) Synthesis of mucus glycoprotein with various EGFR ligands

### 1) Cell preparation

NCI-H292 cells (human pulmonary mucoepidermoid tumor cell strain, purchased from ATCC) were suspended in 10% FCS (Fetal Calf Serum)-containing RPMI-1640 (produced by Gibco Corp.), sowed on a Lab-Tek 8 well chamber slide (produced by Nunc Corp.) in a density of 6.0 × 10⁴ cells / cm², cultured for 72 hours, subjected to the replacement of the culture solution by RPMI-1640 containing 0.1% of BSA, and further cultured for 24 hours.

### Drug treatment

### 2) Drug treatment

On the day of the treatment, the culture solution of each well was replaced by RPMI-1640 (containing 0.1% BSA) containing EGF (5 ng / mL, produced by Becton Dickinson Corp.), HB-EGF (5 ng /mL, produced by R&D Corp.), TGF- α (5 ng / mL, produced by Peprotech Corp.), or PBS to start the treatment. Then, the cells were incubated at 37°C for 24 hours.

### 3) AB-PAS (Alcian Blue-Periodic Acid/Schiff) staining

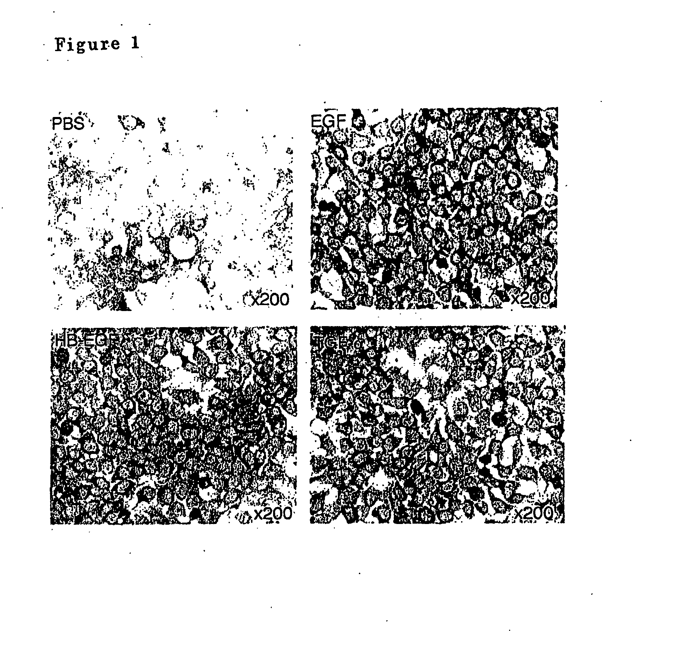

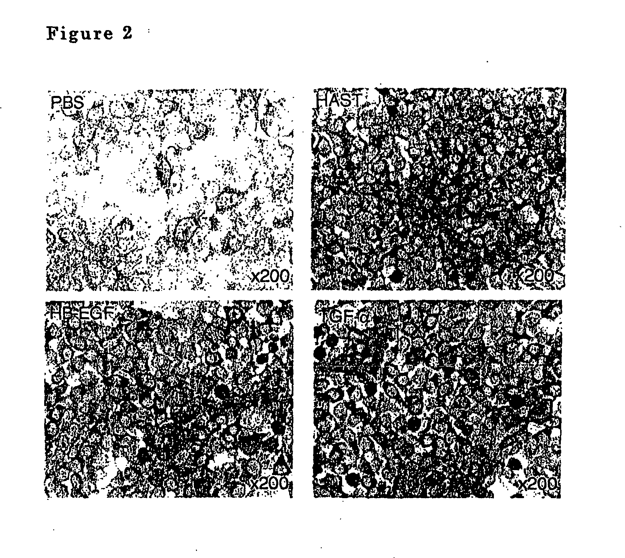

After the prescribed 24 hours treatment, the culture supernatant was removed. The residue was once washed with PBS, mixed with 4% paraformaldehyde / PBS, and then allowed to stand at room temperature for one hour to immobilize the cells. The immobilized cells were twice washed with Milli Q water and then stained with AB-PAS. The staining was carried out according to a book ("All of new dyeing methods" p136-150 (Iyaku Shuppansha)). After staining, the cells were sequentially immersed in 70, 80, and 100% ethanol for five minutes, respectively, to remove water, sealed in an Aquatex (produced by Merck Corp.), and then subjected to the observation of the staining image with an optical microscope. The results are shown in Figure 1, and Figure 2. From the results, it was clarified that all of HAST, TGF- α , EGF, and HB-EGF promoted the production of the mucus glycoprotein.

| Culture conditions | AB-PAS color intensity |
|---|---|
| PBS addition | - |
| EGF addition | ++++++ |
| HB-EGF addition | ++++++ |
| TGF- α addition | ++++++ |

| Culture condition | AB-PAS color intensity |
|---|---|
| PBS addition | - |
| HAST addition | ++++ |
| HB-EGF addition | ++++++ |
| TGF- α addition | ++++++ |

### (2) Mucus glycoprotein synthesis due to HAST and examination of HAST activation-inhibiting effect

### 1) Cell preparation

NCI-H292 cells (purchased from ATCC) were suspended in 10% FCS-containing RPMI-1640 (produced by Gibco Corp.), sowed on a Lab-Tek 8 well chamber slide (produced by Nunc Corp.) in a density of 6.0 × 10⁴ cells / cm², cultured for 72 hours, subjected to the replacement of the culture solution by RPMI-1640 containing 0.1% of BSA, and further cultured for 24 hours.

### 2) Drug treatment

On the day of the treatment, the culture solution of each well was replaced by RPMI-1640 (containing 0.1% BSA) containing PBS alone, PBS and leupeptin (100 µM, produced by SIGMA Corp.), HAST alone (300 mU / mL), HAST (300 mU / mL) and leupeptin (100 µM), or HAST thermally denatured at 99°C for 10 minutes. Thus, the cells were subjected to the start of the treatment, and further incubated at 37°C for 24 hours.

### 3) AB-PAS staining

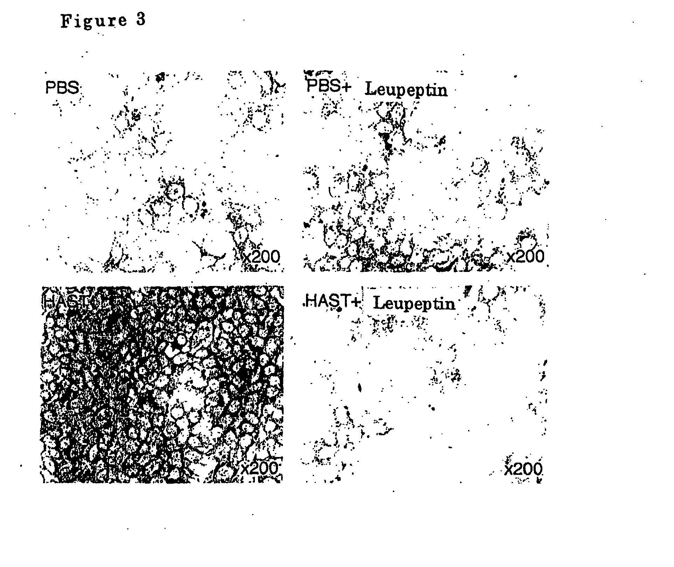

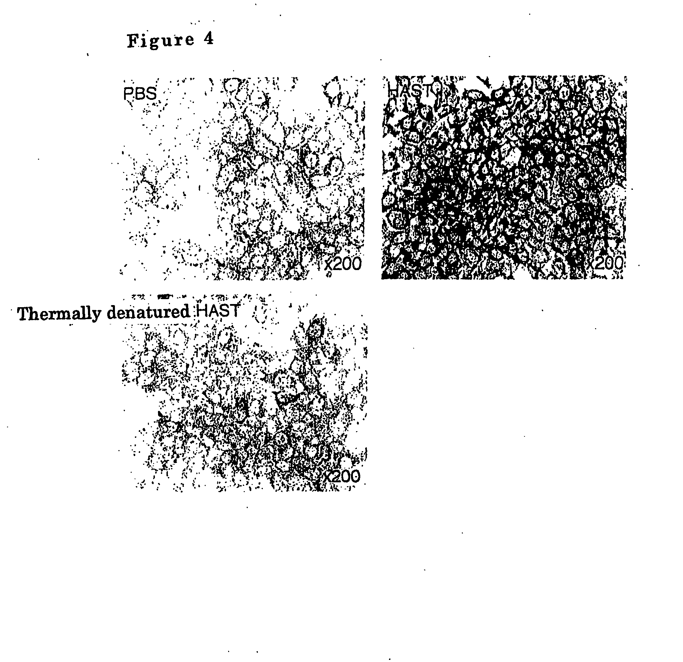

After the prescribed 24 hours treatment, the culture supernatant was removed. The residue was once washed with PBS, mixed with 4% paraformaldehyde / PBS, and then allowed to stand at room temperature for one hour to immobilize the cells. The immobilized cells were twice washed with Milli Q water and then staining with AB-PAS. The staining was carried out according to a book ("All of new dyeing methods" p136·150 (Iyaku Shuppansha)). After staining, the cells were sequentially immersed in 70, 80, and 100% ethanol for five minutes, respectively, to remove water, sealed in an Aquatex (produced by Merck Corp.), and then subjected to the observation of the staining image with an optical microscope. The results are shown in the next tables and Figures 3 and 4. From the results, it was clarified that the mucus glycoprotein production promotion caused by HAST could be controlled by inhibiting the activity of HAST with the protease inhibitor or by the thermal treatment.

| Culture conditions | AB-PAS color intensity |
|---|---|
| PBS addition | - |
| PBS / leupeptin addition | - |
| HAST addition | ++++++ |
| HAST / leupeptin addition | |
| PBS addition | - |
| HAST addition | ++++++ |
| Thermally denaturated HAST addition | - |

### [Example 14] Analysis of Muc5AC mRNA expression quantity due to addition of AST by real time PCR method

### (1) Examination of concentration-dependent Muc5AC production increase inhibition of inhibitor

### 1) Cells

NCI-H292 cells (purchased from ATCC) were suspended in 10% FCS-containing RPMI-1640(produced by Gibco Corp.), sowed on a 96 well plate (produced by Falcon Corp.) in a density of 6.0 × 10⁴ cells / cm², cultured for 72 hours, subjected to the replacement of the culture solution by RPMI-1640 containing 0.1% of BSA, and further cultured for 24 hours.

### 2) Drug treatment

Before added to the cells, leupeptin in various concentrations was reacted with 300 mU / mL of HAST on ice for 30 minutes, and further heated at 37°C. Then, the culture solution in the wells was wholly exchanged by the culture medium containing the HAST or leupeptin to start the treatment, and the treatment was carried out for 24 hours. HAST (300 mU / mL) thermally treated at 99°C for 10 minutes as a control, EGF alone (1 ng / mL), and a combination of EGF (5 ng / mL) with leupeptin (10⁻⁵ M) were also similarly treated. The number of the samples was 3.

### 3) RNA extraction

The RNA was extracted with RNeasy Mini Kit and RNase-free DNase of QIAGEN Corp. for each well. 30 µ L of the RNA solutions were obtained, respectively.

### 4) cDNA synthesis

OmniScript-RT of QIAGEN Corp. was used. A cDNA synthesis reaction was carried out with a random primer and 0.25 µ g of RNA to obtain 20 µ L of the cDNA solution. A part of the cDNA solution was diluted with water 5 times to obtain a sample for TaqMan Assay.

### 5) Taq Man Assay

An EGF treatment sample was continuously diluted five times to prepare a standard. PCR reactions were carried out with cDNA diluted five times and the standard as templates, respectively. Commercially available primers and a commercially available probe (produced by ABI Corp.) were used for β -actin. Further, 5'-TCAACGGAGACTGCGAGTACAC-3' as a forward primer, 5'-TCTTGATGGCCTTGGAGCA-3' as a reverse primer, and 5'-ACTCCTTTCGTGTTGTCACCGAGAACGTC-3' as a probe were used for Muc5AC. The primers and the probe were designed with Primer Express Ver 1.0. The primers were subcontracted and synthesized by Pharmacia Corp. The probe was also subcontracted and synthesized by ABI Corp. The PCR was carried out with TaqMan Universal PCR Mix (produced by ABI Corp.) and GeneAmp 5700 according to a procedure manual. The amplification reaction was carried out at 50°C for two minutes, at 95°C for 10 minutes, further at 95°C for 15 seconds and at 60°C for one minute totally in 40 cycles.

### 6) Analysis

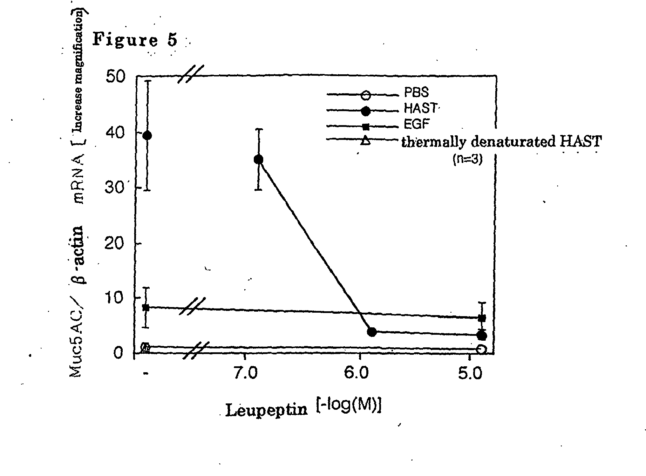

For both the Muc5AC and the *β* -actin, the expression quantities of the samples were relatively compared from the obtained Ct values and the standard straight line. Further, the values of the Muc5AC were divided by the values of the β -actin to correct the values. Finally, the values of the treated samples were divided by the average value of the untreated group to show the data as the increase magnifications, respectively. The results are shown in Figure 5.

From the results, it was shown that the Muc5AC gene expression promotion caused by the HAST could be controlled by inhibiting the activity of the HAST with the protease inhibitor or by the thermal treatment.

### (2) Examination of Muc5AC production increase inhibition of anti-EGF-R antibody

### 1) Cells

NCI-H292 cells (purchased from ATCC) were suspended in 10% FCS-containing RPMI-1640 (produced by Gibco Corp.), sowed on a 96 well plate (produced by Falcon Corp.) in a density of 6.0 × 10⁴ cells / cm², cultured for 72 hours, subjected to the replacement of the culture solution by RPMI-1640 containing 0.1% of BSA, and further cultured for 24 hours.

### 2) Drug treatment

① EGFR-neutralizing antibody treatment : the cultured product was preliminarily treated with 10 *µ*g / mL concentration of an EGFR-neutralizing antibody (clone LA1; produced by Transduction Corp.) or 10 *µ*g / mL concentration of normal mouse IgG1 (produced by R&D Corp.) at 37°C for 30 minutes, and the culture medium was then wholly exchanged by RPMI-1640 (containing 0.1% of BSA) containing normal mouse IgG1 and HAST (300 mU / mL), the EGFR-neutralizing antibody and HAST (300 mU / mL), normal mouse IgG1 and EGF (1 ng / mL), or the EGFR-neutralizing antibody and EGF (1 ng / mL) to start the treatment. The treatment was further carried out for 24 hours. The number of samples was 3.
② Leupeptin treatment HAST (300 mU mL) and EGF (1 ng / mL) were preliminarily treated with leupeptin (10·4 M) on ice for 30 minutes, heated at 37°C, and then wholly exchanged by the culture medium in the wells to start the treatment. The treatment was further carried out for 24 hours. The number of the samples was 3.

### 3) RNA extraction

The RNA was extracted with RNeasy Mini Kit and RNase-free DNase of QIAGEN Corp. for each well. 30 *µ*L of the RNA solutions were obtained, respectively.

### 4) cDNA synthesis

OmniScript-RT of QIAGEN Corp. was used. A cDNA synthesis reaction was carried out with a random primer and 0.25 µg of the RNA to obtain 20 µL of the cDNA solution. A part of the cDNA solution was diluted with water 5 times to obtain a sample for TaqMan Assay.

### 5) TaqMan Assay

An EGF treatment sample was continuously diluted five times to prepare a standard. PCR reactions were carried out with cDNA diluted five times and the standard as templates, respectively. Commercially available primers and a commercially available probe (produced by ABI Corp.) were used for β -actin. Further, 5'-TCAACGGAGACTGCGAGTACAC-3' as a forward primer, 5'-TCTTGATGGCCTTGGAGCA-3' as a reverse primer, and 5'-ACTCCTTTCGTGTTGTCACCGAGAACGTC-3' as a probe were used for Muc5AC. The primers and the probe were designed with Primer Express Ver 1.0. The primers were subcontracted and synthesized by Pharmacia Corp. The probe was also subcontracted and synthesized by ABI Corp. The PCR was carried out with TaqMan Universal PCR Mix (produced by ABI Corp.) and GeneAmp 5700 according to a procedure manual. The amplification reaction was carried out at 50°C for two minutes, at 95°C for 10 minutes, further at 95°C for 15 seconds and at 60°C for one minute totally in 40 cycles.

### 6) Analysis

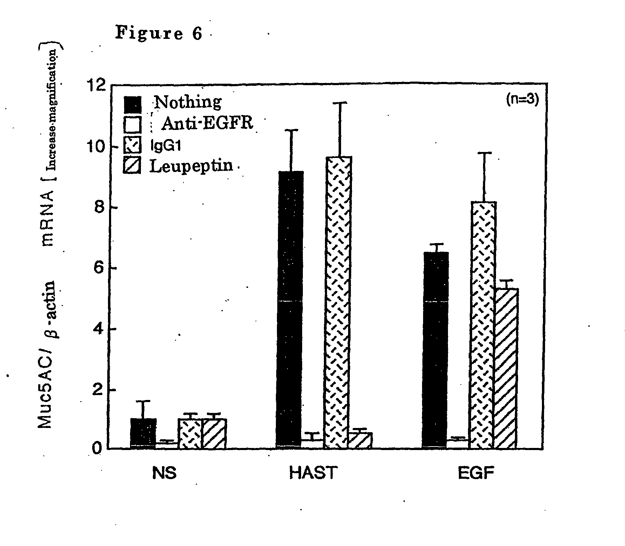

For both the Muc5AC and the β -actin, the expression quantities of the samples were relatively compared from the obtained Ct values and the standard straight line. Further, the values of the Muc5AC were divided by the values of the β -actin to correct the values. Finally, the values of the treated samples were divided by the average value of the untreated group to show the data as the increased magnifications, respectively. The results are shown in Figure 6.

From the results, it was shown that the Muc5AC gene expression promotion caused by the HAST could be controlled by inhibiting the activity of the HAST with the protease inhibitor or by the thermal treatment. Further, it was shown that the HAST promoted the Muc5AC gene expression through the EGFR.

### (3) Comparison with trypsin, esterase, tryptase

### 1) Cells

NCI-H292 cells (purchased from ATCC) were suspended in 10% FCS-containing RPMI-1640 (produced by Gibco Corp.), sowed on a 96 well plate (produced by Falcon Corp.) in a density of 6.0 × 10⁴ cells / cm², cultured for 72 hours, subjected to the replacement of the culture solution by RPMI-1640 containing 0.1% of BSA, and further cultured for 24 hours.

### 2) Drug treatment

On the day of the treatment, the culture solution of each well was replaced by RPMI-1640 (containing 0.1% BSA) containing HAST (0.3 mU / mL), esterase (0.1, 1, 10 U / mL), trypsin (0.1, 1, 10 U / mL) and tryptase (0.1, 1, 10 U / mL), and the treatment was carried out for 24 hour.

### 3) RNA extraction

The RNA was extracted with RNeasy Mini Kit and RNase-free DNase of QIAGEN Corp. for each well. 30µ L of the RNA solutions were obtained, respectively.

### 4) cDNA synthesis

OmniScript-RT of QIAGEN Corp. was used. A cDNA synthesis reaction was carried out with a random primer and 0.25 *µ*g of the RNA to obtain 20 *µ*L of the cDNA solution. A part of the cDNA solution was diluted with water 5 times to obtain a sample for TaqMan Assay.

### 5) TaqMan Assay

An EGF treatment sample was continuously diluted five times to prepare a standard. PCR reactions were carried out with cDNA diluted five times and the standard as templates, respectively. Commercially available primers and a commercially available probe (produced by ABI Corp.) were used for β -actin. Further, 5'-TCAACGGAGACTGCGAGTACAC-3' as a forward primer, 5'-TCTTGATGGCCTTGGAGCA- 3' as a reverse primer, and 5'-ACTCCTTTCGTGTTGTCACCGAGAACGTC-3' as a probe were used for Muc5AC. The primers and the probe were designed with Primer Express Ver 1.0. The primers were subcontracted and synthesized by Pharmacia Corp. The probe was also subcontracted and synthesized by ABI Corp. The PCR was carried out with TaqMan Universal PCR Mix (produced by ABI Corp.) and GeneAmp 5700 according to a procedure manual. The amplification reaction was carried out at 50°C for two minutes, at 95°C for 10 minutes, further at 95°C for 15 seconds and at 60°C for one minute totally in 40 cycles.

### 6) Analysis

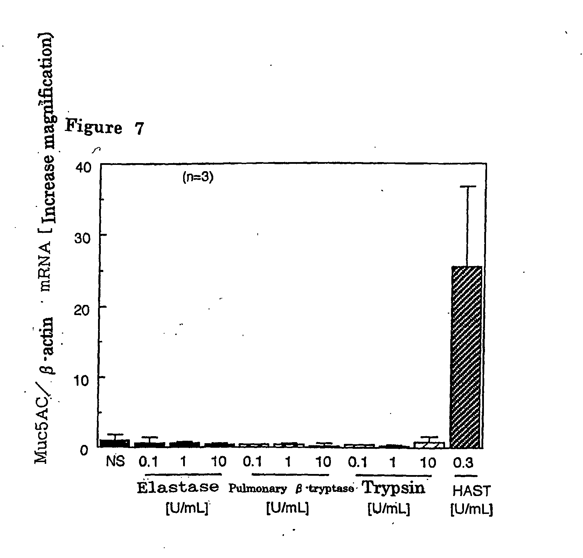

For both the Muc5AC and the β -actin, the expression quantities of the samples were relatively compared from the obtained Ct values and the standard straight line, respectively. Further, the values of the Muc5AC were divided by the values of the β actin to correct the values. Finally, the values of the treated samples were divided by the average value of the untreated group to show the data as the increase magnifications, respectively. The results are shown in Figure 7.

From the results, it was found that the Muc5AC gene expression was promoted by a mechanism which the trypsin, the tryptase and the esterase did not have.

### [Example 15] Cleavage of EGF-L (EGF and HB-EGF) on surface of cell membrane with HAST

### (1) Cleavage of EGF and HB-EGF antigen on surface of H292 cell membrane with HAST

### 1) Cells

NCI-H292 cells (purchased from ATCC) were cultured on a 10 cm dish in 10% FCS-containing RPMI-1640 until to reach a confluent state, subjected to the replacement of the culture medium by RPMI-1640 containing 0.1°/ of BSA, and further cultured for 24 hours. The cultured cells were washed with PBS, treated and floated in PBS containing 5 mM of EDTA at 37°C for five minutes, and then suspended in 0.1 %BSA-containing RPMI-1640 in a density of 1.0×10⁷ cells /mL.

### 2) Drug treatment

The above-described cell suspension and 0.1% BSA-containing RPMI-1640 containing HAST in a double concentration (600 mU / mL) were mixed with each other in equivalent amounts, respectively, and reacted with each other at 37°C for 30 minutes (final cell concentration: 5.0 × 10⁶ cells / mL, .final HAST concentration: 300 mU / mL). The reaction solution was mixed with ice-cooled 1°/ FCS-containing PBS in a volume of 5 times that of the reaction solution to stop the reaction, and the treated solution was then supplied for a surface antigen analysis.

### 3) Surface antigen analysis

The cells subjected to the prescribed treatments were suspended in 1% FCS-containing PBS in a density of 1.0 × 10⁷ cells / mL. 100 µL of the prepared cell suspension was mixed with a primary antigen (EGF back ground dyeing: 1 µg of normal mouse IgG1 (produced by R&D Corp.), EGF dyeing: 1µg of an anti-EGF mouse monoclonal antibody (produced by R&D Corp.), HB-EGF back ground dyeing: free from the primary antigen, HB-EGF dyeing: 2 µg of an anti-HB-EGF goat polyclonal antibody (produced by R&D Corp.)) and reacted on ice for 30 minutes. The reaction product was once washed with 5 mL of 1% FCS-containing PBS, mixed with 2 µg of a secondary antigen (EGF back ground dyeing and EGF dyeing: FITC conjugated F(ab')₂ fragment to mouse IgG (produced by Dako Corp.), HB-EGF back ground dyeing and HB-EGF dyeing: Swine Anti-Goat Ig's Fluorescein Conjugate (produced by KPL Corp.)), and then reacted on ice for 30 minutes. The reaction solution was once washed with 5 mL of 1% FCS-containing PBS, and then mixed with Cell Fix (produced by Becton Dickinson Corp.) to immobilize the cells. The expression of the surface antigen was assayed with FACS Calibur (produced by Becton Dickinson Corp.) and analyzed with Cell Quest (produced by Becton Dickinson Corp.). Average fluorescence intensities were shown in the next table as the indicators of expression intensities.

From the results, it was found that the HAST cleaved proEGF and proHB-EGF on the surfaces of the cell membranes.

**The changes of EGF, HB-EGF expression intensities on the surfaces of the cell membranes by the HAST treatment**

| | Background | PBS | HAST |
|---|---|---|---|
| EGF | 4.62 | 7.26 | 4.88 |
| HB-EGF | 4.5 | 8.72 | 6.94 |

### (2) Examination ofAST activity-inhibiting effect

### 1) Cells

NCI-H292 cells (purchased from ATCC) were cultured on a 10 cm dish in 10% FCS-containing RPMI-1640 until to reach a confluent state, subjected to the replacement of the culture medium by RPMI-1640 containing 0.1% of BSA, and further cultured for 24 hours. The cultured cells were washed with PBS, treated and floated in PBS containing 5 mM of EDTA at 37°C for five minutes, and then suspended in 0.1 %BSA-containing RPMI-1640 in a density of 1.0 × 10⁷cells / mL.

### 2) Drug treatment

HAST (600 mU / mL) was reacted with leupeptin (10 *µ* M) or BB1101 (5 µ M) in 0.1 %BSA-containing RPMI-1640 on ice for 30 minutes, and the reaction solution was mixed with the equivalent amount of a cell suspension preliminarily treated with leupeptin (10 µ M) or BB1101 (5 µ M) at 37°C for 30 minutes, and further treated at 37°C for 30 minutes (final HAST concentration: 300 mU / mL, final leupeptin concentration: 10 µ M, final BB1101 concentration: 5 µM, final cell concentration: 5.0 × 10⁶ cells / mL). The reaction solution was mixed with ice-cooled 1% FCS-containing PBS in an amount of five times that of the reaction solution to stop the reaction, and then supplied for the surface antigen analysis.

### 3) Surface antigen analysis

The cells subjected to the prescribed treatments were suspended in 1% FCS-containing PBS in a density of 1.0 × 10⁷ cells / mL. 100 *µ*L of the prepared cell suspension was mixed with a primary antigen (EGF back ground dyeing: 1*µ*g of normal mouse IgG1 (produced by R&D Corp.), EGF dyeing: 1*µ*g of an anti-EGF mouse monoclonal antibody (produced by R&D Corp.), HB-EGF back ground dyeing: free from the primary antigen, HB-EGF dyeing: 2 *µ*g of an anti-HB-EGF goat polyclonal antibody (produced by R&D Corp.)) and reacted on ice for 30 minutes. The reaction product was once washed with 5 mL of 1% FCS-containing PBS, mixed with 2 µ g of a secondary antigen (EGF back ground dyeing and EGF dyeing: PerCP conjugated F(ab')₂ fragment to mouse IgG (produced by Becton Dickinson Corp.), or Swine Anti-Goat Ig's Fluorescein Conjugate), and then reacted on ice for 30 minutes. The reaction solution was once washed with 5 mL of 1% FCS-containing PBS, and then mixed with Cell Fix (produced by Becton Dickinson Corp.) to immobilize the cells. The expression of the surface antigen was assayed with FACS Calibur (produced by Becton Dickinson Corp.) and analyzed with Cell Quest (produced by Becton Dickinson Corp.). Average fluorescence intensities were shown in the next table as the indicators of expression intensities.

From the results, it was found that the HAST-caused cleavage of proEGF and proHB-EGF on the surfaces of the cell membranes was inhibited by inhibiting the activity of the HAST with the protease inhibitor.

**The changes of EGF, HB-EGF expression intensities on the surfaces of the cell membranes by the HAST treatment**

| | Background | PBS | HAST | HAST + leupeptin | HAST + BB01 |
|---|---|---|---|---|---|
| EGF | 8.44 | 10.79 | 8.33 | 10.18 | 7.49 |
| HB-EGF | 5.58 | 8.61 | 6.26 | 7.3 | 6.65 |

### (3) Evaluation of EGF-L (human HB-EGF) excision due to HAST

### 1) Preparation of human HB-EGF animal cell expression plasmid

A cDNA was synthesized with SuperScript™ Preamplification System for First Strand cDNA Synthesis Kit (produced by GIBCQ-BRL Corp.) using 2.5 *µ*g of human airway PolyA+RNA (produced by Clontech Corp.) and oligo(dT)₁₂₋₁₈ according to an appended protocol. A cDNA fragment encoding a Jaxtamemrane Domain-containing domain was obtained from the signal peptide of the N-terminal of human HB-EGF with the prepared human airway cDNA as a template by an amplification using PyorobestPCR (produced by Takara Shuzo Co.). On the other hand, human placenta alkali phosphatase cDNA was obtained from pSEAO2-Control vector (produced by Clontech Corp.) by an amplification with the Pyorobest PCR.

A DNA fragment: HBEGF (ΔTM)-ALP (SEQ ID NO: 44) obtained by adding human placenta alkali phosphatase to the C-terminal of Jaxtamemrane Domain was prepared by ligating a DNA fragment encoding a domain containing Jaxtamembrane Domain from the signal peptide of the N-terminal of human HB-EGF to a DNA fragment encoding human placenta alkali phosphatase by a PCR method.

A human HBEGF-expressing plasmid: pEF9-HBEGF( Δ TM)-ALP was prepared by inserting the above-described HBEGF into the multi-cloning site of the pEF9 vector prepared in Example 12 (1).

### 2) Expression of HBEGF in human cultured cells

293EBNA cell strain was sowed in a density of 9 × 10⁶ cells / 10 cm dish, cultured overnight, and then transfected with the human HBEGF-expressing plasmid: pEF9-HBEGF (ΔTM)-ALP prepared in 1). 12 µ g of the pEF9-HBEGF(Δ TM)-ALP was subjected to a lipofection with 75 µ L of Lipofectamine 2000 (produced by GIBCO-BRL). The lipofection was carried out according to an appended manual. 72 hour later, the cells were peeled with a pipette, and then centrifuged at 3000 rpm at 4°C for five minutes. The lysate was dissolved in 12 mL of M-PER (produced by PIERCE Corp.), subjected to an SDS-polyacrylamide gel electrophoresis under a non-reducing condition, and then blotted on a nitrocellulose membrane (produced by BIO-RAD Corp.).

The product blotted on the nitrocellulose membrane was treated with 3% BSA-containing PBS at 4°C overnight and then reacted with the anti-human HB-EGF antibody (produced by GENZYME Corp., an antibody recognizing an EGFR-binding site) diluted with 1% BSA-containing PBS in a concentration of 1 *µ*g / mL, at room temperature for one hour. The nitrocellulose membrane was washed with 0.05% Tween 20-containing PBS three times, and then reacted with Swine Anti-Goat Ig's-ALP antibody (produced by BIOSOURCE Corp.) diluted with 1% BSA-containing PBS in a concentration of 1 *µ*g / mL at room temperature for one hour. The nitrocellulose membrane was washed with 0.05% Tween 20-containing PBS three times, further once washed with TBS, and then reacted with NBT (nitroblue tetrazolium chloride) BCIP (5-bromo-4-chloro-3-indolylphosphate p-toluidine salt, produced by PIERCE Corp.) at room temperature for five minutes to develop the color.

### 3) Activity of cleavage of HBEGF(Δ TM)-ALP due to HAST

100 *µ*L of the lysate solubilized with the M-PER was mixed with 10 *µ*L of a recombinant HAST (1.56 U / mL) and incubated at 37°C overnight. The sample was subjected to the western blotting treatment similarly as described above. Consequently, the following facts were identified in the HAST-added sample. A band showing reactivity with the anti-human HB-EGF anti-body (an antibody recognizing an EGFR-binding site) was shifted to a low molecular weight. A mature type having the EGF domain of the HB-EGF was cut out. The band was shifted to a position of about 12 kD.

### [Example 16] Analysis of tyrosine phosphorylation of EGFR due to HAST

### 1) Cells

NCI-H292 cells (purchased from ATCC) were suspended in 10% FCS-containing RPMI-1640 (produced by Gibco Corp.) and then sowed on a 12 well plate (made by Falcon Corp.) in a density of 6.0 × 10⁴ cells / cm². The cells were cultured for 72 hours, subjected to the replacement of the culture medium by 0.1% BSA-containing RPMI-1640, and further cultured for 24 hours.

### 2) Drug treatment

On the day of the treatment, the culture solution of the wells was replaced by RPMI-1640 (containing 0.1% BSA) containing HAST (0.3 U / mL), EGF (1 *µ*g / mL), and the treatments were carried out for 30, 60, 120, 180, 240 minutes, respectively.

### 3) Preparation of cell lysate

The cells treated with the HAST or the EGF for the prescribed time were twice washed with ice-cooled PBS, mixed with 500 µ L of an ice-cooled Lysis buffer solution (50 mM Tris-HCl, pH 7.4, 5 mM EDTA, 1% Triton X-100 (Sigma), 150 mM NaCl, 10% glycerol, 1% Protease inhibitor Cocktail (Sigma), 1% Phosphatase Inhibitor Cocktail (Sigma)), allowed to stand on ice for 15 minutes, and then recovered in a micro tube. The recovered product in the micro tube was centrifuged at 15,000 rpm at 4°C for 10 minutes, and the supernatant was recovered as a sample. The protein concentration of the sample was assayed with BioRad Protein Assay (produced by Bio Rad Corp.), and properly diluted with the Lysis buffer solution so that the protein concentrations in all the samples become 1.48 mg / mL. Subsequently, 406 µ L of the diluted solution was used for the following immunoprecipitation.

### 4) Immunoprecipitation

The above-described prepared cell lysate (1.48 mg / mL, 406 µ L) was mixed with 8 µL of agarose conjugated mouse IgG1 (produced by Santa Cruz Corp.) and 20 µL of Protein G Plus Agarose (produced by Oncogene Corp.), gently stirred at 4°C for two hours, and then centrifuged at 3,000 rpm at 4°C for one minute. The supernatant was recovered, mixed with 4 µg of an anti-EGFR antibody (Clone LA1, produced by Transduction Corp.), gently stirred at 4°C for one hour, further mixed with 20 µL. of Protein G Plus Agarose (produced by Oncogene Corp.), gently stirred at 4°C overnight, and then centrifuged at 3,000 rpm at 4°C for one minute. After the supernatant was removed, the precipitates were washed with 1 mL of an ice-cooled washing buffer solution (10 mM Tris-HCl, pH 7.4, 100 mM NaCl, 0.05% Tween-20 (BioRad) four times, further once washed with a washing buffer solution not containing Tween-20, and then centrifuged at 3,000 rpm at 4°C for one minute. The supernatant was removed, and the precipitates were mixed with 25 µL of a SDS sample buffer solution (125 mM Tris-HCl, pH 6.8, 4% SDS, 10% glycerol, 0.04% bromophenol blue, 4% 2-mercaptoethanol), heated at 99°C for five minutes, and then centrifuged at 3,000 rpm at room temperature for one minute. The supernatant was recovered and used as a sample for a western blotting treatment.

### 5) Western blotting

The proteins separated by the SDS-polyacrylamide gel electrophoresis were transferred to a nylon membrane (Immobilon PVDF membrane, produced by Millipore Corp.) with a semi-dry transfer (produced by Ato Corp.). The transferred proteins were blotted in a blotting buffer solution (1% BSA (produced by Boehringer Mannheim GmbH) -containing washing buffer solution) at 4°C overnight. Subsequently, the blotted proteins were reacted with a HRP-anti-phosphotyrosine antibody diluted with a blocking buffer solution 2,500 times, at room temperature for one hour. The membrane was washed with a washing buffer solution six times, and then subjected to the development of color.

### 6) Color development, detection

ECLplus (produced by Amasharm Pharmacia Biotech Corp.) was used. After washed, the membrane was immersed in an. ECL color-developing solution for five minutes, subjected to the removal of the excessive color-developing solution from the membrane, sealed in Saran Wrap, exposed to an X-ray film for about 2 to 5 minutes, developed, and then subjected to the analysis of the bands of the tyrosine phosphorylated proteins.

### 7) Reprobing

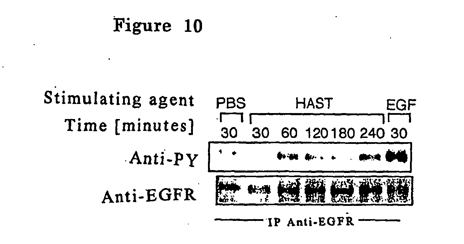

After exposed to the X-ray film, the membrane was washed with a washing buffer solution for five minutes, three times. The membrane was shaken in Restore reagent (produced by PIERCE Corp.) at 37°C for 15 minutes to remove the antibody. The membrane was washed with a washing buffer solution for five minutes, three times, subjected to the development of the color by the above-described method with ECLplus, and then exposed to an X-ray film for 10 minutes. After it was identified that any band was not left in the developed X-ray film, the membrane was washed with a washing buffer solution for five minutes, three times, further immersed in a blocking buffer solution, and allowed to stand at 4°C overnight to block. The blocked product was then reacted with an anti-EGFR antibody (Clone 13, produced by Transduction Corp.) diluted with a blocking buffer solution in a concentration of 0.1*µ* g / mL, at room temperature for one hour. The product was washed with a washing buffer solution six times, then reacted with an HRP-labeled anti-mouse IgG antibody (produced by Amersham Corp.) diluted with a blocking buffer solution 6,000 times, at room temperature for one hour, washed with a washing buffer solution six times, and then subjected to the color development using ECL plus by the above-described method. The color-developed band of EGFR was detected. The results are shown in Figure 10.

Thus, it was clarified that HAST activated EGFR by the promotion of the tyrosine phosphorylation of EGFR.

### [Example 17] Acquisition of anti-AST polyclonal antibody

### (1) Acquisition of anti-peptide antibody

A 20 moiety peptide (SEQ ID NO: 25) ranged from isoleucine at the 187^{th} position of human AST to a moiety at the 205^{th} position and having cysteine at the N-terminal was chemically synthesized with a peptide synthesizer (Applide Biosystems Model 431A). The synthesized peptide was dissolved (10 mg / mL) in a 10 mM phosphate buffer solution (pH 7.5) and then incubated with 10 mg of maleimide-activated hemocyanin (produced by Boehringer Mannheim Biochemica Corp) at 25°C for two hours. The reaction solution was dialyzed with a 10 mM phosphate buffer solution (pH 7.5). The hemocyanin-bound peptide was subcutaneously administered in a rabbit (0.5 mg / administration). The administration was repeated six times at two week intervals. The whole blood was collected, and the serum was prepared. The serum was purified with a sepharose 4B column (made by Pharmacia Corp.) to obtain the anti-AST polyclonal antibody (anti-N19 PAb).

### (2) Acquisition of anti-recombinant AST antibody

The recombinant AST (40 µ g / administration) prepared in the Example 1 and Freund's complete adjuvant (produced by BACTO Corp., 1:1) were subcutaneously administered into a rabbit at two week intervals. After the treatment was carried out four times, the whole blood was collected to obtain an antiserum. IgG was purified from the serum with protein A sepharose 4B column (made by Pharmacia Corp.) according to a conventional method to obtain the anti-AST polyclonal antibody (anti-rAST PAb).

### [Example 18]. Preparation of anti-AST monoclonal antibody

### (1) Immunization of mouse with recombinant AST

The recombinant AST prepared in Example 1 and Freund's complete adjuvant (produced by BACTO Corp., 1:1) were intraperitoneally administered (10 to 20 µg / administration / head) into a Balb / c mouse (7 week-old, male) at two week intervals. After the treatment was carried out four times, the fifth administration was carried out by intravenously administering 50 µg of a recombinant AST solution was administered three days before the day of cell fusion. Blood was collected from the tail vein of the mouse, incubated at 37°C for 30 minutes, and then centrifuged at 3,000 rpm for 10 minutes to recover the serum. The anti-AST antibody titer in the serum was assayed by ELISA.
Hereinafter, the method will be described.

50 µ L of the recombinant AST diluted with 0.05M Na₂CO₃ buffer solution (pH 10.5) in a concentration of 1 µg / mL was added to a 96 hole LISA plate (Falcon 3912, Becton Dickinson Corp.) and reacted at 4°C overnight. The reaction product was washed with PBS containing 0.05% Tween 20 three times, and then treated with PBS containing 3% bovine serum albumin (BSA) at room temperature for one hour. The treated product was washed with PBS containing 0.05% Tween 20 three times, mixed with 50 µ L of a specimen, and reacted at room temperature for one hour. The reaction product was washed with PBS containing 0.05% Tween 20 three times, mixed with 50 µ L of an alkali phosphatase-bound goat anti-mouse IgG antibody (ZYMED Corp.) diluted with 3% BSA-containing PBS 1,000 times, and reacted at room temperature for one hour. The reaction product was washed with PBS containing 0.05% Tween 20 three times, reacted with PNPP (p-nitrophenylphosphate, Wako Pure Chemical Industries, Ltd.) at room temperature for one hour, and then subjected to the assay of absorbance at 405 nm.

### (2) Preparation of hybridoma by cell fusion

A mouse was killed just before the cell fusion, and the spleen cells of the killed mouse were homogenized in PBS. The residues were filtered off with a nylon mesh and then once subjected to a centrifugal washing treatment using PBS. The obtained spleen cells were fused with mouse myeloma cells (P3X63Ag8U. 1) according to a conventional method (Kohler, Milstein; Nature, 256, 495 to 497, 1975).

Namely, 5 × 10⁷ spleen cells and 5 × 10⁶ mouse myeloma cells P3 × 63 Ag8U. 1(P3U1) were washed with RPMI 1640 culture medium and then centrifuged at 1,500 rpm for five minutes to obtain the cell pellet. 1 mL of the cell pellet was gradually mixed with a 35% polyethylene glycol solution (5.75 mL of RPMI 1640 culture medium + 3.5 mL of polyethylene glycol + 0.75 mL of dimethylsulfoxide) for two minutes to gently float the cells. The mixture was gradually mixed with 1 mL of RPMI 1640 culture medium for two minutes and further with 2 mL of RPMI 1640 culture medium for two minutes. The mixture was gradually mixed with 4 mL of GIT-HAT culture medium (GIT culture medium containing 95 *µ*M hypoxanthine, 0.4 *µ*M aminopterin, 1.6 *µ*M thymidine, and 5% FCS) for two minutes, further mixed with 8 mL of GIT-HAT culture medium for two minutes, and then incubated at 37°C for 30 minutes. The incubated product was dispensed into a 96 well flat bottom plate in whose each well about 10⁴ mouse peritoneal exudate cells were sowed, and then cultured in the presence of 5% CO₂ at 37°C.

One week later, the culture product was subjected to the exchange of a half of the culture medium by GIT-HT culture medium (a culture medium obtained by removing aminopterin from GIT-HAT culture medium), and further cultured in the presence of 5% CO₂ at 37°C for about one week to obtain the several hybridoma colonies per well.

### (3) Screening of hybridoma

Two week later, the screening of the hybridoma was carried out with a plate coated with the recombinant AST. The 50 mM Na₂CO₃ buffer solution (1 *µ* g / mL, pH 10.5) of the recombinant AST was dispensed into a 96 well plate (Falcon Corp., produced from PVC) in a volume of 50 *µ*L per well, and then allowed to stand at 4°C overnight. The product was washed and then blocked with 200 *µ*L / well of 3% BSA / PBS at 37°C for one hour. The product was again washed, mixed with 50 *µ*L / well of the culture supernatant, allowed to stand at room temperature for one hour, and then washed with 0.05% Tween / PBS three times.

Subsequently, the product was mixed with 50 *µ*l / well of goat anti-mouse IgG-alkali phosphatase conjugate (Tago Corp.) diluted with PBS containing 3% BSA and 0.2% skim milk 2,000 times, left a room temperature for one hour, again washed, mixed with 100 *µ* L / well of the 1 mg / mL solution of disodium p-nitrophenylphosphate (Wako Pure Chemical Industries) dissolved in a 1M diethanolamine buffer solution (pH 9.8) containing 0.25 mM magnesium chloride, and reacted at room temperature for 30 minutes. The absorbance of the product at 405 nm was examined with an ELISA reader instrument (Vmax, Molecular Davice Corp.) for 96 well plates, and the hybridoma secreting the monoclonal antibody bound to the recombinant AST was selected.

### (4) Cloning of hybridoma

The selected hybridoma was twice cloned by a limiting dilution method to establish the hybridoma. Concretely, mouse peritoneal exudate cells prepared in HT culture medium in a density of 10⁶ cells / mL were dispensed into wells, respectively, and the hybridoma cells suspended in HT culture medium in a rate of 0.5 cell / well were sowed in the wells, and then cultured in the presence of 5% CO₂ at 37°C for two weeks. The culture supernatant was screened by the above-described ELISA method, and a single colony was picked up to establish the cells.

### (5) Culture of hybridoma and purification of antibody

Six clones among twenty anti-AST antibody-producing hybridoma clones obtained by the above-described operations were fit to and cultured in an eRDF culture medium (Gibco BRL Corp.) which is a serum-free culture medium containing insulin, transferrin, ethanolamine and selenite. The culture supernatant was recovered. The antibody in the supernatant was purified with a protein G column by a conventional method.

### (6) Sub-typing of AST monoclonal antibody

Six purified anti-AST monoclonal antibody clones obtained by the operations of (5) were sub-typed with IsoStrip (an isotyping kit for mouse monoclonal antibodies, Boehringer Corp.) according to a protocol. Consequently, it was found that three clones and two clones were IgG1 and IgM, respectively.

### [Example 19] Western blotting with anti-AST antibody

A recombinant AST or natural AST was fractionated by an SDS polyacrylamide gel electrophoresis under a reducing condition, and then subjected to a western blotting on a nitrocellulose membrane (BIO-RAD Corp.). The product was treated with 3% BSA·containing PBS at room temperature overnight and then reacted with the anti-AST antibody obtained in Example 17, 18 and diluted with the 3% BAS-containing PBS in a density of 10 µ g / mL, at room temperature for one hour. The nitrocellulose membrane was washed with 1% BSA-containing PBS fix times, and then reacted with goat anti-human IgG antibody (TAGO Corp.) bound to an alkali phosphatase diluted with 3% BSA-containing PBS in a density of 25 *µ*g / mL, at room temperature for one hour. The nitrocellulose membrane was washed with 1% BSA-containing PBS six times and then reacted with NBT / BICP (PIERCE Corp.) at room temperature for 10 minutes to develop the color.

Thereby, it was shown that the anti-AST antibodies were bound to the recombinant AST, respectively. In the reactivity of the western blotting, the reactivity of anti-N19 PAb among the polyclonal antibodies was high, and the reactivity of anti-rAST PAb was low. Further, the anti-rAST PAb did not exhibit a cross reactivity with mast cell-originated tryptase and trypsin. Normal rabbit IgG was used as a negative control.

### [Example 20] Evaluation of AST-inhibiting activity

### (1) Method for assaying AST activity

The definitions of the assaying method and the activity unit are as shown in the above-described Example 2.

### (2) Evaluation of AST-inhibiting activity of anti-AST antibody

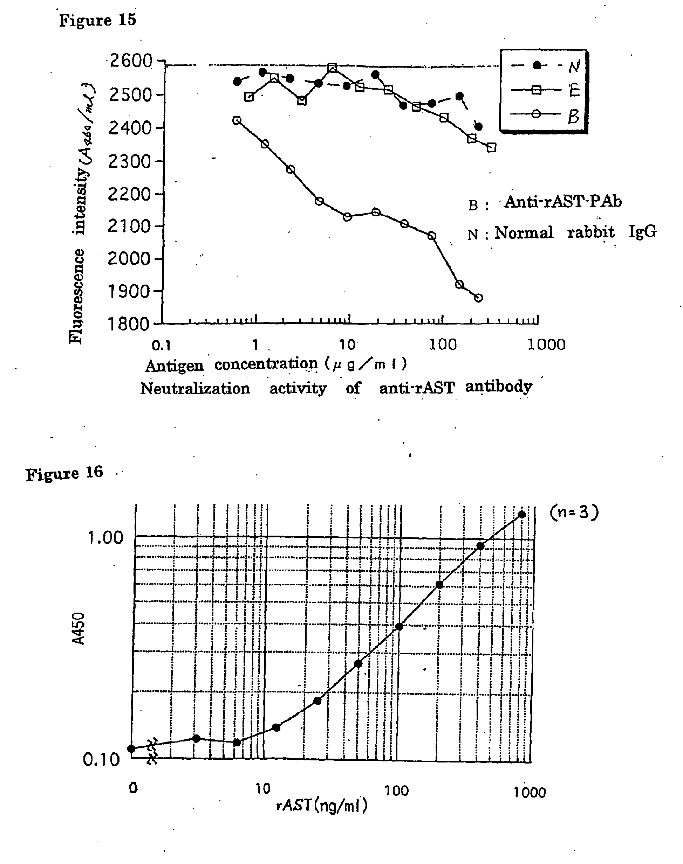

0.5 mL of a 50 mM Tris-HCl buffer solution (pH 8.6) containing 2 ng / mL of the recombinant AST and 0.01% of BSA was mixed with 50 µL of an anti-AST antibody-containing solution, and incubated at 37 °C for 30 minutes. The incubation product was mixed with 0.5 mL of a 50 mM Tris-HCl buffer solution (pH 8.6) containing 200 *µ*M of a trypsin synthesis substrate: Boc-Phe-Ser-Arg-MCA, and incubated at 37°C for one hour. The product was mixed with 1 mL of 30% acetic acid. The quantity of the produced AMC was assayed by a fluorometry (fluorescent light: 460 nm, and exciting light: 380 nm), and the enzymatic activity-inhibiting ability of the anti-AST antibody was evaluated. The results are shown in Figure 15.

In the anti-AST monoclonal antibody obtained by immunizing the recombinant AST (insect cell-baculovirus system) obtained in Example, an activity-inhibiting effect of 27% was recognized in a concentration of 236 µ g / mL.

### (3) Evaluation of AST-inhibiting activity of compound

0.05 mL of a 50 mM Tris-HCl buffer solution (pH 8.6) containing 2 ng / mL of the recombinant AST and 0.01% of Tween 20 was mixed with 2 µL of a DMSO solution containing nafamostat mesylate or gabexate mesylate as an evaluation target compound in various concentrations, and with 0.05 mL of a 50 mM Tris-HCl buffer solution (pH 8.6) containing 30 µM of a trypsin synthesis substrate: Boc-Phe-Ser-Arg-MCA, and incubated at 24°C for 6 minutes. The quantities of the produced AMC were assayed by a fluorometry (fluorescent light: 460 nm, exciting light: 380 nm) with the passage of time, and the enzymatic activity-inhibiting ability of the compound was evaluated. The results are shown in the next table as IC 50 (molar concentration exhibiting the inhibiting activity of 50%).

| Compound name | IC 50 (µM) |
|---|---|
| Nafamostat mesylate | 0.23 |
| Gabexate mesylate | 0.14 |

### [Example 21] Construction of AST assay system

### (1) Construction of sandwich ELISA system with polyclonal antibody

The following operations using the anti-AST polyclonal antibody (anti-rASTPAb) obtained in Example 17 were carried out to construct the sandwich ELISA system.

### 1) Preparation of horse radish peroxidase (HRP)-labeled antibody

### ① Preparation of antibody (F(ab')₂)

1 mL of the 2.0 mg / mL PBS solution of an antibody (IgG) was mixed with 100 *µ*L of a 1M acetate buffer solution (pH 4.2) and 20 *µ*L of the same buffer solution containing 40 *µ*g of pepsin, and reacted at 37°C for four hours. After the reaction is finished, the reaction solution was subjected to a separation treatment using a PBS-equilibrated Sephadex G 25 column (φ 2 cm × 45 cm) to collect the antibody (F(ab')₂).

### ② HRP label of antibody (F(ab')₂

2 mL of a solution (pH 7.4) of 1 mg / mL of the antibody (F(ab')₂), 0.01M phosphate and 0.15M NaCl was mixed with 50 *µ*L of the dimethylformamide solution (10 mg / mL) of N-(m-maleimide benzoic acid)-N-succinimide ester (MBS), and reacted at 25°C for 30 minutes. The reaction solution was then subjected to a gel filtration treatment using a column filled with Sephadex G-25 in a 0.1M phosphate buffer solution (pH 6.0) to separate the maleimidized antibody from the unreacted MBS.

On the other hand, 2 mL of a 0.1M phosphate buffer solution (pH 6.5) containing 10 mg / mL of HRP was mixed with 120 µL of a dimethylformamide solution containing 60 mg / mL of S-acetylmercaptosuccinic anhydride, and reacted at 25°C for two hours. The reaction solution was mixed 800 µL of a 0.1M Tris-HCl buffer solution (pH 7.0), 160 µL of 0.1M EDTA and 1.6 mL of 1M hydroxylamine, and reacted at 0°C for four minutes. Subsequently, the reaction solution was charged in a collodion bag and then dialyzed with a 0.1M phosphate buffer solution (pH 6.0) and a 5 mM EDTA-containing solution at 4°C for three days to obtain the thiolated HRP.

Subsequently, 2 mg of the maleimidized antibody was mixed with 4 mg of the thiolated HRP, and the mixture was concentrated with a collodion bag under cooling with ice until to give a protein concentration of 4 to 10 mg / mL, and then allowed to stand at 15 to 20°C overnight. The solution was subjected to a gel filtration using a column filled with Ultrogel AcA44 (produced by Pharmacia LKB Corp.) to obtain the anti-AST (F(ab')₂) HRP-labeled antibody.

### 2) Preparation of antibody-immobilized plate

A 96 well plate (produced by Sumitomo Bakelite Co., MS-3796F / carboplate) was well washed, allowed to stand in the 20 µ g / mL PBS solution of an antibody at 4°C for a whole day and night, washed with PBS, and then allowed to stand in the 1% PBS solution of bovine serum albumin (BSA) at 4°C for a whole day and night to carry out the post-coating treatment, thus obtaining the antibody-immobilized plate.

### 3) Construction of assay system

100 µL of a 1% BSA-0.1% skim milk-containing 10 mM PBS (pH 7.2) solution (diluted specimen solution) containing a purified recombinant AST (standard substance) in a concentration of 0 to 1,600 ng / mL was added to the anti-AST antibody IgG-immobilized plate prepared in 2), and then incubated at 25°C for four hours. Subsequently, the plate was washed with 0.05% Tween 20-containing 10 mM PBS (pH 7.2). 100 µL of the 1% BSA-containing 10 mM (pH 7.2) 100-fold diluted solution of the anti-AST (F(ab')2) HRP-labeled antibody (500 µ g / mL) prepared in 1) was added to each well, and incubated at 25°C for two hours. The wells were subjected to the suctional removal of the solutions in each well, and then washed with 0.05% Tween 20-containing 10 mM PBS (pH 7.2). 100 µ L of a 0.1M phosphate / citrate buffer solution (pH 4.3) containing 0.02% of 3, 3', 5, 5'-tetramethylbenzidine hydrochloride and 2.5 mM of hydrogen peroxide was added to each well, and reacted at 25°C for 30 minutes. 100 µ L of a 0.5M sulfuric acid aqueous solution was added to each well as a reaction stopper to stop the enzyme reaction. The absorption intensity of the solution at a wavelength of 450 nm was assayed with a spectrophotometer, and the assayed absorption intensities were plotted in response to the standard substance concentrations of 0 to 1,600 ng / mL. The results are shown in Figure 16.

### 4) Detection of AST in intravital component

### ① Detection of AST in saliva

AST in human saliva was assayed with the assay system in 3). The saliva collected from a human being was centrifuged at 15,000 rpm for 15 minutes, and the supernatant was used as an assay sample. Six samples were assayed in an undiluted liquid state or in a state diluted with a 1% BSA-0.1% slim milk-containing 10 mM PBS (pH 7.2) solution (specimen-diluting solution) sixteen times. Since the assayed values of the three samples were low, the values of the undiluted solutions were adopted, and for the other three samples, the values of the 16-fold diluted solutions were adopted as the AST concentrations of the saliva samples. The assay results are shown together with the AST, activities (trypsin substrate-degrading activities) in the samples in the next table.

| | AST (ng / mL) | |
|---|---|---|
| Saliva samples | ELISA | AST activity conversion* |
| KM 123 | 1616 | 3333 |
| KY 123 | 24 | 120 |
| KT 123 | 22 | 518 |
| HO 123 | 720 | 1772 |
| KY 112 | 25 | 208 |
| HO 112 | 784 | 2349 |

| | | |
|---|---|---|
| * Recombinant HAT conversion trypsin-like enzyme activity | | |

### ② Detection of AST in sputum

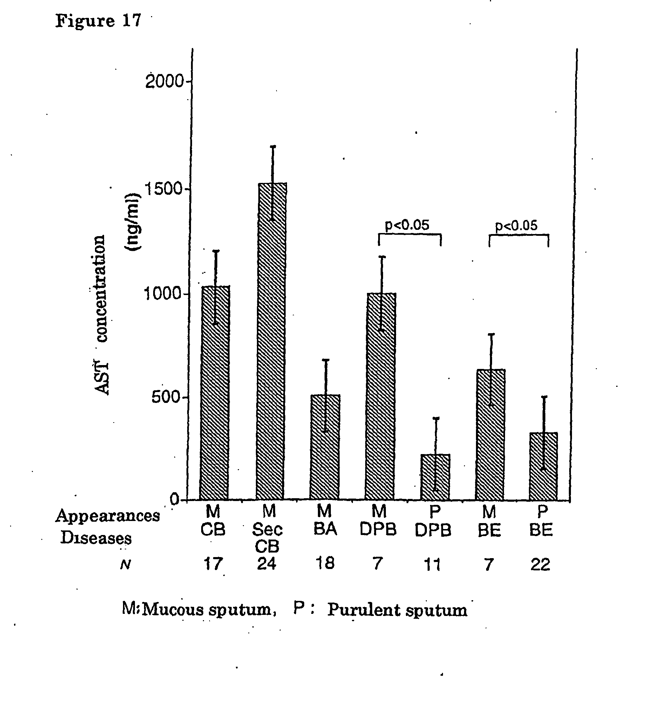

Sputum was collected from a chronic respiratory inflammation patient, diluted with a nine-fold volume of physiological salt solution, homogenized, and then centrifuged at 10,000 × g for 10 minutes. The supernatant was used as an assay specimen. The specimen was diluted twenty times, and assayed. The results are shown in Figure 17. It was found that the assay values of purulent sputa were lower than those of mucous sputa.

Herein, the mucous-mucos purulent sputa were collected from the following patients.
CB : chronic bronchitis
SecCB : secondary bronchitis
BA : bronchial asthma
BE : bronchiectasis
DPB : diffuse panbronchiolitis

Further, the purulent sputa were collected from the following patients.
BE : : bronchiectasis
DPB : diffuse panbronchiolitis

### (2) Construction of sandwich ELISA system with monoclonal antibody and polyclonal antibody

### 1) Evaluation of liquid phase antigenic reactivity of each anti-AST monoclonal antibody

The evaluation of a binding property to the recombinant AST in a liquid phase was carried out with respect to three kinds of anti-AST monoclonal antibodies which were IgG1 type by a sub-typing.

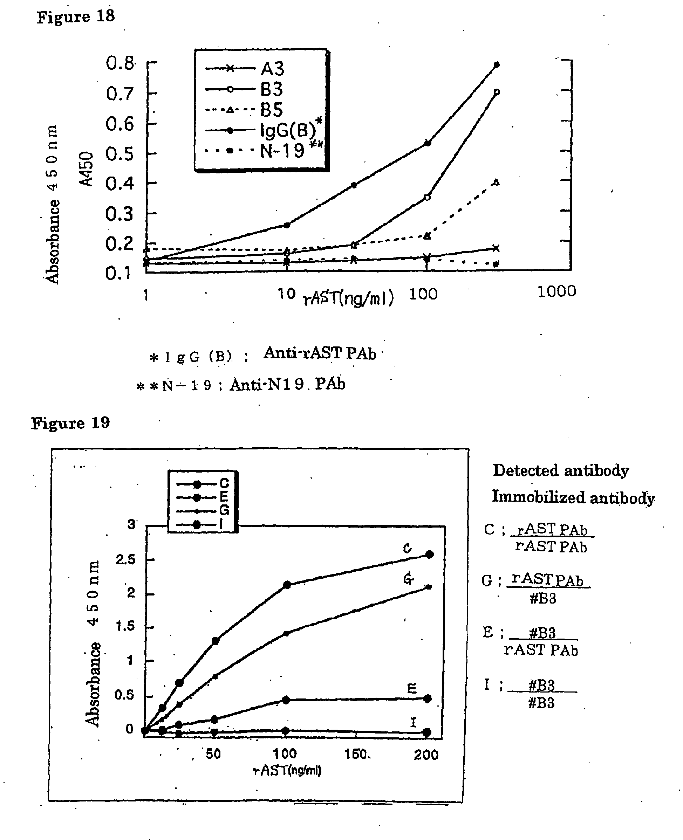

50 *µ*L / well of a PBS solution (1 and 5 *µ*g / mL) of each anti-AST monoclonal antibody was added to a 96 well plate, and allowed to stand at 4°C overnight to immobilize the antibody. 100 *µ*L of a 1% BSA-0.1% skim milk-containing 10 mM PBS (pH 7.2) solution (specimen-diluting solution) containing the purified recombinant AST (standard substance) in a range of 0 to 300 ng / mL was added to the prepared anti-AST monoclonal antibody-immobilized plate, and then incubated at 25°C for four hours. Subsequently, the plate was washed with 0.05% Tween 20-containing 10 mM PBS (pH 7.2), subjected to the addition of 100 µL of a solution obtained by diluting the HRP-labeled antibody (500 µg / mL) prepared in (1) 1) with 1% BSA-containing 10 mM PBS (pH 7.2) 100 times to each well, and then incubated at 25°C for two hours. Then, the solution was sucked off from each well, and the well was washed with 0.05% Tween 20-containing 10 mM PBS (pH 7.2). 100 µL of a 0.1M phosphate / citrate buffer solution (pH 4.3) containing 0.02% of 3,3',5,5'-tetramethylbenzidine hydrochloride and 2.5 mM of hydrogen peroxide was added to each well. The reaction was carried out at 25°C for 30 minutes, and 100 µ L of a 0.5M aqueous sulfuric acid solution was added to each well to stop the enzymatic reaction. Subsequently, the absorption intensity of the solution at a wavelength of 450 nm was assayed with a spectrophotometer. The assayed absorption intensities were plotted in response to the standard substance concentrations of 0 to 300 ng / mL. The results are shown in Figure 18.

### 2) Construction of assay system

### ① Preparation of biotinylated antibody

The biotinylation of IgG was carried out with the # B3 anti-AST monoclonal antibody having the highest liquid phase antigenic reactivity in the evaluation results of 1) and with the anti-AST polyclonal antibody (anti-rAST PAb) obtained in Example 17.

1 mL of a 1 to 3 mg / mL IgG / PBS (-) solution was mixed with the equivalent volume of 0.2M NaHCO₃ (pH 8.0), further mixed with 10 mM of a biotinylating reagent having a molar concentration of 100 times, and then incubated at 37°C for one hour. As the biotinylating reagent, NHS-LC-Biotin (produced by PIERCE Corp., #21335) dissolved in dimethylformamide in a concentration of 10 mM was used. The reaction solution was mixed with one-tenth amount of 1M monoethanolamine and further incubated at 37°C for one hour. The reaction solution was diluted with PBS (-) into 2.5 mL, and then applied to a G-25 gel filtration column to allow to flow out the PBS(-), thus recovering the biotinylated IgG. The recovered biotinylated IgG was mixed with BSA in a final concentration of 3%, further mixed with NaN₈ in a final concentration of 0.1%, and then stored.

### ② Preparation of antibody-immobilized plate

A 96 well plate (produced by Sumitomo Bakelite Co., MS-3796F/carboplate) was well washed, subjected to the addition of 100 µ L of the 20 µ g / mL PBS solution of an antibody (#B3 and anti-rAST PAb) to each well, and allowed to stand at 4°C for a whole day and night. The treated plate was washed with PBS-0.05% Tween 20, subjected to the addition of 380 µ L of a 1% bovine serum albumin PBS solution to each well, and then allowed to stand at 4°C for a whole day and night to post-treat the wells, thus obtaining the antibody-immobilized plate.

### ③ Construction of assay system

50 µL of a 1% BSA-0.1% skim milk-containing 10 mM PBS (pH 7.2) solution (specimen-diluting solution) containing the purified recombinant AST (standard substance) in a range of 0 to 400 nm / mL, and 50 µL of a 1% BSA-0.1% skim milk-containing 10 mM PBS (pH 7.2) solution containing the biotinylated IgG prepared in ① were added to the anti-AST IgG-immobilized plate prepared in ②, and then allowed to stand at 4°C overnight. The plate was washed, subjected to the addition of 100 µL / well of a 1% BSA-0.1% skim milk-containing 10 mM PBS (pH 7.2) solution obtained by diluting streptavidin-labeled peroxidase (produced by TAGO Corp., # SNN1004) 10,000 times to each well, incubated a 25°C for one hour, subjected to the suctional removal of the solution in each well, washed with 0.05% Tween 20-containing 10 mM PBS (pH 7.2), and further subjected to the addition of 100 µL of a 0.1M phosphate / citrate buffer solution (pH 4.3) containing 0.02% of 3,3',5,5'-tetramethylbenzidine hydrochloride and 2. 5 mM of hydrogen peroxide to each well. The reaction was carried out at 25°C for 15 minutes, and 100 *µ*L of a 0.5M aqueous sulfuric acid solution was added to each well to stop the enzymatic reaction. Subsequently, the absorption intensity of the solution at a wavelength of 450 nm was assayed with a spectrophotometer. The assayed absorption intensities were plotted in response to the standard substance concentrations of 0 to 200 ng / mL. The results are shown in Figure 19.

### Industrial applicability

The AST having the structure in which the whole propeptide moiety or a part thereof is bound to the trypsin-like protein moiety via the disulfide bond is disclosed above. The AST having said structure can be used to construct an inhibitor-screening system using the PAR activation, mucus production promotion, EGFR signal transduction system activation, or intracellular calcium influx promotion activity as an indicator, and thus screen an inhibitor, thereby obtaining an AST inhibitor and an inhibitory polypeptide (including inhibitory antibodies). It can be expected that the thus obtained AST inhibitor, the AST PAR activation inhibitor, the AST mucus production promotion inhibitor, the EGFR signal transduction system activation inhibitor, or the intracellular calcium influx promotion inhibitor is used as a therapeutic drug to control or modify a physiological action such as the wide sense airway inflammation actions, mucus secretion promotion action, airway remodeling action, mucociliary self defence system action, coagulation · fibrinogenolysis system action, cell proliferation action, cancer proliferation or metastasis action, or anti-virus infection action, to prevent disease crisis or to improve and treat the state of the disease.

In addition, on the basis of the sequence of the animal AST, such as mouse AST, it can be judged in what disease state model animal and at what time the AST participates in the state of a disease by assaying or controlling the protein expression or gene expression of the animal AST. Further, the therapeutic effects of the AST inhibitor and the inhibitory protein selected by utilization of the present invention can be judged, and the clinical doses of the AST inhibitor or the inhibitory polypeptide can also be estimated.

Further, the expression sites of tissues can be detected by immunohistochemistry using a monoclonal antibody or polyclonal antibody.

Furthermore, the enzyme immunoassay system, diagnostic reagent or diagnostic kit using such a monoclonal antibody or polyclonal antibody enables the assay of the concentration of the AST in saliva, sputum, serum or the like, and is thereby useful for understanding and diagnosing the state of an AST-participating disease.

## Claims

1. A method for screening for a compound or a polypeptide capable of inhibiting AST by mixing:
i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
iii) a cell expressing said enzyme, or
iv) a tissue expressing said enzyme,
the compound or the polypeptide to be assayed, and further a PAR-expressing cell, and then using the PAR activation as an indicator.

2. The detection method according to claim 1, wherein the protease-activated receptor is PAR2.

3. A method for screening for a compound or a polypeptide capable of inhibiting AST, by mixing:
i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
iii) a cell expressing said enzyme, or
iv) a tissue expressing said enzyme,
the compound or the polypeptide to be assayed, and further a cell having an intracellular calcium influx ability, and then using the intracellular calcium influx as an indicator.

4. A method for screening for a compound or a polypeptide capable of inhibiting AST, by mixing:
i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
iii) a cell expressing said enzyme, or
iv) a tissue expressing said enzyme,
the compound or the polypeptide to be assayed, and further a cell having a mucus-producing ability, and then using the secretion product as an indicator.

5. The detection method according to claim 4, wherein a method for assaying the mucus-producing ability is an AB-PAS staining method.

6. The detection method according to claim 4, wherein the indicator of the mucus-producing ability is the quantity of expressed Muc5AC.

7. The detection method according to claim 4, wherein the indicator of the mucus-producing ability is the quantity of released or produced EGF, HB-EGF, TGF-α, Amphiregulin or Betacellulin.

8. The detection method according to claim 4, wherein the indicator of the mucus-producing ability is the activation of an EGF-R signal transduction pathway.

9. A method for screening for a compound or a polypeptide capable of inhibiting EGF. HB-EGF, TGF-α, Amphiregulin or Betacellutin, by mixing:
i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
iii) a cell expressing said enzyme, or
iv) a tissue expressing said enzyme,
the compound or the polypeptide to be assayed, and further a cell having an EGF, HB-EGF, TGF-α, Amphiregulin or Betacellulin, and then using the release quantity of the EGF, HB-EGF, TGF-α, Amphiregulin or Betacellulin, as an indicator.

10. A method for screening for a compound or polypeptide capable of inhibiting AST, by mixing:
i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
iii) a cell expressing said enzyme, or
iv) a tissue expressing said enzyme,
the compound or the polypeptide to be assayed, and further a cell having an EGFR signal transduction pathway, and then using the activation of the EGFR signal transduction pathway as an indicator.

11. A method for screening for a compound or a polypeptide capable of inhibiting AST, by mixing:
i) an airway-specific trypsin-like enzyme which has a structure in which a propeptide moiety consisting of the amino acid sequence from any one of positions 145-170, to Arg at the 186^{th} position of the amino acid sequence represented by SEQ ID NO: 1 is bound to a trypsin-like protein moiety consisting of a sequence of 232 amino acids from Ile at the 187^{th} position to Ile at the 418^{th} position of the amino acid sequence represented by SEQ ID NO: 1 via a disulfide bond, wherein the disulphide bond is formed between the cysteine residue at position 173 of said propeptide moiety and the cysteine residue at position 292 of said trypsin-like protein moiety of the amino acid sequence represented by SEQ ID NO: 1, or
ii) a mammalian airway-specific trypsin-like enzyme which is a part of a mammalian counterpart protein having 66% or more homology with the airway-specific trypsin-like enzyme represented by SEQ ID NO: 1 and has a structure in which a moiety corresponding to said propeptide moiety consisting of the amino acid sequence from any one of positions 145-170, to Arg at the 186^{th} position is bound to a moiety corresponding to said trypsin-like protein moiety consisting of a sequence of 232 amino acids from Ile at the 187^{th} position to Ile at the 418^{th} position via a disulfide bond, wherein the disulphide bond is formed between the cysteine residue at position 173 of said propeptide moiety and the cysteine residue at position 292 of said trypsin-like protein moiety, or
iii) a cell expressing said enzyme, or
iv) a tissue expressing said enzyme,
the compound or the polypeptide to be assayed, and further a cell and using the release quantity or expression quantity of II-8 or the transcription activity of the II-8 promoter as an indicator.

## Patentansprüche

1. Verfahren zum Screenen für ein(e) zur Hemmung von AST befähigte(s) Verbindung oder Polypeptid, durch Mischen:
(i) eines atemwegsspezifischen Trypsin-ähnlichen Enzyms mit einer Struktur, in welcher eine Propeptideinheit, bestehend aus der Aminosäuresequenz von einer der Positionen 145-170 bis Arg an der 186. Position der in SEQ ID Nr.: 1 dargestellten Aminosäuresequenz, mit einer Trypsin-ähnlichen Proteineinheit, bestehend aus einer Sequenz von 232 Aminosäuren von Ile an der 187. Position bis Ile an der 418. Position der in SEQ ID NR.: 1 dargestellten Aminosäuresequenz, über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit der in SEQ ID Nr.: 1 dargestellten Aminosäuresequenz gebildet ist, oder
(ii) eines atemwegsspezifischen Trypsin-ähnlichen Säugerenzyms, welches ein Teil eines Gegenstück-Säugerproteins ist, welches 66% oder mehr Homologie mit dem in SEQ ID NR.: 1 dargestellten atemwegsspezifischen Trypsin-ähnlichen Enzym aufweist, und eine Struktur aufweist, in welcher eine der Propeptideinheit entsprechende Einheit, bestehend aus der Aminosäuresequenz von einer der Positionen 145-170 bis Arg an der 186. Position mit einer der Trypsin-ähnlichen Proteineinheit entsprechenden Einheit, bestehend aus einer Sequenz von 232 Aminosäuren von Ile an der 187. Position bis Ile an der 418. Position, über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit gebildet ist, oder
(iii) einer das Enzym exprimierenden Zelle, oder
(iv) eines das Enzym exprimierenden Gewebes,
der Verbindung oder des Polypeptids, welche(s) untersucht werden soll, und weiter einer PAR-exprimierenden Zelle,
und dann das Verwenden der PAR-Aktivierung als Indikator.

2. Nachweisverfahren nach Anspruch 1, wobei der Protease-aktivierte Rezeptor PAR2 ist.

3. Verfahren zum Screenen für ein(e) zur Hemmung von AST befähigte(s) Verbindung oder Polypeptid, durch Mischen:
(i) eines atemwegsspezifischen Trypsin-ähnlichen Enzyms mit einer Struktur, in welcher eine Propeptideinheit, bestehend aus der Aminosäuresequenz von einer der Positionen 145-170 bis Arg an der 186. Position der in SEQ ID Nr.: 1 dargestellten Aminosäuresequenz, mit einer Trypsin-ähnlichen Proteineinheit, bestehend aus einer Sequenz von 232 Aminosäuren von Ile an der 187. Position bis Ile an der 418. Position der in SEQ ID NR.: 1 dargestellten Aminosäuresequenz, über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit der in SEQ ID Nr.: 1 dargestellten Aminosäuresequenz gebildet ist, oder
(ii) eines atemwegsspezifischen Trypsin-ähnlichen Säugerenzyms, welches ein Teil eines Gegenstück-Säugerproteins ist, welches 66% oder mehr Homologie mit dem in SEQ ID NR.: 1 dargestellten atemwegsspezifischen Trypsin-ähnlichen Enzym aufweist, und eine Struktur aufweist, in welcher eine der Propeptideinheit entsprechende Einheit, bestehend aus der Aminosäuresequenz von einer der Positionen 145-170 bis Arg an der 186. Position mit einer der Trypsin-ähnlichen Proteineinheit entsprechenden Einheit, bestehend aus einer Sequenz von 232 Aminosäuren von Ile an der 187. Position bis Ile an der 418. Position, über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit gebildet ist, oder
(iii) einer das Enzym exprimierenden Zelle, oder
(iv) eines das Enzym exprimierenden Gewebes,
der Verbindung oder des Polypeptids, welche(s) untersucht werden soll, und weiter einer Zelle mit einer intrazellulären Calciumeinströmbefähigung,
und dann das Verwenden des intrazellulären Calciumeinstroms als Indikator.

4. Verfahren zum Screenen für ein(e) zur Hemmung von AST befähigte(s) Verbindung oder Polypeptid, durch Mischen:
(i) eines atemwegsspezifischen Trypsin-ähnlichen Enzyms mit einer Struktur, in welcher eine Propeptideinheit, bestehend aus der Aminosäuresequenz von einer der Positionen 145-170 bis Arg an der 186. Position der in SEQ ID Nr.: 1 dargestellten Aminosäuresequenz, mit einer Trypsin-ähnlichen Proteineinheit, bestehend aus einer Sequenz von 232 Aminosäuren von Ile an der 187. Position bis Ile an der 418. Position der in SEQ ID NR.: 1 dargestellten Aminosäuresequenz, über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit der in SEQ ID Nr.: 1 dargestellten Aminosäuresequenz gebildet ist, oder
(ii) eines atemwegsspezifischen Trypsin-ähnlichen Säugerenzyms, welches ein Teil eines Gegenstück-Säugerproteins ist, welches 66% oder mehr Homologie mit dem in SEQ ID NR.: 1 dargestellten atemwegsspezifischen Trypsin-ähnlichen Enzym aufweist, und eine Struktur aufweist, in welcher eine der Propeptideinheit entsprechende Einheit, bestehend aus der Aminosäuresequenz von einer der Positionen 145-170 bis Arg an der 186. Position mit einer der Trypsin-ähnlichen Proteineinheit entsprechenden Einheit, bestehend aus einer Sequenz von 232 Aminosäuren von Ile an der 187. Position bis Ile an der 418. Position, über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit gebildet ist, oder
(iii) einer das Enzym exprimierenden Zelle, oder
(iv) eines das Enzym exprimierenden Gewebes,
der Verbindung oder des Polypeptids, welche(s) untersucht werden soll, und weiter einer Zelle mit einer Schleimbildungsfähigkeit,
und dann das Verwenden des Sekretionsprodukts als Indikator.

5. Nachweisverfahren nach Anspruch 4, wobei ein Verfahren zum Untersuchen der Schleimbildungsfähigkeit ein AB-PAS Anfärbeverfahren ist.

6. Nachweisverfahren nach Anspruch 4, wobei der Indikator der Schleimbildungsfähigkeit die Menge von exprimiertem Muc5AC ist.

7. Nachweisverfahren nach Anspruch 4, wobei der Indikator der Schleimbildungsfähigkeit die Menge von freigesetztem oder hergestellten EGF, HB-EGF, TGF-α, Amphiregulin oder Betacellulin ist.

8. Nachweisverfahren nach Anspruch 4, wobei der Indikator der Schleimbildungsfähigkeit die Aktivierung eines EGF-R Signaltransduktionswegs ist.

9. Verfahren zum Screenen für ein(e) zur Hemmung von EGF, HB-EGF, TGF-α, Amphiregulin oder Betacellulin befähigte(s) Verbindung oder Polypeptid, durch Mischen:
(i) eines atemwegsspezifischen Trypsin-ähnlichen Enzyms mit einer Struktur, in welcher eine Propeptideinheit, bestehend aus der Aminosäuresequenz von einer der Positionen 145-170 bis Arg an der 186. Position der in SEQ ID Nr.: 1 dargestellten Aminosäuresequenz, mit einer Trypsin-ähnlichen Proteineinheit, bestehend aus einer Sequenz von 232 Aminosäuren von Ile an der 187. Position bis Ile an der 418. Position der in SEQ ID NR.: 1 dargestellten Aminosäuresequenz, über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit der in SEQ ID Nr.: 1 dargestellten Aminosäuresequenz gebildet ist, oder
(ii) eines atemwegsspezifischen Trypsin-ähnlichen Säugerenzyms, welches ein Teil eines Gegenstück-Säugerproteins ist, welches 66% oder mehr Homologie mit dem in SEQ ID NR.: 1 dargestellten atemwegsspezifischen Trypsin-ähnlichen Enzym aufweist, und eine Struktur aufweist, in welcher eine der Propeptideinheit entsprechende Einheit, bestehend aus der Aminosäuresequenz von einer der Positionen 145-170 bis Arg an der 186. Position mit einer der Trypsin-ähnlichen Proteineinheit entsprechenden Einheit, bestehend aus einer Sequenz von 232 Aminosäuren von Ile an der 187. Position bis Ile an der 418. Position über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit gebildet ist, oder
(iii) einer das Enzym exprimierenden Zelle, oder
(iv) eines das Enzym exprimierenden Gewebes,
der Verbindung oder des Polypeptids, welche(s) untersucht werden soll, und weiter einer Zelle mit EGF, HB-EGF, TGF-α, Amphiregulin oder Betacellulin, und dann das Verwenden der Freisetzungsmenge von EGF, HB-EGF, TGF-α, Amphiregulin oder Betacellulin als Indikator.

10. Verfahren zum Screenen für ein(e) zur Hemmung von AST befähigte(s) Verbindung oder Polypeptid, durch Mischen:
(i) eines atemwegsspezifischen Trypsin-ähnlichen Enzyms mit einer Struktur, in welcher eine Propeptideinheit, bestehend aus der Aminosäuresequenz von einer der Positionen 145-170 bis Arg an der 186. Position der in SEQ ID Nr.: 1 dargestellten Aminosäuresequenz, mit einer Trypsin-ähnlichen Proteineinheit, bestehend aus einer Sequenz von 232 Aminosäuren von Ile an der 187. Position bis Ile an der 418. Position der in SEQ ID NR.: 1 dargestellten Aminosäuresequenz, über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit der in SEQ ID Nr.: 1 dargestellten Aminosäuresequenz gebildet ist, oder
(ii) eines atemwegsspezifischen Trypsin-ähnlichen Säugerenzyms, welches ein Teil eines Gegenstück-Säugerproteins ist, welches 66% oder mehr Homologie mit dem in SEQ ID NR.: 1 dargestellten atemwegsspezifischen Trypsin-ähnlichen Enzym aufweist, und eine Struktur aufweist, in welcher eine der Propeptideinheit entsprechende Einheit, bestehend aus der Aminosäuresequenz von einer der Positionen 145-170 bis Arg an der 186. Position mit einer der Trypsin-ähnlichen Proteineinheit entsprechenden Einheit, bestehend aus einer Sequenz von 232 Aminosäuren von Ile an der 187. Position bis Ile an der 418. Position über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit gebildet ist, oder
(iii) einer das Enzym exprimierenden Zelle, oder
(iv) eines das Enzym exprimierenden Gewebes,
der Verbindung oder des Polypeptids, welche(s) untersucht werden soll, und weiter einer Zelle mit einem EGFR-Signaltransduktionsweg, und dann das Verwenden der Aktivierung des EGFR-Signaltransduktionsweges als Indikator.

11. Verfahren zum Screenen für ein(e) zur Hemmung von AST befähigte(s) Verbindung oder Polypeptid, durch Mischen:
(i) eines atemwegsspezifischen Trypsin-ähnlichen Enzyms mit einer Struktur, in welcher eine Propeptideinheit, bestehend aus der Aminosäuresequenz von einer der Positionen 145-170 bis Arg an der 186. Position der in SEQ ID Nr.: 1 dargestellten Aminosäuresequenz, mit einer Trypsin-ähnlichen Proteineinheit, bestehend aus einer Sequenz von 232 Aminosäuren von Ile an der 187. Position bis Ile an der 418. Position der in SEQ ID NR.: 1 dargestellten Aminosäuresequenz, über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit der in SEQ ID Nr.: 1 dargestellten Aminosäuresequenz gebildet ist, oder
(ii) eines atemwegsspezifischen Trypsin-ähnlichen Säugerenzyms, welches ein Teil eines Gegenstück-Säugerproteins ist, welches 66% oder mehr Homologie mit dem in SEQ ID NR.: 1 dargestellten atemwegsspezifischen Trypsin-ähnlichen Enzym aufweist, und eine Struktur aufweist, in welcher eine der Propeptideinheit entsprechende Einheit, bestehend aus der Aminosäuresequenz von einer der Positionen 145-170 bis Arg an der 186. Position mit einer der Trypsin-ähnlichen Proteineinheit entsprechenden Einheit, bestehend aus einer Sequenz von 232 Aminosäuren von Ile an der 187. Position bis Ile an der 418. Position über eine Disulfidbindung verbunden ist, wobei die Disulfidbindung zwischen dem Cysteinrest an Position 173 der Propeptideinheit und dem Cysteinrest an Position 292 der Trypsin-ähnlichen Proteineinheit gebildet ist, oder
(iii) einer das Enzym exprimierenden Zelle, oder
(iv) eines das Enzym exprimierenden Gewebes,
der Verbindung oder des Polypeptids, welche(s) untersucht werden soll, und weiter einer Zelle und das Verwenden der Freisetzungsmenge oder Expressionsmenge von IL-8 oder der Transkriptionsaktivität des IL-8-Promotors als Indikator.

## Revendications

1. Méthode de criblage d'un composé ou d'un polypeptide capable d'inhiber l'AST, en mélangeant :
i) une enzyme de type trypsine spécifique des voies aériennes, qui présente une structure dans laquelle une fraction propeptide, constituée de la séquence d'acides aminés depuis l'une quelconque des positions 145 à 170 à Arg en position 186 de la séquence d'acides aminés représentée par l'ID SEQ n° 1, est liée à une fraction de protéine de type trypsine constituée d'une séquence de 232 acides aminés depuis Ile en position 187 à Ile en position 418 de la séquence d'acides aminés représentée par l'ID SEQ n° 1 via une liaison disulfure, où la liaison disulfure est formée entre le résidu cystéine en position 173 de ladite fraction propeptide et le résidu cystéine en position 292 de ladite fraction de protéine de type trypsine de la séquence d'acides aminés représentée par l'ID SEQ n° 1, ou
ii) une enzyme de type trypsine spécifique des voies aériennes d'un mammifère, qui est une partie d'une protéine correspondante de mammifère présentant 66 % d'homologie ou plus avec l'enzyme de type trypsine spécifique des voies aériennes représentée par l'ID SEQ n° 1 et présente une structure dans laquelle une fraction correspondant à ladite fraction propeptide, constituée de la séquence d'acides aminés depuis une quelconque des positions 145 à 170 à Arg en position 186, est liée à une fraction correspondant à ladite fraction de protéine de type trypsine constituée d'une séquence de 232 acides aminés depuis Ile en position 187 à Ile en position 418 via une liaison disulfure, où la liaison disulfure est formée entre le résidu cystéine en position 173 de ladite fraction propeptide et le résidu cystéine en position 292 de ladite fraction de protéine de type trypsine, ou
iii) une cellule exprimant ladite enzyme, ou
iv) un tissu exprimant ladite enzyme,
le composé ou le polypeptide à analyser, et en outre une cellule exprimant un RAP (récepteur activé de protéase) ; puis en utilisant l'activation du RAP en tant qu'indicateur.

2. Méthode de détection selon la revendication 1, dans laquelle le récepteur activé de protéase est le récepteur RAP-2.

3. Méthode de criblage d'un composé ou d'un polypeptide capable d'inhiber l'AST, en mélangeant :
i) une enzyme de type trypsine spécifique des voies aériennes, qui présente une structure dans laquelle une fraction propeptide, constituée de la séquence d'acides aminés depuis l'une quelconque des positions 145 à 170 à Arg en position 186 de la séquence d'acides aminés représentée par l'ID SEQ n° 1, est liée à une fraction de protéine de type trypsine constituée d'une séquence de 232 acides aminés depuis Ile en position 187 à Ile en position 418 de la séquence d'acides aminés représentée par l'ID SEQ n° 1 via une liaison disulfure, où la liaison disulfure est formée entre le résidu cystéine en position 173 de ladite fraction propeptide et le résidu cystéine en position 292 de ladite fraction de protéine de type trypsine de la séquence d'acides aminés représentée par l'ID SEQ n° 1, ou
ii) une enzyme de type trypsine spécifique des voies aériennes d'un mammifère, qui est une partie d'une protéine correspondante de mammifère présentant 66 % d'homologie ou plus avec l'enzyme de type trypsine spécifique des voies aériennes représentée par l'ID SEQ n° 1 et présente une structure dans laquelle une fraction correspondant à ladite fraction propeptide, constituée de la séquence d'acides aminés depuis une quelconque des positions 145 à 170 à Arg en position 186, est liée à une fraction correspondant à ladite fraction de protéine de type trypsine constituée d'une séquence de 232 acides aminés depuis Ile en position 187 à Ile en position 418 via une liaison disulfure, où la liaison disulfure est formée entre le résidu cystéine en position 173 de ladite fraction propeptide et le résidu cystéine en position 292 de ladite fraction de protéine de type trypsine, ou
iii) une cellule exprimant ladite enzyme, ou
iv) un tissu exprimant ladite enzyme,
le composé ou le polypeptide à analyser, et en outre une cellule ayant une aptitude à l'influx de calcium intracellulaire ; puis en utilisant l'influx de calcium intracellulaire en tant qu'indicateur.

4. Méthode de criblage d'un composé ou d'un polypeptide capable d'inhiber l'AST, en mélangeant :
i) une enzyme de type trypsine spécifique des voies aériennes, qui présente une structure dans laquelle une fraction propeptide, constituée de la séquence d'acides aminés depuis l'une quelconque des positions 145 à 170 à Arg en position 186 de la séquence d'acides aminés représentée par l'ID SEQ n° 1, est liée à une fraction de protéine de type trypsine constituée d'une séquence de 232 acides aminés depuis Ile en position 187 à Ile en position 418 de la séquence d'acides aminés représentée par l'ID SEQ n° 1 via une liaison disulfure, où la liaison disulfure est formée entre le résidu cystéine en position 173 de ladite fraction propeptide et le résidu cystéine en position 292 de ladite fraction de protéine de type trypsine de la séquence d'acides aminés représentée par l'ID SEQ n° 1, ou
ii) une enzyme de type trypsine spécifique des voies aériennes d'un mammifère, qui est une partie d'une protéine correspondante de mammifère présentant 66 % d'homologie ou plus avec l'enzyme de type trypsine spécifique des voies aériennes représentée par l'ID SEQ n° 1 et présente une structure dans laquelle une fraction correspondant à ladite fraction propeptide, constituée de la séquence d'acides aminés depuis une quelconque des positions 145 à 170 à Arg en position 186, est liée à une fraction correspondant à ladite fraction de protéine de type trypsine constituée d'une séquence de 232 acides aminés depuis Ile en position 187 à Ile en position 418 via une liaison disulfure, où la liaison disulfure est formée entre le résidu cystéine en position 173 de ladite fraction propeptide et le résidu cystéine en position 292 de ladite fraction de protéine de type trypsine, ou
iii) une cellule exprimant ladite enzyme, ou
iv) un tissu exprimant ladite enzyme,
le composé ou le polypeptide à analyser, et en outre une cellule ayant une aptitude à produire du mucus ; puis en utilisant le produit de sécrétion en tant qu'indicateur.

5. Méthode de détection selon la revendication 4, dans laquelle une méthode d'analyse de l'aptitude à la production de mucus est une méthode de coloration au bleu alcian/acide périodique-Schiff (BA/APS).

6. Méthode de détection selon la revendication 4, dans laquelle l'indicateur de l'aptitude à la production de mucus est la quantité de Muc5AC exprimée.

7. Méthode de détection selon la revendication 4, dans laquelle l'indicateur de l'aptitude à la production de mucus est la quantité d'EGF, de HB-EGF, de TGF-α, d'amphiréguline ou de bêtacelluline libérée ou produite.

8. Méthode de détection selon la revendication 4, dans laquelle l'indicateur de l'aptitude à la production de mucus est l'activation du processus de transduction de signal d'EGF-R.

9. Méthode de criblage d'un composé ou d'un polypeptide capable d'inhiber l'EGF, le HB-EGF, la TGF-α, l'amphiréguline ou la bêtacelluline en mélangeant :
i) une enzyme de type trypsine spécifique des voies aériennes, qui présente une structure dans laquelle une fraction propeptide, constituée de la séquence d'acides aminés depuis l'une quelconque des positions 145 à 170 à Arg en position 186 de la séquence d'acides aminés représentée par l'ID SEQ n° 1, est liée à une fraction de protéine de type trypsine constituée d'une séquence de 232 acides aminés depuis Ile en position 187 à Ile en position 418 de la séquence d'acides aminés représentée par l'ID SEQ n° 1 via une liaison disulfure, où la liaison disulfure est formée entre le résidu cystéine en position 173 de ladite fraction propeptide et le résidu cystéine en position 292 de ladite fraction de protéine de type trypsine de la séquence d'acides aminés représentée par l'ID SEQ n° 1, ou
ii) une enzyme de type trypsine spécifique des voies aériennes d'un mammifère, qui est une partie d'une protéine correspondante de mammifère présentant 66 % d'homologie ou plus avec l'enzyme de type trypsine spécifique des voies aériennes représentée par l'ID SEQ n° 1 et présente une structure dans laquelle une fraction correspondant à ladite fraction propeptide, constituée de la séquence d'acides aminés depuis une quelconque des positions 145 à 170 à Arg en position 186, est liée à une fraction correspondant à ladite fraction de protéine de type trypsine constituée d'une séquence de 232 acides aminés depuis Ile en position 187 à Ile en position 418 via une liaison disulfure, où la liaison disulfure est formée entre le résidu cystéine en position 173 de ladite fraction propeptide et le résidu cystéine en position 292 de ladite fraction de protéine de type trypsine, ou
iii) une cellule exprimant ladite enzyme, ou
iv) un tissu exprimant ladite enzyme,
le composé ou le polypeptide à analyser, et en outre une cellule comportant un EGF, HB-EGF, TGF-α, amphiréguline ou bêtacelluline ; puis en utilisant la quantité libérée d'EGF, de HB-EGF, de TGF-α, d'amphiréguline ou de bêtacelluline en tant qu'indicateur.

10. Méthode de criblage d'un composé ou d'un polypeptide capable d'inhiber l'AST en mélangeant :
i) une enzyme de type trypsine spécifique des voies aériennes, qui présente une structure dans laquelle une fraction propeptide, constituée de la séquence d'acides aminés depuis l'une quelconque des positions 145 à 170 à Arg en position 186 de la séquence d'acides aminés représentée par l'ID SEQ n° 1, est liée à une fraction de protéine de type trypsine constituée d'une séquence de 232 acides aminés depuis Ile en position 187 à Ile en position 418 de la séquence d'acides aminés représentée par l'ID SEQ n° 1 via une liaison disulfure, où la liaison disulfure est formée entre le résidu cystéine en position 173 de ladite fraction propeptide et le résidu cystéine en position 292 de ladite fraction de protéine de type trypsine de la séquence d'acides aminés représentée par l'ID SEQ n° 1, ou
ii) une enzyme de type trypsine spécifique des voies aériennes d'un mammifère, qui est une partie d'une protéine correspondante de mammifère présentant 66 % d'homologie ou plus avec l'enzyme de type trypsine spécifique des voies aériennes représentée par l'ID SEQ n° 1 et présente une structure dans laquelle une fraction correspondant à ladite fraction propeptide, constituée de la séquence d'acides aminés depuis une quelconque des positions 145 à 170 à Arg en position 186, est liée à une fraction correspondant à ladite fraction de protéine de type trypsine constituée d'une séquence de 232 acides aminés depuis Ile en position 187 à Ile en position 418 via une liaison disulfure, où la liaison disulfure est formée entre le résidu cystéine en position 173 de ladite fraction propeptide et le résidu cystéine en position 292 de ladite fraction de protéine de type trypsine, ou
iii) une cellule exprimant ladite enzyme, ou
iv) un tissu exprimant ladite enzyme,
le composé ou le polypeptide à analyser, et en outre une cellule ayant un processus de transduction de signal d'EGF-R ; puis en utilisant l'activation du processus de transduction de signal EGF-R en tant qu'indicateur.

11. Méthode de criblage d'un composé ou d'un polypeptide capable d'inhiber l'AST en mélangeant :
i) une enzyme de type trypsine spécifique des voies aériennes, qui présente une structure dans laquelle une fraction propeptide, constituée de la séquence d'acides aminés depuis l'une quelconque des positions 145 à 170 à Arg en position 186 de la séquence d'acides aminés représentée par l'ID SEQ n° 1, est liée à une fraction de protéine de type trypsine constituée d'une séquence de 232 acides aminés depuis Ile en position 187 à Ile en position 418 de la séquence d'acides aminés représentée par l'ID SEQ n° 1 via une liaison disulfure, où la liaison disulfure est formée entre le résidu cystéine en position 173 de ladite fraction propeptide et le résidu cystéine en position 292 de ladite fraction de protéine de type trypsine de la séquence d'acides aminés représentée par l'ID SEQ n° 1, ou
ii) une enzyme de type trypsine spécifique des voies aériennes d'un mammifère, qui est une partie d'une protéine correspondante de mammifère présentant 66 % d'homologie ou plus avec l'enzyme de type trypsine spécifique des voies aériennes représentée par l'ID SEQ n° 1 et présente une structure dans laquelle une fraction correspondant à ladite fraction propeptide, constituée de la séquence d'acides aminés depuis une quelconque des positions 145 à 170 à Arg en position 186, est liée à une fraction correspondant à ladite fraction de protéine de type trypsine constituée d'une séquence de 232 acides aminés depuis Ile en position 187 à Ile en position 418 via une liaison disulfure, où la liaison disulfure est formée entre le résidu cystéine en position 173 de ladite fraction propeptide et le résidu cystéine en position 292 de ladite fraction de protéine de type trypsine, ou
iii) une cellule exprimant ladite enzyme, ou
iv) un tissu exprimant ladite enzyme,
le composé ou le polypeptide à analyser, et en outre une cellule ; puis en utilisant la quantité libérée ou la quantité exprimée d'I1-8 ou l'activité de transcription du promoteur d'Il-8 en tant qu'indicateur.


### SEQUENCE LISTING

<110> TEIJIN LIMITED
<120> AIRWAY-SPECIFIC TRYPSIN-LIKE ENZYMES AND METHOD OF USING THE SAME
<130> P033256EP
<140> EP 01958544.7
   <141> 2001-08-28
<150> JP P2000-257104
   <151> 2000-08-28
<150> JP P2001-059753
   <151> 2001-03-05
<160> 44
<210> 1
   <211> 418
   <212> PRT
   <213> Homo sapiens
<400> 1
   Met Tyr Arg Pro Ala Arg Val Thr Ser Thr Ser Arg Phe Leu Asn
   1 5 10 15
   Pro Tyr Val Val Cys Phe Ile Val Val Ala Gly Val Val Ile Leu
   20 25 30
   Ala Val Thr Ile Ala Leu Leu Val Tyr Phe Leu Ala Phe Asp Gln
   35 40 45
   Lys Ser Tyr Phe Tyr Arg Ser Ser Phe Gln Leu Leu Asn Val Glu
   50 55 60
   Tyr Asn Ser Gln Leu Asn Ser Pro Ala Thr Gln Glu Tyr Arg Thr
   65 70 75
   Leu Ser Gly Arg Ile Glu Ser Leu Ile Thr Lys Thr Phe Lys Glu
   80 85 90
   Ser Asn Leu Arg Asn Gln Phe Ile Arg Ala His Val Ala Lys Leu
   95 100 105
   Arg Gln Asp Gly Ser Gly Val Arg Ala Asp Val Val Met Lys Phe
   110 115 120
   Gln Phe Thr Arg Asn Asn Asn Gly Ala Ser Met Lys Ser Arg Ile
   125 130 135
   Glu Ser Val Leu Arg Gln Met Leu Asn Asn Ser Gly Asn Leu Glu
   140 145 150
   Ile Asn Pro Ser Thr Glu Ile Thr Ser Leu Thr Asp Gln Ala Ala
   155 160 165
   Ala Asn Trp Leu Ile Asn Glu Cys Gly Ala Gly Pro Asp Leu Ile
   170 175 180
   Thr Leu Ser Glu Gln Arg Ile Leu Gly Gly Thr Glu Ala Glu Glu
   185 190 195
   Gly Ser Trp Pro Trp Gln Val Ser Leu Arg Leu Asn Asn Ala His
   200 205 210
   His Cys Gly Gly Ser Leu Ile Asn Asn Met Trp Ile Leu Thr Ala
   215 220 225
   Ala His Cys Phe Arg Ser Asn Ser Asn Pro Arg Asp Trp Ile Ala
   230 235 240
   Thr Ser Gly Ile Ser Thr Thr Phe Pro Lys Leu Arg Met Arg Val
   245 250 255
   Arg Asn Ile Leu Ile His Asn Asn Tyr Lys Ser Ala Thr His Glu
   260 265 270
   Asn Asp Ile Ala Leu Val Arg Leu Glu Asn Ser Val Thr Phe Thr
   275 280 285
   Lys Asp Ile His Ser Val Cys Leu Pro Ala Ala Thr Gln Asn Ile
   290 295 300
   Pro Pro Gly Ser Thr Ala Tyr Val Thr Gly Trp Gly Ala Gln Glu
   305 310 315
   Tyr Ala Gly His Thr Val Pro Glu Leu Arg Gln Gly Gln Val Arg
   320 325 330
   Ile Ile Ser Asn Asp Val Cys Asn Ala Pro His Ser Tyr Asn Gly
   335 340 345
   Ala Ile Leu Ser Gly Met Leu Cys Ala Gly Val Pro Gln Gly Gly
   350 355 360
   Val Asp Ala Cys Gln Gly Asp Ser Gly Gly Pro Leu Val Gln Glu
   365 370 375
   Asp Ser Arg Arg Leu Trp Phe Ile Val Gly Ile Val Ser Trp Gly
   380 385 390
   Asp Gln Cys Gly Leu Pro Asp Lys Pro Gly Val Tyr Thr Arg Val
   395 400 405
   Thr Ala Tyr Leu Asp Trp Ile Arg Gln Gln Thr Gly Ile
   410 415 418
<210> 2
   <211> 417
   <212> PRT
   <213> Mus musculus
<400> 2
   Met Tyr Arg Pro Arg Pro Met Leu Ser Pro Ser Arg Phe Phe Thr
   1 5 10 15
   Pro Phe Ala Val Ala Phe Val Val Ile Ile Thr Val Gly Leu Leu
   20 25 30
   Ala Met Met Ala Gly Leu Leu Ile His Phe Leu Ala Phe Asp Lys
   35 40 45
   Lys Ala Tyr Phe Tyr His Ser Ser Phe Gln Ile Leu Asn Val Glu
   50 55 60
   Tyr Thr Glu Ala Leu Asn Ser Pro Ala Thr His Glu Tyr Arg Thr
   65 70 75
   Leu Ser Glu Arg Ile Glu Ala Met Ile Thr Asp Glu Phe Arg Gly
   80 85 90
   Ser Ser Leu Lys Ser Glu Phe Ile Arg Thr His Val Val Lys Leu
   95 100 105
   Arg Lys Glu Gly Thr Gly Val Val Ala Asp Val Val Met Lys Phe
   110 115 120
   Arg Ser Ser Lys Arg Asn Asn Arg Lys Val Met Lys Thr Arg Ile
   125 130 135
   Gln Ser Val Leu Arg Arg Leu Ser Ser Ser Gly Asn Leu Glu Ile
   140 145 150
   Ala Pro Ser Asn Glu Ile Thr Ser Leu Thr Asp Gln Asp Thr Glu
   155 160 165
   Asn Val Leu Thr Gln Glu Cys Gly Ala Arg Pro Asp Leu Ile Thr
   170 175 180
   Leu Ser Glu Glu Arg Ile Ile Gly Gly Met Gln Ala Glu Pro Gly
   185 190 195
   Asp Trp Pro Trp Gln Val Ser Leu Gln Leu Asn Asn Val His His
   200 205 210
   Cys Gly Gly Ala Leu Ile Ser Asn Met Trp Val Leu Thr Ala Ala
   215 220 225
   His Cys Phe Lys Ser Tyr Pro Asn Pro Gln Tyr Trp Thr Ala Thr
   230 235 240
   Phe Gly Val Ser Thr Met Ser Pro Arg Leu Arg Val Arg Val Arg
   245 250 255
   Ala Ile Leu Ala His Asp Gly Tyr Ser Ser Val Thr Arg Asp Asn
   260 265 270
   Asp Ile Ala Val Val Gln Leu Asp Arg Ser Val Ala Phe Ser Arg
   275 280 285
   Asn Ile His Arg Val Cys Leu Pro Ala Ala Thr Gln Asn Ile Ile
   290 295 300
   Pro Gly Ser Val Ala Tyr Val Thr Gly Trp Gly Ser Leu Thr Tyr
   305 310 315
   Gly Gly Asn Ala Val Thr Asn Leu Arg Gln Gly Glu Val Arg Ile
   320 325 330
   Ile Ser Ser Glu Glu Cys Asn Thr Pro Ala Gly Tyr Ser Gly Ser
   335 340 345
   Val Leu Pro Gly Met Leu Cys Ala Gly Met Arg Ser Gly Ala Val
   350 355 360
   Asp Ala Cys Gln Gly Asp Ser Gly Gly Pro Leu Val Gln Glu Asp
   365 370 375
   Ser Arg Arg Leu Trp Phe Val Val Gly Ile Val Ser Trp Gly Tyr
   380 385 390
   Gln Cys Gly Leu Pro Asn Lys Pro Gly Val Tyr Thr Arg Val Thr
   395 400 405
   Ala Tyr Arg Asn Trp Ile Arg Gln Gln Thr Gly Ile
   410 415 417
<210> 3
   <211> 1500
   <212> DNA
   <213> Homo sapiens
<400> 3
418
<210> 4
   <211> 1462
   <212> DNA
   <213> Mus musculus
<400> 4
<210> 5
   <211> 1462
   <212> RNA
   <213> Mus musculus
<400> 5
   cgccgggcag gucaaagcag cuggacacac agaaacaagg accucuucau uauucaagag 60
   uaaaauguau aggccaagac caaugcuauc accgucaaga uucuucacuc ccuuugcagu 120
   agcuuucguu gucauaauaa cgguagggcu ccuggccaug auggcagguc uacuuauuca 180
   cuuuuuagcu uuugacaaga aagcuuacuu uuaucauagc agcuuucaaa uccuaaacgu 240
   ugaauacacu gaggcuuuaa acucaccagc uacacacgaa uacagaaccu ugagugaaag 300
   aauugaggcu augauuacug augaauuucg aggaucaagu cuaaaaagug aguuuaucag 360
   gacacauguu gucaaacuaa gaaaagaagg gacuggugug guugcggaug uugucaugaa 420
   auuucgaucu aguaaacgua acaacagaaa gguaaugaaa accagaauuc aaucugugcu 480
   acgaagacuc agcagcucug gaaacuugga aauagccccu ucgaaugaga uaacaucacu 540
   cacugaccag gauacagaaa auguuuugac ucaagaaugu ggagcacguc cagaccuuau 600
   aacacuguca gaagagagaa ucauuggagg caugcaagcu gagcccggug acuggcccug 660
   gcaagucagu cuacagcuca auaaugucca ccacugugga ggugcccuga ucaguaacau 720
   guggguccug acagcagcuc auugcuucaa aagcuauccu aauccucaau auuggacagc 780
   caccuuuggg guuucuacaa ugagcccuag gcugagagug agaguaaggg cuauuuuagc 840
   ccacgacggg uacagcuccg uaacucguga caaugacauc gcaguuguac aacuugacag 900
   aucugucgcc uuuuccagaa auauccauag gguaugucuc ccagcagcaa cccaaaauau 960
   caucccuggu ucugucgcau auguuacagg auggggaucu cucacauaug gaggcaacgc 1020
   agucacaaau cuacggcaag gagaagucag aauaauaagu ucagaagaau gcaauacgcc 1080
   agcugguuac aguggaagug ucuugccagg aaugcugugu gcuggaaugc guucaggggc 1140
   cguggaugca ugccagggug auucaggugg cccgcuagua caagaagacu caaggcggcu 1200
   uugguuuguu gugggcauug ugagcugggg auaucagugu ggccucccaa auaagccagg 1260
   cguguauacu cgagugacag ccuaccgcaa cuggaucaga cagcagacgg gaaucuagug 1320
   caaccgagga aaaaacgugc caugaggucu cuguauccaa gugugacuga cucggaugcc 1380
   auggcuucac auuucaacug caaaggagac uggaaaugcc ccuucugacg ucccauuaca 1440
   uaaauugguu uaacuguuua gu 1462
<210> 6
   <211> 1462
   <212> DNA
   <213> Mus musculus
<400> 6
<210> 7
   <211> 28
   <212> DNA
   <213> Artificial Sequence
<220>
   <223> PCR primer
<400> 7
   aaggatccat ggggccgcgg cggctgct 28
<210> 8
   <211> 28
   <212> DNA
   <213> Artificial Sequence
<220>
   <223> PCR primer
<400> 8
   tgggaattcc taagttaaca gctttttg 28
<210> 9
   <211> 29
   <212> DNA
   <213> Artificial Sequence
<220>
   <223> PCR primer
<400> 9
   cctttggatc caggaggatg cggagcccc 29
<210> 10
   <211> 33
   <212> DNA
   <213> Artificial Sequence
<220>
   <223> PCR primer
<400> 10
   ctgtggaatt cccatctgag gacctggaaa act 33
<210> 11
   <211> 33
   <212> DNA
   <213> Artificial Sequence
<220>
   <223> PCR primer
<400> 11
   cctttggatc catgaaagcc ctcatctttg cag 33
<210> 12
   <211> 35
   <212> DNA
   <213> Artificial Sequence
<220>
   <223> PCR primer
<400> 12
   cctttgaatt cctattttgt aaggtaagca gtgga 35
<210> 13
   <211> 32
   <212> DNA
   <213> Artificial Sequence
<220>
   <223> PCR primer
<400> 13
   cctttagatc tatgtggggg cgactgctcc tg 32
<210> 14
   <211> 31
   <212> DNA
   <213> Artificial Sequence
<220>
   <223> PCR primer
<400> 14
   cctttgaatt ctgtcactgg agcaaagagg a 31
<210> 15
   <211> 24
   <212> DNA
   <213> Artificial Sequence
<220>
   <223> PCR primer
<400> 15
   tttactgttt tcgtaacagt tttg 24
<210> 16
   <211> 20
   <212> DNA
   <213> Artificial Sequence
<220>
   <223> PCR primer
<400> 16
   caacaacgca cagaatctag 20
<210> 17
   <211> 20
   <212> DNA
   <213> Artificial Sequence
<220>
   <223> PCR primer
<400> 17
   ctgacngcng cncaytgctt 20
<210> 18
   <211> 18
   <212> DNA
   <213> Artificial Sequence
<220>
   <223> PCR primer
<400> 18
   gtcrccctgr cangcrtc 18
<210> 19
   <211> 20
   <212> DNA
   <213> Artificial Sequence
<220>
   <223> PCR primer
<400> 19
   acgcattcca gcacacagca 20
<210> 20
   <211> 27
   <212> DNA
   <213> Artificial Sequence
<220>
   <223> PCR primer
<400> 20
   cacaaatcta cggcaaggag aggtcag 27
<210> 21
   <211> 27
   <212> DNA
   <213> Artificial Sequence
<220>
   <223> PCR primer
<400> 21
   gaacttatta ttctgacctc tccttgc 27
<210> 22
   <211> 20
   <212> DNA
   <213> Artificial Sequence
<220>
   <223> PCR primer
<400> 22
   ggaatgctgt gtgctggaat 20
<210> 23
   <211> 41
   <212> DNA
   <213> Artificial Sequence
<220>
   <223> PCR primer
<400> 23
   ccaaaggatc caaatgtata ggccaagacc aatgctatca c 41
<210> 24
   <211> 39
   <212> DNA
   <213> Artificial Sequence
<220>
   <223> PCR primer
<400> 24
   cctttgaatt cggaacgtca gaaggggcat ttccagtct 39
<210> 25
   <211> 20
   <212> PRT
   <213> Homo sapiens
<400> 25
   Ile Leu Gly Gly Thr Glu Ala Glu Glu Gly Ser Trp Pro Trp Gln
   1 5 10 15
   Val Ser Leu Arg Cys
   20
<210> 26
   <211> 1555
   <212> DNA
   <213> Sus scrofa
<220>
   <221> CDS
   <222> (106) ... (1359)
<220>
   <221> propeptide portion
   <222> (106) ... (663)
<220>
   <221> trypsin-like protein portion
   <222> (664)... (1359)
<400> 26
   gttctttcta gatccacata atacattagt agtcattcat ttgagtggaa atctcagagt 60
   ggtcagatag cagcaaaaag gacagcctca caattcagga ttaaa atg tat agg cca 117
   Met Tyr Arg Pro
   1
   gca aca gta cga tca gct tca aga tct ctg aat cca tac aca gta tgt 165
   Ala Thr Val Arg Ser Ala Ser Arg Ser Leu Asn Pro Tyr Thr Val Cys
   5 10 15 20
   ttt att gtt gtc gca gtg gtg gtg atc ctg gca gtg acc ata gct cta 213
   Phe Ile Val Val Ala Val Val Val Ile Leu Ala Val Thr Ile Ala Leu
   25 30 35
   ctt gtc cac ttt tta gct ttt gat caa aaa gct tac ttt tac cat agt 261
   Leu Val His Phe Leu Ala Phe Asp Gln Lys Ala Tyr Phe Tyr His Ser
   40 45 50
   aga ttt caa atc cta aat gtt gaa tat agt gat gag cta aat tca cca 309
   Arg Phe Gln Ile Leu Asn Val Glu Tyr Ser Asp Glu Leu Asn Ser Pro
   55 60 65
   gct aca cag aaa tat agg tct ttg agt gga aga att gaa tct atg att 357
   Ala Thr Gln Lys Tyr Arg Ser Leu Ser Gly Arg Ile Glu Ser Met Ile
   70 75 80
   act aga aca ttt aag gag tca aat tta aga aat cag ttc gtg aga gct 405
   Thr Arg Thr Phe Lys Glu Ser Asn Leu Arg Asn Gln Phe Val Arg Ala
   85 90 95 100
   cat gtt gtc aaa ctg agg caa agt ggt agt ggt gtg ata gca gat att 453
   His Val Val Lys Leu Arg Gln Ser Gly Ser Gly Val Ile Ala Asp Ile
   105 110 115
   gtc atg aaa ttt aaa ttc acc aga tat aac aat gga gca tca atg aaa 501
   Val Met Lys Phe Lys Phe Thr Arg Tyr Asn Asn Gly Ala Ser Met Lys
   120 125 130
   agc aga att gag tct gtt tta cgc caa atg ctg aat aac act gga aac 549
   Ser Arg Ile Glu Ser Val Leu Arg Gln Met Leu Asn Asn Thr Gly Asn
   135 140 145
   ttg gta atg aac cct tca act gag tta aca cca ata aca gac cag gat 597
   Leu Val Met Asn Pro Ser Thr Glu Leu Thr Pro Ile Thr Asp Gln Asp
   150 155 160
   aca gta aat att ttc act caa gga tgt ggg gcc cgt cca gac cta ata 645
   Thr Val Asn Ile Phe Thr Gln Gly Cys Gly Ala Arg Pro Asp Leu Ile
   165 170 175 180
   aca ttg tct gaa gag aga atc ata ggc ggc act aag gct gag gaa gga 693
   Thr Leu Ser Glu Glu Arg Ile Ile Gly Gly Thr Lys Ala Glu Glu Gly
   185 190 195
   gac tgg ccc tgg caa gtc agt ctg cag cgg aat aat ctt cat cac tgt 741
   Asp Trp Pro Trp Gln Val Ser Leu Gln Arg Asn Asn Leu His His Cys
   200 205 210
   gga ggc gtc ttg atc agt aac aga tgg atc ctg act gca gct cac tgc 789
   Gly Gly Val Leu Ile Ser Asn Arg Trp Ile Leu Thr Ala Ala His Cys
   215 220 225
   ttc aga agc tac tct gat cct cgc cag tgg act gtc acc ttt ggt att 837
   Phe Arg Ser Tyr Ser Asp Pro Arg Gln Trp Thr Val Thr Phe Gly Ile
   230 235 240
   tcc act ata ttt cct aaa gac aga ata gga gta agg aat att tta atc 885
   Ser Thr Ile Phe Pro Lys Asp Arg Ile Gly Val Arg Asn Ile Leu Ile
   245 250 255 260
   cat aac aat tat aac cct gaa act cat gaa aat gat att gcg ctt gta 933
   His Asn Asn Tyr Asn Pro Glu Thr His Glu Asn Asp Ile Ala Leu Val
   265 270 275
   caa ctc aac aga gaa gtc gcc ttt acc aaa aat atc cat tca gtg tgt 981
   Gln Leu Asn Arg Glu Val Ala Phe Thr Lys Asn Ile His Ser Val Cys
   280 285 290
   ctc cca gag gcc acc caa act att cca cct ggt tcc acg gct tat gta 1029
   Leu Pro Glu Ala Thr Gln Thr Ile Pro Pro Gly Ser Thr Ala Tyr Val
   295 300 305
   aca gga tgg gga tca caa aga tat agc ggc aac aca gtt cca gat cta 1077
   Thr Gly Trp Gly Ser Gln Arg Tyr Ser Gly Asn Thr Val Pro Asp Leu
   310 315 320
   gag caa gta cgg gtc aat ata ata agt aac gat gta tgt aat tcg cca 1125
   Glu Gln Val Arg Val Asn Ile Ile Ser Asn Asp Val Cys Asn Ser Pro
   325 330 335 340
   gct ggt tat aat ggg gat gtc ctg cct gga atg cta tgt gct ggg cta 1173
   Ala Gly Tyr Asn Gly Asp Val Leu Pro Gly Met Leu Cys Ala Gly Leu
   345 350 355
   cct gaa ggg gga gca gat gca tgc cag ggt gac tct ggt ggc cca cta 1221
   Pro Glu Gly Gly Ala Asp Ala Cys Gln Gly Asp Ser Gly Gly Pro Leu
   360 365 370
   cag cag gag gac tca cgg cgg ctt tgg ttc ctt gtg ggg ata gta agc 1269
   Gln Gln Glu Asp Ser Arg Arg Leu Trp Phe Leu Val Gly Ile Val Ser
   375 380 385
   tgg ggg tat cag tgt ggt ctg cca gat aag cca gga gtg tac act cga 1317
   Trp Gly Tyr Gln Cys Gly Leu Pro Asp Lys Pro Gly Val Tyr Thr Arg
   390 395 400
   gtg aca gcc tac cgg gac tgg ata gcc caa caa act ggg atc tag cacaa 1367
   Val Thr Ala Tyr Arg Asp Trp Ile Ala Gln Gln Thr Gly Ile
   405 410 415 418
   taaatacacc tctctagcaa gccggtgcac acacatgctc gtctacaatt ccaaacttta 1427
   ctttccagcc acaaaagaat acatgtttca caaacactat ttaatcttta ttactatgga 1487
   ttttatattc tctcaagaag atttagatga atgttgcatg gtactgtgga tatatgccgg 1547
   gggaaaca 1555
<210> 27
   <211 > 418
   <212> PRT
   <213> Sus scrofa
<220>
   <221> propeptide portion
   <222> (1)... (186)
<220>
   <221> trypsin-like protein portion
   <222> (187)... (418)
<400> 27
   Met Tyr Arg Pro Ala Thr Val Arg Ser Ala Ser Arg Ser Leu Asn
   1 5 10 15
   Pro Tyr Thr Val Cys Phe Ile Val Val Ala Val Val Val Ile Leu
   20 25 30
   Ala Val Thr Ile Ala Leu Leu Val His Phe Leu Ala Phe Asp Gln
   35 40 45
   Lys Ala Tyr Phe Tyr His Ser Arg Phe Gln Ile Leu Asn Val Glu
   50 55 60
   Tyr Ser Asp Glu Leu Asn Ser Pro Ala Thr Gln Lys Tyr Arg Ser
   65 70 75
   Leu Ser Gly Arg Ile Glu Ser Met Ile Thr Arg Thr Phe Lys Glu
   80 85 90
   Ser Asn Leu Arg Asn Gln Phe Val Arg Ala His Val Val Lys Leu
   95 100 105
   Arg Gln Ser Gly Ser Gly Val Ile Ala Asp Ile Val Met Lys Phe
   110 115 120
   Lys Phe Thr Arg Tyr Asn Asn Gly Ala Ser Met Lys Ser Arg Ile
   125 130 135
   Glu Ser Val Leu Arg Gln Met Leu Asn Asn Thr Gly Asn Leu Val
   140 145 150
   Met Asn Pro Ser Thr Glu Leu Thr Pro Ile Thr Asp Gln Asp Thr
   155 160 165
   Val Asn Ile Phe Thr Gln Gly Cys Gly Ala Arg Pro Asp Leu Ile
   170 175 180
   Thr Leu Ser Glu Glu Arg Ile Ile Gly Gly Thr Lys Ala Glu Glu
   185 190 195
   Gly Asp Trp Pro Trp Gln Val Ser Leu Gln Arg Asn Asn Leu His
   200 205 210
   His Cys Gly Gly Val Leu Ile Ser Asn Arg Trp Ile Leu Thr Ala
   215 220 225
   Ala His Cys Phe Arg Ser Tyr Ser Asp Pro Arg Gln Trp Thr Val
   230 235 240
   Thr Phe Gly Ile Ser Thr Ile Phe Pro Lys Asp Arg Ile Gly Val
   245 250 255
   Arg Asn Ile Leu Ile His Asn Asn Tyr Asn Pro Glu Thr His Glu
   260 265 270
   Asn Asp Ile Ala Leu Val Gln Leu Asn Arg Glu Val Ala Phe Thr
   275 280 285
   Lys Asn Ile His Ser Val Cys Leu Pro Glu Ala Thr Gln Thr Ile
   290 295 300
   Pro Pro Gly Ser Thr Ala Tyr Val Thr Gly Trp Gly Ser Gln Arg
   305 310 315
   Tyr Ser Gly Asn Thr Val Pro Asp Leu Glu Gln Val Arg Val Asn
   320 325 330
   Ile Ile Ser Asn Asp Val Cys Asn Ser Pro Ala Gly Tyr Asn Gly
   335 340 345
   Asp Val Leu Pro Gly Met Leu Cys Ala Gly Leu Pro Glu Gly Gly
   350 355 360
   Ala Asp Ala Cys Gln Gly Asp Ser Gly Gly Pro Leu Gln Gln Glu
   365 370 375
   Asp Ser Arg Arg Leu Trp Phe Leu Val Gly Ile Val Ser Trp Gly
   380 385 390
   Tyr Gln Cys Gly Leu Pro Asp Lys Pro Gly Val Tyr Thr Arg Val
   395 400 405
   Thr Ala Tyr Arg Asp Trp Ile Ala Gln Gln Thr Gly Ile
   410 415 418
<210> 28
   <211> 1520
   <212> DNA
   <213> Macaca fascicularis
<220>
   <221> CDS
   <222> (100) ... (1356)
<220>
   <221> propeptide portion
   <222> (100)... (657)
<220>
   <221> trypsin-like protein portion
   <222> (658)... (1356)
<210> 29
   <211> 418
   <212> PRT
   <213> Macaca fascicularis
<220>
   <221> propeptide portion
   <222> (1) ... (186)
<220>
   <221> trypsin-like protein portion
   <222> (187)... (418)
<400> 29
   Met Tyr Arg Pro Ala Arg Val Pro Ser Thr Ser Arg Phe Leu Asn Pro
   1               5                   10                  15
   Tyr Val Val Cys Phe Ile Val Val Ala Gly Val Val Ile Leu Ala Val
               20                  25                  30
   Thr Ile Ala Leu Leu Val Tyr Phe Leu Ala Phe Asp Gln Lys Ser Tyr
           35                  40                  45
   Phe Tyr Arg Ser Ser Phe Gln Leu Leu Asn Val Glu Tyr Asn Ser Gln
       50                  55                  60
   Leu Asn Ser Pro Ala Thr Gln Glu Tyr Arg Thr Leu Ser Gly Arg Ile
   65                  70                  75                  80
   Glu Ser Leu Ile Thr Arg Thr Phe Lys Glu Ser Asn Leu Arg Asn Gln
                   85                  90                  95
   Phe Met Arg Ala His Val Val Lys Leu Ser Gln Asp Gly Ser Gly Val
               100                 105                 110
   Arg Ala Asp Val Val Met Lys Phe Arg Phe Thr Arg Asn Asn Asn Gly
           115                 120                 125
   Ala Ser Met Lys Ser Arg Ile Glu Ser Val Leu Gln Gln Met Leu Asn
       130                 135                 140
   Asn Ser Gly Asn Leu Glu Ile Asn Ser Ser Thr Glu Ile Thr Ser Leu
   145                 150                 155                 160
   Thr Asp Gln Ala Ala Ala Asn Trp Leu Ile Asn Glu Cys Gly Ala Gly
                   165                 170                 175
   Pro Asp Leu Ile Thr Leu Ser Glu Gln Arg Ile Ile Gly Gly Thr Glu
               180                 185                 190
   Ala Glu Glu Gly Ser Trp Pro Trp Gln Val Ser Leu Arg Val Asn Asn
           195                 200                 205
   Ala His His Cys Gly Gly Ser Leu Ile Ser Asn Thr Trp Ile Leu Thr
       210                 215                 220
   Ala Ala His Cys Phe Arg Ser Asn Ser Asn Pro Arg Glu Trp Ile Ala
   225                 230                 235                 240
   Thr Phe Gly Ile Ser Thr Thr Asn Pro Arg Leu Arg Met Arg Val Arg
                   245                 250                 255
   Ser Ile Leu Ile His Asn Asn Tyr Ile Ser Ala Thr His Glu Asn Asp
   260 265 270
   Ile Ala Leu Val Arg Leu Glu Asn Ser Val Thr Phe Thr Arg Asp Ile
   275 280 285
   His Ser Val Cys Leu Pro Ala Ala Thr Gln Asn Ile Pro Leu Gly Ser
   290 295 300
   Thr Ala Tyr Val Thr Gly Trp Gly Ala Gln Glu Tyr Ala Gly Ser Thr
   305 310 315 320
   Val Ser Glu Leu Arg Gln Ala Gln Val Arg Ile Ile Ser Asn Asp Val
   325 330 335
   Cys Asn Ala Pro Tyr Ser Tyr Asn Gly Gly Ile Leu Pro Gly Met Leu
   340 345 350
   Cys Ala Gly Val Pro Gln Gly Gly Val Asp Ala Cys Gln Gly Asp Ser
   355 360 365
   Gly Gly Pro Leu Val Gln Glu Asp Ser Arg Arg Leu Trp Phe Leu Val
   370 375 380
   Gly Ile Val Ser Trp Gly Asp Gln Cys Gly Leu Pro Asp Arg Pro Gly
   385 390 395 400
   Val Tyr Thr Arg Val Thr Thr Tyr Arg Asp Trp Ile Arg Gln Arg Thr
   405 410 415
   Gly Ile
   418
<210> 30
   <211> 1406
   <212> DNA
   <213> Canis familiaris
<220>
   <221> CDS
   <222> (110)... (1366)
<220>
   <221> propeptide portion
   <222> (110) ... (667)
<220>
   <221> trypsin-like protein portion
   <222> (668)... (1366)
<400> 30
   gagttccttc atagatcaca cataatatat gagagtcatt catttgtgta gaaatctcag 60
   agaagttaga taggcagcag aaaggatctc ttcattattc aggatcaaa atg tat agg 118
                                                         Met Tyr Arg
                                                          1
   cca gca cgg gtg cca tca ggc tca aga ttc ctg aat cca tat gta gtg 166
   Pro Ala Arg Val Pro Ser Gly Ser Arg Phe Leu Asn Pro Tyr Val Val
       5                   10                  15
   tgt ttc gtt gtt gtg gca ggg gtg gta atc ctg gcc gta acc ata gct 214
   Cys Phe Val Val Val Ala Gly Val Val Ile Leu Ala Val Thr Ile Ala
   20                  25                  30                  35
   cta ctc atc cac ttc tta gcg ttt gat caa aag tct tac ttt tac cat 262
   Leu Leu Ile His Phe Leu Ala Phe Asp Gln Lys Ser Tyr Phe Tyr His
                   40                  45                  50
   agc agt ttt caa atc cta aat gtc caa tat agt aat caa tta aat tca 310
   Ser Ser Phe Gln Ile Leu Asn Val Gln Tyr Ser Asn Gln Leu Asn Ser
               55                  60                  65
   cca ggg aca caa gaa tac agg act ttg agt gga aga att gaa tct ctg 358
   Pro Gly Thr Gln Glu Tyr Arg Thr Leu Ser Gly Arg Ile Glu Ser Leu
           70                  75                  80
   att act aaa aca ttc aga gaa tca aat tta aga aat cag ttc atc aga 406
   Ile Thr Lys Thr Phe Arg Glu Ser Asn Leu Arg Asn Gln Phe Ile Arg
       85                  90                  95
   gct cat gtt gtc aaa ctg agg caa gaa ggt aat ggt gtg ata gca gat 454
   Ala His Val Val Lys Leu Arg Gln Glu Gly Asn Gly Val Ile Ala Asp
   100                 105                 110                 115
   gtt gtc atg aaa ttt aaa ttc act aga aat aac aat gga gca ttg atg 502
   Val Val Met Lys Phe Lys Phe Thr Arg Asn Asn Asn Gly Ala Leu Met
                   120                 125                 130
   aaa agc aga att aag tct gtt tta cac caa atg ctg aat aat tct gga 550
   Lys Ser Arg Ile Lys Ser Val Leu His Gln Met Leu Asn Asn Ser Gly
   135 140 145
   aac ttg gaa ata agc cct tca act gag ata aca tcc att act gac cag 598
   Asn Leu Glu Ile Ser Pro Ser Thr Glu Ile Thr Ser Ile Thr Asp Gln
   150 155 160
   gat aca gta aat att ttc act gaa gga tgt ggg gcc cgt cca gac cta 646
   Asp Thr Val Asn Ile Phe Thr Glu Gly Cys Gly Ala Arg Pro Asp Leu
   165 170 175
   ata act ttg tct gag gag agg atc cta gga ggc aac aag gct gaa gaa 694
   Ile Thr Leu Ser Glu Glu Arg Ile Leu Gly Gly Asn Lys Ala Glu Glu
   180 185 190 195
   gga gat tgg cca tgg caa gtc agt cta cag aag aat aat gtt cac cac 742
   Gly Asp Trp Pro Trp Gln Val Ser Leu Gln Lys Asn Asn Val His His
   200 205 210
   tgt gga ggt gtc ctg atc agt agc atg tgg atc ctg tca gca gct cac 790
   Cys Gly Gly Val Leu Ile Ser Ser Met Trp Ile Leu Ser Ala Ala His
   215 220 225
   tgc ttc aga agc caa tct aat cct cgt cag tgg act gcc atc ttt ggt 838
   Cys Phe Arg Ser Gln Ser Asn Pro Arg Gln Trp Thr Ala Ile Phe Gly
   230 235 240
   gct tca ata gca ttt cct aaa cag aaa aga aga gta agg act att tta 886
   Ala Ser Ile Ala Phe Pro Lys Gln Lys Arg Arg Val Arg Thr Ile Leu
   245 250 255
   atc cat aac aat tat aac cct gca act cat gaa aat gat att gca gct 934
   Ile His Asn Asn Tyr Asn Pro Ala Thr His Glu Asn Asp Ile Ala Ala
   260 265 270 275
   ata caa ctt gaa gga ggt atc aac ttt acc aaa aat atc cat agg gtg 982
   Ile Gln Leu Glu Gly Gly Ile Asn Phe Thr Lys Asn Ile His Arg Val
   280 285 290
   tgt ctc cca gag gct acc cag aat att cca cct ggt tct tct gct tat 1030
   Cys Leu Pro Glu Ala Thr Gln Asn Ile Pro Pro Gly Ser Ser Ala Tyr
   295 300   305
   gta aca gga tgg gga tct caa gaa tac ggt ggt aac aca gtt tca gat 1078
   Val Thr Gly Trp Gly Ser Gln Glu Tyr Gly Gly Asn Thr Val Ser Asp
   310 315 320
   cta cag caa gca cgg gtc aga ata ata agt aat gat gta tgt aat gca 1126
   Leu Gln Gln Ala Arg Val Arg Ile Ile Ser Asn Asp Val Cys Asn Ala
   325 330 335
   cca act agt tat aac gga gct gtc agg cct gga atg ctc tgt gct ggc 1174
   Pro Thr Ser Tyr Asn Gly Ala Val Arg Pro Gly Met Leu Cys Ala Gly
   340 345 350 355
   cta cct caa ggt gga gtg gat gca tgc cgg ggt gac tcg ggt ggc cca 1222
   Leu Pro Gln Gly Gly Val Asp Ala Cys Arg Gly Asp Ser Gly Gly Pro
   360 365 370
   ctg gtt caa gag gac tca cgg cgg ctt tgg ttc ctc gtg gga ata gta 1270
   Leu Val Gln Glu Asp Ser Arg Arg Leu Trp Phe Leu Val Gly Ile Val
   375 380 385
   agc tgg ggg gac cga tgc ggt ctg cca gat aag cca gga gtg tac act 1318
   Ser Trp Gly Asp Arg Cys Gly Leu Pro Asp Lys Pro Gly Val Tyr Thr
   390 395 400
   cga gtg aca gcc tac cgt gac tgg ata act gaa aaa act gga gtc tag c 1367
   Arg Val Thr Ala Tyr Arg Asp Trp Ile Thr Glu Lys Thr Gly Val
   405 410 415 418
   acaataaatg catctttgtg aaaaaaaaaa aaaaaaaaa 1406
<210> 31
   <211> 418
   <212> PRT
   <213> Canis familiaris
<220>
   <221> propeptide portion
   <222> (1) ... (186)
<220>
   <221> trypsin-like protein portion
   <222> (187) ... (418)
<400> 31
   Met Tyr Arg Pro Ala Arg Val Pro Ser Gly Ser Arg Phe Leu Asn Pro
   1 5 10 15
   Tyr Val Val Cys Phe Val Val Val Ala Gly Val Val Ile Leu Ala Val
   20 25 30
   Thr Ile Ala Leu Leu Ile His Phe Leu Ala Phe Asp Gln Lys Ser Tyr
   35 40 45
   Phe Tyr His Ser Ser Phe Gln Ile Leu Asn Val Gln Tyr Ser Asn Gln
   50 55 60
   Leu Asn Ser Pro Gly Thr Gln Glu Tyr Arg Thr Leu Ser Gly Arg Ile
   65 70 75 80
   Glu Ser Leu Ile Thr Lys Thr Phe Arg Glu Ser Asn Leu Arg Asn Gln
   85 90 95
   Phe Ile Arg Ala His Val Val Lys Leu Arg Gln Glu Gly Asn Gly Val
   100 105 110
   Ile Ala Asp Val Val Met Lys Phe Lys Phe Thr Arg Asn Asn Asn Gly
   115 120 125
   Ala Leu Met Lys Ser Arg Ile Lys Ser Val Leu His Gln Met Leu Asn
   130 135 140
   Asn Ser Gly Asn Leu Glu Ile Ser Pro Ser Thr Glu Ile Thr Ser Ile
   145 150 155 160
   Thr Asp Gln Asp Thr Val Asn Ile Phe Thr Glu Gly Cys Gly Ala Arg
   165 170 175
   Pro Asp Leu Ile Thr Leu Ser Glu Glu Arg Ile Leu Gly Gly Asn Lys
   180 185 190
   Ala Glu Glu Gly Asp Trp Pro Trp Gln Val Ser Leu Gln Lys Asn Asn
   195 200 205
   Val His His Cys Gly Gly Val Leu Ile Ser Ser Met Trp Ile Leu Ser
   210 215 220
   Ala Ala His Cys Phe Arg Ser Gln Ser Asn Pro Arg Gln Trp Thr Ala
   225 230 235 240
   Ile Phe Gly Ala Ser Ile Ala Phe Pro Lys Gln Lys Arg Arg Val Arg
   245 250 255
   Thr Ile Leu Ile His Asn Asn Tyr Asn Pro Ala Thr His Glu Asn Asp
   260 265 270
   Ile Ala Ala Ile Gln Leu Glu Gly Gly Ile Asn Phe Thr Lys Asn Ile
   275 280 285
   His Arg Val Cys Leu Pro Glu Ala Thr Gln Asn Ile Pro Pro Gly Ser
   290 295 300
   Ser Ala Tyr Val Thr Gly Trp Gly Ser Gln Glu Tyr Gly Gly Asn Thr
   305 310 315 320
   Val Ser Asp Leu Gln Gln Ala Arg Val Arg Ile Ile Ser Asn Asp Val
   325 330 335
   Cys Asn Ala Pro Thr Ser Tyr Asn Gly Ala Val Arg Pro Gly Met Leu
   340 345 350
   Cys Ala Gly Leu Pro Gln Gly Gly Val Asp Ala Cys Arg Gly Asp Ser
   355 360 365
   Gly Gly Pro Leu Val Gln Glu Asp Ser Arg Arg Leu Trp Phe Leu Val
   370 375 380
   Gly Ile Val Ser Trp Gly Asp Arg Cys Gly Leu Pro Asp Lys Pro Gly
   385 390 395 400
   Val Tyr Thr Arg Val Thr Ala Tyr Arg Asp Trp Ile Thr Glu Lys Thr
   405 410 415
   Gly Val
   418
<210> 32
   <211> 1381
   <212> DNA
   <213> Bos taurus
<220>
   <221> CDS
   <222> (73)... (1326)
<220>
   <221> propeptide portion
   <222> (73) ... (627)
<220>
   <221> tryps in-like protein portion
   <222> (628) ... (1326)
<400> 32
   gggattcatt tgagtggaaa tctcagagca gttgcatagg cggaaaaaag gacttcatta 60
   ttaaggatta aa atg tat agg cca gca cga gtg aca tca gcc tca aga tcc 111
   Met Tyr Arg Pro Ala Arg Val Thr Ser Ala Ser Arg Ser
   1 5 10
   ctg aat cca tat cta gta ttt ttt gtt gtc gtc gca gtg gtg gtg atc 159
   Leu Asn Pro Tyr Leu Val Phe Phe Val Val Val Ala Val Val Val Ile
   15 20 25
   ctg gca gtg atc ata ggt cta ctt gtc tac ttt tta gct ttt gat caa 207
   Leu Ala Val Ile Ile Gly Leu Leu Val Tyr Phe Leu Ala Phe Asp Gln
   30 35 40 45
   aaa tct tac ttt tac caa agc agc att caa atc ttg ggt aaa caa tat 255
   Lys Ser Tyr Phe Tyr Gln Ser Ser Ile Gln Ile Leu Gly Lys Gln Tyr
   50 55 60
   agt gat gag tta agt tca cca gct aca gag aaa tat agg act ttg agt 303
   Ser Asp Glu Leu Ser Ser Pro Ala Thr Glu Lys Tyr Arg Thr Leu Ser
   65 70 75
   gga aga att gaa tct atg att act aaa aca ttc aaa gag tca gat tta 351
   Gly Arg Ile Glu Ser Met Ile Thr Lys Thr Phe Lys Glu Ser Asp Leu
   80 85 90
   aaa aat gag ttc atc aaa gct cat gtt gtc aaa ctg agg caa agt ggc 399
   Lys Asn Glu Phe Ile Lys Ala His Val Val Lys Leu Arg Gln Ser Gly
   95 100 105
   aat aat gtg ata gca gat att atc atg aaa ttt aaa ttc acc aga aga 447
   Asn Asn Val Ile Ala Asp Ile Ile Met Lys Phe Lys Phe Thr Arg Arg
   110 115 120 125
   atc aat gaa gca tca atg aaa agc aga att gag tct att tta cgc caa 495
   Ile Asn Glu Ala Ser Met Lys Ser Arg Ile Glu Ser Ile Leu Arg Gln
   130 135 140
   atg ccg aat aac tct gaa gac ttg aac atg aat cca act cag gta ata 543
   Met Pro Asn Asn Ser Glu Asp Leu Asn Met Asn Pro Thr Gln Val Ile
   145 150 155
   tca ata act ggc cag gat aca ata caa ctg ttc act cga gaa tgt ggg 591
   Ser Ile Thr Gly Gln Asp Thr Ile Gln Leu Phe Thr Arg Glu Cys Gly
   160 165 170
   gtc cgc tca gac ctg ata acc ttg tct gag gag aga atc ata gga ggc 639
   Val Arg Ser Asp Leu Ile Thr Leu Ser Glu Glu Arg Ile Ile Gly Gly
   175 180 185
   agt aaa gct gag aaa gga gac tgg cca tgg caa gtc agt cta cag tgg 687
   Ser Lys Ala Glu Lys Gly Asp Trp Pro Trp Gln Val Ser Leu Gln Trp
   190 195 200 205
   agc agt tct cac cgc tgc gga gga gcc ttg atc agt aat agg tgg atc 735
   Ser Ser Ser His Arg Cys Gly Gly Ala Leu Ile Ser Asn Arg Trp Ile
   210 215 220
   ctg tca gca gct cac tgc ttc aga agc cac tct gat cct cgc caa tgg 783
   Leu Ser Ala Ala His Cys Phe Arg Ser His Ser Asp Pro Arg Gln Trp
   225 230 235
   att gcc acc ttt ggt act tcc aca ata tct cct caa ctg aga gta gga 831
   Ile Ala Thr Phe Gly Thr Ser Thr Ile Ser Pro Gln Leu Arg Val Gly
   240 245 250
   gta agg aat att tta atc cat gac aat tat aaa cct gaa act cat gaa 879
   Val Arg Asn Ile Leu Ile His Asp Asn Tyr Lys Pro Glu Thr His Glu
   255 260 265
   aac gat att gca ctc gta caa ctt gat aga gaa gtc acc ttt aac aga 927
   Asn Asp Ile Ala Leu Val Gln Leu Asp Arg Glu Val Thr Phe Asn Arg
   270 275 280 285
   tat att cat aca gtg tgt ctc cca gag gct aac cag gcc att tca gct 975
   Tyr Ile His Thr Val Cys Leu Pro Glu Ala Asn Gln Ala Ile Ser Ala
   290 295 300
   ggt tcc act gct tat gta aca gga tgg gga tct cag agt tat agc ggc 1023
   Gly Ser Thr Ala Tyr Val Thr Gly Trp Gly Ser Gln Ser Tyr Ser Gly
   305 310 315
   agc aca gtt tca gat cta aac caa gga cgg gtc aat ata ata agt aat 1071
   Ser Thr Val Ser Asp Leu Asn Gln Gly Arg Val Asn Ile Ile Ser Asn
   320 325 330
   act gta tgt aac aca cca gct ggt tat aat gga gcc gtc ctg tct gga 1119
   Thr Val Cys Asn Thr Pro Ala Gly Tyr Asn Gly Ala Val Leu Ser Gly
   335 340 345
   atg cta tgt gct gga cta cct gaa ggt gga gtg gac gcg tgc cag ggt 1167
   Met Leu Cys Ala Gly Leu Pro Glu Gly Gly Val Asp Ala Cys Gln Gly
   350 355 360 365
   gac tct ggt ggc cct cta gta caa gaa gac tca cgg caa cac tgg ttc 1215
   Asp Ser Gly Gly Pro Leu Val Gln Glu Asp Ser Arg Gln His Trp Phe
   370 375 380
   atc gtg ggg ata gta agc tgg ggg tat caa tgt ggt ctg cca gat aaa 1263
   Ile Val Gly Ile Val Ser Trp Gly Tyr Gln Cys Gly Leu Pro Asp Lys
   385 390 395
   cca gga gta tac act aga gtg aca gcc tac cgt gac tgg ata acc caa 1311
   Pro Gly Val Tyr Thr Arg Val Thr Ala Tyr Arg Asp Trp Ile Thr Gln
   400 405 410
   caa act ggt atc tag tgcaataaat acatcttggt tcaagagcca aaaaaaaaaa a 1367
   Gln Thr Gly Ile
   415 417
   aaaaaaaaaa aaaa 1381
<210> 33
   <211> 417
   <212> PRT
   <213> Bos taurus
<220>
   <221> propeptide portion
   <222> (1) ... (185)
<220>
   <221> trypsin-like protein portion
   <222> (186)... (417)
<400> 33
   Met Tyr Arg Pro Ala Arg Val Thr Ser Ala Ser Arg Ser Leu Asn Pro
   1 5 10 15
   Tyr Leu Val Phe Phe Val Val Val Ala Val Val Val Ile Leu Ala Val
   20 25 30
   Ile Ile Gly Leu Leu Val Tyr Phe Leu Ala Phe Asp Gln Lys Ser Tyr
   35 40 45
   Phe Tyr Gln Ser Ser Ile Gln Ile Leu Gly Lys Gln Tyr Ser Asp Glu
   50 55 60
   Leu Ser Ser Pro Ala Thr Glu Lys Tyr Arg Thr Leu Ser Gly Arg Ile
   65 70 75 80
   Glu Ser Met Ile Thr Lys Thr Phe Lys Glu Ser Asp Leu Lys Asn Glu
   85 90 95
   Phe Ile Lys Ala His Val Val Lys Leu Arg Gln Ser Gly Asn Asn Val
   100 105 110
   Ile Ala Asp Ile Ile Met Lys Phe Lys Phe Thr Arg Arg Ile Asn Glu
   115 120 125
   Ala Ser Met Lys Ser Arg Ile Glu Ser Ile Leu Arg Gln Met Pro Asn
   130 135 140
   Asn Ser Glu Asp Leu Asn Met Asn Pro Thr Gln Val Ile Ser Ile Thr
   145 150 155 160
   Gly Gln Asp Thr Ile Gln Leu Phe Thr Arg Glu Cys Gly Val Arg Ser
   165 170 175
   Asp Leu Ile Thr Leu Ser Glu Glu Arg Ile Ile Gly Gly Ser Lys Ala
   180 185 190
   Glu Lys Gly Asp Trp Pro Trp Gln Val Ser Leu Gln Trp Ser Ser Ser
   195 200 205
   His Arg Cys Gly Gly Ala Leu Ile Ser Asn Arg Trp Ile Leu Ser Ala
   210 215 220
   Ala His Cys Phe Arg Ser His Ser Asp Pro Arg Gln Trp Ile Ala Thr
   225 230 235 240
   Phe Gly Thr Ser Thr Ile Ser Pro Gln Leu Arg Val Gly Val Arg Asn
   245 250 255
   Ile Leu Ile His Asp Asn Tyr Lys Pro Glu Thr His Glu Asn Asp Ile
   260 265 270
   Ala Leu Val Gln Leu Asp Arg Glu Val Thr Phe Asn Arg Tyr Ile His
   275 280 285
   Thr Val Cys Leu Pro Glu Ala Asn Gln Ala Ile Ser Ala Gly Ser Thr
   290 295 300
   Ala Tyr Val Thr Gly Trp Gly Ser Gln Ser Tyr Ser Gly Ser Thr Val
   305 310 315 320
   Ser Asp Leu Asn Gln Gly Arg Val Asn Ile Ile Ser Asn Thr Val Cys
   325 330 335
   Asn Thr Pro Ala Gly Tyr Asn Gly Ala Val Leu Ser Gly Met Leu Cys
   340 345 350
   Ala Gly Leu Pro Glu Gly Gly Val Asp Ala Cys Gln Gly Asp Ser Gly
   355 360 365
   Gly Pro Leu Val Gln Glu Asp Ser Arg Gln His Trp Phe Ile Val Gly
   370 375 380
   Ile Val Ser Trp Gly Tyr Gln Cys Gly Leu Pro Asp Lys Pro Gly Val
   385 390 395 400
   Tyr Thr Arg Val Thr Ala Tyr Arg Asp Trp Ile Thr Gln Gln Thr Gly
   405 410 415
   Ile
   417
<210> 34
   <211> 1419
   <212> DNA
   <213> Oryctolagus cuniculus
<220>
   <221> CDS
   <222> (73) ... (1329)
<220>
   <221> propeptide portion
   <222> (73) ... (630)
<220>
   <221> trypsin-like protein portion
   <222> (631) ... (1329)
<400> 34
   gtcacttgag tgggaatctc aaagcagttg gattggcaga aaaataggac ctcttcgcta 60
   ctcaggatta aa atg tat agg cca gca cgg gga tca tcg act tca aga ttc 111
   Met Tyr Arg Pro Ala Arg Gly Ser Ser Thr Ser Arg Phe
   1 5 10
   ctg aat cca tac gtg att tgt ttc att gtt gta gca gtc gtg gtg atc 159
   Leu Asn Pro Tyr Val Ile Cys Phe Ile Val Val Ala Val Val Val Ile
   15 20 25
   ctg gca gtg atc gta gct cta ctt att cac ttt tta gct ttt gat aaa 207
   Leu Ala Val Ile Val Ala Leu Leu Ile His Phe Leu Ala Phe Asp Lys
   30 35 40 45
   aag tct tgc ttt ttt cac agc agc ttt caa att cga aat gtt caa tat 255
   Lys Ser Cys Phe Phe His Ser Ser Phe Gln Ile Arg Asn Val Gln Tyr
   50 55 60
   agc gat cag tta aat tca cca gct aca cag gaa tac aga tta ttg agt 303
   Ser Asp Gln Leu Asn Ser Pro Ala Thr Gln Glu Tyr Arg Leu Leu Ser
   65 70 75
   gaa aga att gaa tct atg att agt caa aca cta caa gga tca aac tta 351
   Glu Arg Ile Glu Ser Met Ile Ser Gln Thr Leu Gln Gly Ser Asn Leu
   80 85 90
   aga aat cag ttc att aga gct cat gtt gtc aaa ctg agg cag gat agt 399
   Arg Asn Gln Phe Ile Arg Ala His Val Val Lys Leu Arg Gln Asp Ser
   95 100 105
   aat agt gtg ata gca gat gtt gta atg aaa ttc cga gtc agt aga aac 447
   Asn Ser Val Ile Ala Asp Val Val Met Lys Phe Arg Val Ser Arg Asn
   110 115 120 125
   aac aat ggt gat gca atg aaa aga agg gtt cag gat gtt tta cag caa 495
   Asn Asn Gly Asp Ala Met Lys Arg Arg Val Gln Asp Val Leu Gln Gln
   130 135 140
   atg ctg aat aac tct gga agc ttg gaa ata aac cct tca act acg gta 543
   Met Leu Asn Asn Ser Gly Ser Leu Glu Ile Asn Pro Ser Thr Thr Val
   145 150 155
   aca gag att act ggc cag gat aca gaa act atc ttc acc caa caa tgt 591
   Thr Glu Ile Thr Gly Gln Asp Thr Glu Thr Ile Phe Thr Gln Gln Cys
   160 165 170
   ggg gcc cgt cca gac cta ata aca ttg tct gaa gag aga atc att gga 639
   Gly Ala Arg Pro Asp Leu Ile Thr Leu Ser Glu Glu Arg Ile Ile Gly
   175 180 185
   ggc acc cag gct gag gag gga gac tgg ccc tgg caa gtc agt ctc cag 687
   Gly Thr Gln Ala Glu Glu Gly Asp Trp Pro Trp Gln Val Ser Leu Gln
   190 195 200 205
   ccc aac aat gct cac cat tgt gga ggc att ttg atc agt aac acg tgg 735
   Pro Asn Asn Ala His His Cys Gly Gly Ile Leu Ile Ser Asn Thr Trp
   210 215 220
   atc ctg aca gca gca cac tgc ttc cgg agc tac tct gat cct cgt caa 783
   Ile Leu Thr Ala Ala His Cys Phe Arg Ser Tyr Ser Asp Pro Arg Gln
   225 230 235
   tgg act gcc acc ttt ggt att tcc aca gca gtt tct aga cag aga atg 831
   Trp Thr Ala Thr Phe Gly Ile Ser Thr Ala Val Ser Arg Gln Arg Met
   240 245 250
   aga ata agg aca att tta gtc cat aac aat tat aga tct gca acg cat 879
   Arg Ile Arg Thr Ile Leu Val His Asn Asn Tyr Arg Ser Ala Thr His
   255 260 265
   gaa aat gat att gca gct gtg caa ctt gaa gga gct atc aca ttt aca 927
   Glu Asn Asp Ile Ala Ala Val Gln Leu Glu Gly Ala Ile Thr Phe Thr
   270 275 280 285
   aga aac atc cat agt gtg tgt ctc cca gag gcc act cag aac att aca 975
   Arg Asn Ile His Ser Val Cys Leu Pro Glu Ala Thr Gln Asn Ile Thr
   290 295 300
   cct ggt tct tca gct tat gta aca gga tgg ggg tct cta gaa tat ggt 1023
   Pro Gly Ser Ser Ala Tyr Val Thr Gly Trp Gly Ser Leu Glu Tyr Gly
   305 310 315
   ggc aac acg gtt acc tat cta cag caa gga cgg gtc aga ata ata agt 1071
   Gly Asn Thr Val Thr Tyr Leu Gln Gln Gly Arg Val Arg Ile Ile Ser
   320 325 330
   aat gaa gta tgt aac gca cca gcg agt tac aat ggc gct gtc ttg cct 1119
   Asn Glu Val Cys Asn Ala Pro Ala Ser Tyr Asn Gly Ala Val Leu Pro
   335 340 345
   aca atg gtg tgt gca gga tta tct caa gga gga gtg gac gca tgc cag 1167
   Thr Met Val Cys Ala Gly Leu Ser Gln Gly Gly Val Asp Ala Cys Gln
   350 355 360 365
   ggt gac tct ggt ggc cca ctg gta caa gaa gac tcg cgc cgg ctt tgg 1215
   Gly Asp Ser Gly Gly Pro Leu Val Gln Glu Asp Ser Arg Arg Leu Trp
   370 375 380
   ttc gtt gtg ggc att gta agc tgg ggc tat cag tgt ggc ctg cct gac 1263
   Phe Val Val Gly Ile Val Ser Trp Gly Tyr Gln Cys Gly Leu Pro Asp
   385 390 395
   aaa cca gga gtg tac aca cga gtg aca gcc tac cgt gac tgg att aga 1311
   Lys Pro Gly Val Tyr Thr Arg Val Thr Ala Tyr Arg Asp Trp Ile Arg
   400 405 410
   gaa caa acg ggg atc tag ctggataaat gcacctctgc tgcaaagccg gttcgcaa 1367
   Glu Gln Thr Gly Ile
   415 418
   ctgagcctgt caaaaatcca agctttaatt ctccgctgaa aagaacagga ag 1419
<210> 35
   <211> 418
   <212> PRT
   <213> Oryctolagus cuniculus
<220>
   <221> propeptide portion
   <222> (1)... (186)
<220>
   <221> tryps in-like protein portion
   <222> (187) ... (418)
<400> 35
   Met Tyr Arg Pro Ala Arg Gly Ser Ser Thr Ser Arg Phe Leu Asn Pro
   1 5 10 15
   Tyr Val Ile Cys Phe Ile Val Val Ala Val Val Val Ile Leu Ala Val
   20 25 30
   Ile Val Ala Leu Leu Ile His Phe Leu Ala Phe Asp Lys Lys Ser Cys
   35 40 45
   Phe Phe His Ser Ser Phe Gln Ile Arg Asn Val Gln Tyr Ser Asp Gln
   50 55 60
   Leu Asn Ser Pro Ala Thr Gln Glu Tyr Arg Leu Leu Ser Glu Arg Ile
   65 70 75 80
   Glu Ser Met Ile Ser Gln Thr Leu Gln Gly Ser Asn Leu Arg Asn Gln
   85 90 95
   Phe Ile Arg Ala His Val Val Lys Leu Arg Gln Asp Ser Asn Ser Val
   100 105 110
   Ile Ala Asp Val Val Met Lys Phe Arg Val Ser Arg Asn Asn Asn Gly
   115 120 125
   Asp Ala Met Lys Arg Arg Val Gln Asp Val Leu Gln Gln Met Leu Asn
   130 135 140
   Asn Ser Gly Ser Leu Glu Ile Asn Pro Ser Thr Thr Val Thr Glu Ile
   145 150 155 160
   Thr Gly Gln Asp Thr Glu Thr Ile Phe Thr Gln Gln Cys Gly Ala Arg
   165 170 175
   Pro Asp Leu Ile Thr Leu Ser Glu Glu Arg Ile Ile Gly Gly Thr Gln
   180 185 190
   Ala Glu Glu Gly Asp Trp Pro Trp Gln Val Ser Leu Gln Pro Asn Asn
   195 200 205
   Ala His His Cys Gly Gly Ile Leu Ile Ser Asn Thr Trp Ile Leu Thr
   210 215 220
   Ala Ala His Cys Phe Arg Ser Tyr Ser Asp Pro Arg Gln Trp Thr Ala
   225 230 235 240
   Thr Phe Gly Ile Ser Thr Ala Val Ser Arg Gln Arg Met Arg Ile Arg
   245 250 255
   Thr Ile Leu Val His Asn Asn Tyr Arg Ser Ala Thr His Glu Asn Asp
   260 265 270
   Ile Ala Ala Val Gln Leu Glu Gly Ala Ile Thr Phe Thr Arg Asn Ile
   275 280 285
   His Ser Val Cys Leu Pro Glu Ala Thr Gln Asn Ile Thr Pro Gly Ser
   290 295 300
   Ser Ala Tyr Val Thr Gly Trp Gly Ser Leu Glu Tyr Gly Gly Asn Thr
   305 310 315 320
   Val Thr Tyr Leu Gln Gln Gly Arg Val Arg Ile Ile Ser Asn Glu Val
   325 330 335
   Cys Asn Ala Pro Ala Ser Tyr Asn Gly Ala Val Leu Pro Thr Met Val
   340 345 350
   Cys Ala Gly Leu Ser Gln Gly Gly Val Asp Ala Cys Gln Gly Asp Ser
   355 360 365
   Gly Gly Pro Leu Val Gln Glu Asp Ser Arg Arg Leu Trp Phe Val Val
   370 375 380
   Gly Ile Val Ser Trp Gly Tyr Gln Cys Gly Leu Pro Asp Lys Pro Gly
   385 390 395 400
   Val Tyr Thr Arg Val Thr Ala Tyr Arg Asp Trp Ile Arg Glu Gln Thr
   405 410 415
   Gly Ile
   418
<210> 36
   <211> 1576
   <212> DNA
   <213> Cavia porcellus
<220>
   <221> CDS
   <222> (102) ... (1358)
<220>
   <221> propeptide portion
   <222> (102) ... (659)
<220>
   <221> trypsin-like protein portion
   <222> (660) ... (1358)
<400> 36
   ggacttctgc tcctcagaca ctcagcactc cagtcatttc agtgggaatc tcaaaggagt 60
   tgacaagcag gagaacgatc gcttcatctt tcaggataaa g atg tat agg cca gca 116
   Met Tyr Arg Pro Ala
   1 5
   aca gtg tca tct aac tcg aga ttc ctg aac ccc tgg gta gtc ttc ttc 164
   Thr Val Ser Ser Asn Ser Arg Phe Leu Asn Pro Trp Val Val Phe Phe
   10 15 20
   act gtg ttc gcg gtg gtg ggg atc ctg gcc atg att gta gga ctg ctc 212
   Thr Val Phe Ala Val Val Gly Ile Leu Ala Met Ile Val Gly Leu Leu
   25 30 35
   att cac ttt tta gct ttt gat caa aaa tct tgc ttt tac cac agt gat 260
   Ile His Phe Leu Ala Phe Asp Gln Lys Ser Cys Phe Tyr His Ser Asp
   40 45 50
   gtt caa ata atg aat gtt gaa tac aat gat cag tta agc tca ccc ggt 308
   Val Gln Ile Met Asn Val Glu Tyr Asn Asp Gln Leu Ser Ser Pro Gly
   55 60 65
   aca caa gaa tac agg att ttg agt gaa agg att gaa tct atg atc act 356
   Thr Gln Glu Tyr Arg Ile Leu Ser Glu Arg Ile Glu Ser Met Ile Thr
   70 75 80 85
   aat gca ttc cag cag tcc aat tta aga aat cag ttt atc aga gcc cat 404
   Asn Ala Phe Gln Gln Ser Asn Leu Arg Asn Gln Phe Ile Arg Ala His
   90 95 100
   gtt gta aga ctg agg caa gag ggt aac ggt gtg gta gca gat gtt gtc 452
   Val Val Arg Leu Arg Gln Glu Gly Asn Gly Val Val Ala Asp Val Val
   105 110 115
   atg aag ttt cga ttc agt aga cgt aac aat gga gaa tcc atg aaa gcc 500
   Met Lys Phe Arg Phe Ser Arg Arg Asn Asn Gly Glu Ser Met Lys Ala
   120 125 130
   aga att cag tct att tta cag caa atg ctg aat aac tct gga aac ctg 548
   Arg Ile Gln Ser Ile Leu Gln Gln Met Leu Asn Asn Ser Gly Asn Leu
   135 140 145
   gaa ata agc cct tca gcc ggg gta aca gaa att aac gac cag gaa aca 596
   Glu Ile Ser Pro Ser Ala Gly Val Thr Glu Ile Asn Asp Gln Glu Thr
   150 155 160 165
   gaa aat atg ttt act caa gca tgc ggg gcc cgt cca gac ctg atg acg 644
   Glu Asn Met Phe Thr Gln Ala Cys Gly Ala Arg Pro Asp Leu Met Thr
   170 175 180
   ctg tct gca gag aga gtc gtt gga ggt act caa gct gac cag ggc gac 692
   Leu Ser Ala Glu Arg Val Val Gly Gly Thr Gln Ala Asp Gln Gly Asp
   185 190 195
   tgg ccg tgg caa gtc agt ctg cag gtc aac ggt ggc cat cgc tgc gga 740
   Trp Pro Trp Gln Val Ser Leu Gln Val Asn Gly Gly His Arg Cys Gly
   200 205 210
   ggc gtc ctg gtc agc aac cag tgg gtc ctg act gca gcc cac tgc ttc 788
   Gly Val Leu Val Ser Asn Gln Trp Val Leu Thr Ala Ala His Cys Phe
   215 220 225
   aga agc tac cct aat gct caa caa tgg act gcc acc ttt ggt att tcc 836
   Arg Ser Tyr Pro Asn Ala Gln Gln Trp Thr Ala Thr Phe Gly Ile Ser
   230 235 240 245
   aca acc tct cct act ctg aga gtg aga gtg agg act att tca atc cac 884
   Thr Thr Ser Pro Thr Leu Arg Val Arg Val Arg Thr Ile Ser Ile His
   250 255 260
   aac aat tac aat cct gtg act cat gag aat gat att gca gct gtg cag 932
   Asn Asn Tyr Asn Pro Val Thr His Glu Asn Asp Ile Ala Ala Val Gln
   265 270 275
   ctg gaa agg gct gtc acc ttc acc agg gat gtt cac cga gta tgt ctc 980
   Leu Glu Arg Ala Val Thr Phe Thr Arg Asp Val His Arg Val Cys Leu
   280 285 290
   ccc gca gcc acc cag act gtc aca cct ggt tct aca gct tat gta aca 1028
   Pro Ala Ala Thr Gln Thr Val Thr Pro Gly Ser Thr Ala Tyr Val Thr
   295 300 305
   gga tgg ggc tcg ata atc tat ggt ggc aac acg gtc aga tat cta cgg 1076
   Gly Trp Gly Ser Ile Ile Tyr Gly Gly Asn Thr Val Arg Tyr Leu Arg
   310 315 320 325
   caa gga caa gtc cag ata ata agt act agt gag tgt aac gca cca gcc 1124
   Gln Gly Gln Val Gln Ile Ile Ser Thr Ser Glu Cys Asn Ala Pro Ala
   330 335 340
   agt tac aac ggt gct gtc ctg cct ggg atg ctg tgt gct ggc gtg ccg 1172
   Ser Tyr Asn Gly Ala Val Leu Pro Gly Met Leu Cys Ala Gly Val Pro
   345 350 355
   aca ggt gca gtg gac gcg tgc cag gga gat tct ggt ggc cca cta gtc 1220
   Thr Gly Ala Val Asp Ala Cys Gln Gly Asp Ser Gly Gly Pro Leu Val
   360 365 370
   caa gaa gac tca cgg cgg ctt tgg ttc ctg gtg gga ata gtg agc tgg 1268
   Gln Glu Asp Ser Arg Arg Leu Trp Phe Leu Val Gly Ile Val Ser Trp
   375 380 385
   ggc tat cag tgt ggt gtg ccc gac aag ccc gga gta tat act cga gtg 1316
   Gly Tyr Gln Cys Gly Val Pro Asp Lys Pro Gly Val Tyr Thr Arg Val
   390 395 400 405
   acc aca tac cgt aac tgg att aga caa gta act ggg gtc tag cgcaaccca 1367
   Thr Thr Tyr Arg Asn Trp Ile Arg Gln Val Thr Gly Val
   410 415 418
   tgcatttctg ttgcaaagtg atcaagtgca ataaatgcat ctctgttgca aagtctgaat 1427
   gcaggtgtgc ctggctacaa ttccaaagct ttacttttca gcagaaaaat aaagctgcgc 1487
   ttgtttcatt tttacatact gttacaaaat agagcagaat aaaacattat tcactcttcc 1547
   tcaaaaaaaa aaaaaaaaaa aaaaaaaaa 1576
<210> 37
   <211> 418
   <212> PRT
   <213> Cavia porcellus
<220>
   <221> propeptide portion
   <222> (1)... (186)
<220>
   <221> trypsin-like protein portion
   <222> (187)... (418)
<400> 37
   Met Tyr Arg Pro Ala Thr Val Ser Ser Asn Ser Arg Phe Leu Asn Pro
   1 5 10 15
   Trp Val Val Phe Phe Thr Val Phe Ala Val Val Gly Ile Leu Ala Met
   20 25 30
   Ile Val Gly Leu Leu Ile His Phe Leu Ala Phe Asp Gln Lys Ser Cys
   35 40 45
   Phe Tyr His Ser Asp Val Gln Ile Met Asn Val Glu Tyr Asn Asp Gln
   50 55 60
   Leu Ser Ser Pro Gly Thr Gln Glu Tyr Arg Ile Leu Ser Glu Arg Ile
   65 70 75 80
   Glu Ser Met Ile Thr Asn Ala Phe Gln Gln Ser Asn Leu Arg Asn Gln
   85 90 95
   Phe Ile Arg Ala His Val Val Arg Leu Arg Gln Glu Gly Asn Gly Val
   100 105 110
   Val Ala Asp Val Val Met Lys Phe Arg Phe Ser Arg Arg Asn Asn Gly
   115 120 125
   Glu Ser Met Lys Ala Arg Ile Gln Ser Ile Leu Gln Gln Met Leu Asn
   130 135 140
   Asn Ser Gly Asn Leu Glu Ile Ser Pro Ser Ala Gly Val Thr Glu Ile
   145 150 155 160
   Asn Asp Gln Glu Thr Glu Asn Met Phe Thr Gln Ala Cys Gly Ala Arg
   165 170 175
   Pro Asp Leu Met Thr Leu Ser Ala Glu Arg Val Val Gly Gly Thr Gln
   180 185 190
   Ala Asp Gln Gly Asp Trp Pro Trp Gln Val Ser Leu Gln Val Asn Gly
   195 200 205
   Gly His Arg Cys Gly Gly Val Leu Val Ser Asn Gln Trp Val Leu Thr
   210 215 220
   Ala Ala His Cys Phe Arg Ser Tyr Pro Asn Ala Gln Gln Trp Thr Ala
   225 230 235 240
   Thr Phe Gly Ile Ser Thr Thr Ser Pro Thr Leu Arg Val Arg Val Arg
   245 250 255
   Thr Ile Ser Ile His Asn Asn Tyr Asn Pro Val Thr His Glu Asn Asp
   260 265 270
   Ile Ala Ala Val Gln Leu Glu Arg Ala Val Thr Phe Thr Arg Asp Val
   275 280 285
   His Arg Val Cys Leu Pro Ala Ala Thr Gln Thr Val Thr Pro Gly Ser
   290 295 300
   Thr Ala Tyr Val Thr Gly Trp Gly Ser Ile Ile Tyr Gly Gly Asn Thr
   305 310 315 320
   Val Arg Tyr Leu Arg Gln Gly Gln Val Gln Ile Ile Ser Thr Ser Glu
   325 330 335
   Cys Asn Ala Pro Ala Ser Tyr Asn Gly Ala Val Leu Pro Gly Met Leu
   340 345 350
   Cys Ala Gly Val Pro Thr Gly Ala Val Asp Ala Cys Gln Gly Asp Ser
   355 360 365
   Gly Gly Pro Leu Val Gln Glu Asp Ser Arg Arg Leu Trp Phe Leu Val
   370 375 380
   Gly Ile Val Ser Trp Gly Tyr Gln Cys Gly Val Pro Asp Lys Pro Gly
   385 390 395 400
   Val Tyr Thr Arg Val Thr Thr Tyr Arg Asn Trp Ile Arg Gln Val Thr
   405 410 415
   Gly Val
   418
<210> 38
   <211> 1538
   <212> DNA
   <213> Mesocricetus auratus
<220>
   <221> CDS
   <222> (206)... (1459)
<220>
   <221> propeptide portion
   <222> (206)...(760)
<220>
   <221> trypsin-like protein portion
   <222> (761)... (1459)
<400> 38
   gtcccagtct ctaattctgg tcccaagtac agtgttggtt caagttcagt ctttcagact 60
   tgttttgcaa gttaggtttt aattacatgg gatttcctcc tttggcttca gatacacata 120
   gcacaaaaga gtcactcaat tgagtgggaa tctcaaagca tctggacaca cagaagaaag 180
   gacctcgcca ttactcagga gtaaa atg tat agg cca aga cca gtg ata cca 232
   Met Tyr Arg Pro Arg Pro Val Ile Pro
   1 5
   caa tca agg ttc ttc agt ccg ttt gta gtg gct ttt gtt gtc ata aca 280
   Gln Ser Arg Phe Phe Ser Pro Phe Val Val Ala Phe Val Val Ile Thr
   10 15 20 25
   acR gta ctg atc ctg gcc atg act ata ggt cta ctt att cac ttc tta 328
   Thr Val Leu Ile Leu Ala Met Thr Ile Gly Leu Leu Ile His Phe Leu
   30 35 40
   gct ttt gac aag aaa act tac ttt tac cac agc agc ttt caa atc cta 376
   Ala Phe Asp Lys Lys Thr Tyr Phe Tyr His Ser Ser Phe Gln Ile Leu
   45 50 55
   aat gtt gaa tac act gag gct tta aat tcc cca gct aca cag gaa tac 424
   Asn Val Glu Tyr Thr Glu Ala Leu Asn Ser Pro Ala Thr Gln Glu Tyr
   60 65 70
   agg cat ttg agt gaa aga att gaa tct atg att act gat gca ttc cga 472
   Arg His Leu Ser Glu Arg Ile Glu Ser Met Ile Thr Asp Ala Phe Arg
   75 80 85
   gaa tca aat tta aga agt gag ttt atc aga aca cac gtt gtt aaa ctg 520
   Glu Ser Asn Leu Arg Ser Glu Phe Ile Arg Thr His Val Val Lys Leu
   90 95 100 105
   agg aaa gaa ggg aat ggt gtg atc gca gat gct gtc atg aaa ttt cga 568
   Arg Lys Glu Gly Asn Gly Val Ile Ala Asp Ala Val Met Lys Phe Arg
   110 115 120
   tct agt aaa cgc agc aat aga aaa tcc atg aaa aac aga att cat tct 616
   Ser Ser Lys Arg Ser Asn Arg Lys Ser Met Lys Asn Arg Ile His Ser
   125 130 135
   gtg cta caa ata ctg agt aac tct gga agt ttg gaa ata acc cct tca 664
   Val Leu Gln Ile Leu Ser Asn Ser Gly Ser Leu Glu Ile Thr Pro Ser
   140 145 150
   aat gag ata aca tca ctc act gac caa gat aca gaa aat ttt ttg act 712
   Asn Glu Ile Thr Ser Leu Thr Asp Gln Asp Thr Glu Asn Phe Leu Thr
   155 160 165
   caa gaa tgt gga gcc cgt cca gac ctt ata aca ctg tca gaa gag aga 760
   Gln Glu Cys Gly Ala Arg Pro Asp Leu Ile Thr Leu Ser Glu Glu Arg
   170 175 180 185
   gtt att gga ggc act cta gct gaa aca ggt gac tgg ccc tgg caa gtc 808
   Val Ile Gly Gly Thr Leu Ala Glu Thr Gly Asp Trp Pro Trp Gln Val
   190 195 200
   agt cta caa ctc aat aat gtc cac cac tgt gga ggt atc ctg atc agt 856
   Ser Leu Gln Leu Asn Asn Val His His Cys Gly Gly Ile Leu Ile Ser
   205 210 215
   aac ttg tgg gtc ctg aca gca gct cac tgc ttc aga agc tac tct aat 904
   Asn Leu Trp Val Leu Thr Ala Ala His Cys Phe Arg Ser Tyr Ser Asn
   220 225 230
   cct cta caa tgg act gcc acc ttt ggt gtt tct aca ata cgt cct atc 952
   Pro Leu Gln Trp Thr Ala Thr Phe Gly Val Ser Thr Ile Arg Pro Ile
   235 240 245
   tta aga gta aga gta agg tct att gta tcc cat aac aat tac aga cct 1000
   Leu Arg Val Arg Val Arg Ser Ile Val Ser His Asn Asn Tyr Arg Pro
   250 255 260 265
   aca act cgt gat aat gat att gca gtt gta caa ctt gaa aga cct atc 1048
   Thr Thr Arg Asp Asn Asp Ile Ala Val Val Gln Leu Glu Arg Pro Ile
   270 275 280
   acc ttt aac aga aat atc cac agg gtg tgt ctc cca gct gcg acc caa 1096
   Thr Phe Asn Arg Asn Ile His Arg Val Cys Leu Pro Ala Ala Thr Gln
   285 290 295
   agt atc ata cct ggt tct att gca tat gtc aca gga tgg ggg tcg ctc 1144
   Ser Ile Ile Pro Gly Ser Ile Ala Tyr Val Thr Gly Trp Gly Ser Leu
   300 305 310
   act tat gga ggc aac aca gtc acc gat cta cgg caa gga cag gtc aga 1192
   Thr Tyr Gly Gly Asn Thr Val Thr Asp Leu Arg Gln Gly Gln Val Arg
   315 320 325
   ata gta agt acc gac gag tgc aat gaa cca gct ggg tac agt gga agc 1240
   Ile Val Ser Thr Asp Glu Cys Asn Glu Pro Ala Gly Tyr Ser Gly Ser
   330 335 340 345
   gtc ttg cct gga atg ctc tgt gct gga gtg cct tca ggt gct gtg gat 1288
   Val Leu Pro Gly Met Leu Cys Ala Gly Val Pro Ser Gly Ala Val Asp
   350 355 360
   gcg tgc caa ggt gat tct ggt ggc cca cta gta cag gaa gac tca cgg 1336
   Ala Cys Gln Gly Asp Ser Gly Gly Pro Leu Val Gln Glu Asp Ser Arg
   365 370 375
   atg ctt tgg ttt gtt gtg ggg att gta agc tgg gga tat cag tgc ggc 1384
   Met Leu Trp Phe Val Val Gly Ile Val Ser Trp Gly Tyr Gln Cys Gly
   380 385 390
   ctg cca aat aaa cca gga gtg tac acg cga gtg aca acc tac cgc gac 1432
   Leu Pro Asn Lys Pro Gly Val Tyr Thr Arg Val Thr Thr Tyr Arg Asp
   395 400 405
   tgg att cgc cag caa gct gga gtc tag tgccacgaaa gtgctgtgaa gtttgtg 1486
   Trp Ile Arg Gln Gln Ala Gly Val
   410 415 417
   tgcaaatgtc ctgactcaga ttcaactgca aaagaaaccg gaatgtctat tt 1538
<210> 39
   <211> 417
   <212> PRT
   <213> Mesocricetus auratus
<220>
   <221> propeptide portion
   <222> (1) ... (185)
<220>
   <221> trypsin-like protein portion
   <222> (186)... (417)
<400> 39
   Met Tyr Arg Pro Arg Pro Val Ile Pro Gln Ser Arg Phe Phe Ser Pro
   1 5 10 15
   Phe Val Val Ala Phe Val Val Ile Thr Thr Val Leu Ile Leu Ala Met
   20 25 30
   Thr Ile Gly Leu Leu Ile His Phe Leu Ala Phe Asp Lys Lys Thr Tyr
   35 40 45
   Phe Tyr His Ser Ser Phe Gln Ile Leu Asn Val Glu Tyr Thr Glu Ala
   50 55 60
   Leu Asn Ser Pro Ala Thr Gln Glu Tyr Arg His Leu Ser Glu Arg Ile
   65 70 75 80
   Glu Ser Met Ile Thr Asp Ala Phe Arg Glu Ser Asn Leu Arg Ser Glu
   85 90 95
   Phe Ile Arg Thr His Val Val Lys Leu Arg Lys Glu Gly Asn Gly Val
   100 105 110
   Ile Ala Asp Ala Val Met Lys Phe Arg Ser Ser Lys Arg Ser Asn Arg
   115 120 125
   Lys Ser Met Lys Asn Arg Ile His Ser Val Leu Gln Ile Leu Ser Asn
   130 135 140
   Ser Gly Ser Leu Glu Ile Thr Pro Ser Asn Glu Ile Thr Ser Leu Thr
   145 150 155 160
   Asp Gln Asp Thr Glu Asn Phe Leu Thr Gln Glu Cys Gly Ala Arg Pro
   165 170 175
   Asp Leu Ile Thr Leu Ser Glu Glu Arg Val Ile Gly Gly Thr Leu Ala
   180 185 190
   Glu Thr Gly Asp Trp Pro Trp Gln Val Ser Leu Gln Leu Asn Asn Val
   195 200 205
   His His Cys Gly Gly Ile Leu Ile Ser Asn Leu Trp Val Leu Thr Ala
   210 215 220
   Ala His Cys Phe Arg Ser Tyr Ser Asn Pro Leu Gln Trp Thr Ala Thr
   225 230 235 240
   Phe Gly Val Ser Thr Ile Arg Pro Ile Leu Arg Val Arg Val Arg Ser
   245 250 255
   Ile Val Ser His Asn Asn Tyr Arg Pro Thr Thr Arg Asp Asn Asp Ile
   260 265 270
   Ala Val Val Gln Leu Glu Arg Pro Ile Thr Phe Asn Arg Asn Ile His
   275 280 285
   Arg Val Cys Leu Pro Ala Ala Thr Gln Ser Ile Ile Pro Gly Ser Ile
   290 295 300
   Ala Tyr Val Thr Gly Trp Gly Ser Leu Thr Tyr Gly Gly Asn Thr Val
   305 310 315 320
   Thr Asp Leu Arg Gln Gly Gln Val Arg Ile Val Ser Thr Asp Glu Cys
   325 330 335
   Asn Glu Pro Ala Gly Tyr Ser Gly Ser Val Leu Pro Gly Met Leu Cys
   340 345 350
   Ala Gly Val Pro Ser Gly Ala Val Asp Ala Cys Gln Gly Asp Ser Gly
   355 360 365
   Gly Pro Leu Val Gln Glu Asp Ser Arg Met Leu Trp Phe Val Val Gly
   370 375 380
   Ile Val Ser Trp Gly Tyr Gln Cys Gly Leu Pro Asn Lys Pro Gly Val
   385 390 395 400
   Tyr Thr Arg Val Thr Thr Tyr Arg Asp Trp Ile Arg Gln Gln Ala Gly
   405 410 415
   Val
   417
<210> 40
   <211> 21
   <212> DNA
   <213> Artificial Sequence
<220>
   <223> PCR primer
<400> 40
   ctggccstgg caagtcagtc t 21
<210> 41
   <211> 29
   <212> DNA
   <213> Artificial Sequence
<220>
   <223> PCR primer
<400> 41
   gtggrtcctg acwgcmgcyc aytgcttca 29
<210> 42
   <211> 27
   <212> DNA
   <213> Artificial Sequence
<220>
   <223> PCR primer
<400> 42
   tgwgtcttcy tgkactagyg ggccacc 27
<210> 43
   <211> 29
   <212> DNA
   <213> Artificial Sequence
<220>
   <223> PCR primer
<400> 43
   caytgatmkc cccagctyac watbcccac 29
<210> 44
   <211> 1998
   <212> DNA
   <213> Artificial Sequence
<220>
   <223> fused DNA for human EGF and human placental alkalyne phosphatase
<400> 44
   atg aag ctg ctg ccg tcg gtg gtg ctg aag ctc ttt ctg gct gca gtt 48
   ctc tcg gca ctg gtg act ggc gag agc ctg gag cgg ctt cgg aga ggg 96
   cta gct gct gga acc agc aac ccg gac cct ccc act gta tcc acg gac 144
   cag ctg cta ccc cta gga ggc ggc cgg gac cgg aaa gtc cgt gac ttg 192
   caa gag gca gat ctg gac ctt ttg aga gtc act tta tcc tcc aag cca 240
   caa gca ctg gcc aca cca aac aag gag gag cac ggg aaa aga aag aag 288
   aaa ggc aag ggg cta ggg aag aag agg gac cca tgt ctt cgg aaa tac 336
   aag gac ttc tgc atc cat gga gaa tgc aaa tat gtg aag gag ctc cgg 384
   gct ccc tcc tgc atc tgc cac ccg ggt tac cat gga gag agg tgt cat 432
   ggg ctg agc ctc cca gtg gaa aat cgc tta tat acc tat gac cac aca 480
   acc atc ctg atc atc cca gtt gag gag gag aac ccg gac ttc tgg aac 528
   cgc gag gca gcc gag gcc ctg ggt gcc gcc aag aag ctg cag cct gca 576
   cag aca gcc gcc aag aac ctc atc atc ttc ctg ggc gat ggg atg ggg 624
   gtg tct acg gtg aca gct gcc agg atc cta aaa ggg cag aag aag gac 672
   aaa ctg ggg cct gag ata ccc ctg gcc atg gac cgc ttc cca tat gtg 720
   gct ctg tcc aag aca tac aat gta gac aaa cat gtg cca gac agt gga 768
   gcc aca gcc acg gcc tac ctg tgc ggg gtc aag ggc aac ttc cag acc 816
   att ggc ttg agt gca gcc gcc cgc ttt aac cag tgc aac acg aca cgc 864
   ggc aac gag gtc atc tcc gtg atg aat cgg gcc aag aaa gca ggg aag 912
   tca gtg gga gtg gta acc acc aca cga gtg cag cac gcc tcg cca gcc 960
   ggc acc tac gcc cac acg gtg aac cgc aac tgg tac tcg gac gcc gac 1008
   gtg cct gcc tcg gcc cgc cag gag ggg tgc cag gac atc gct acg cag 1056
   ctc atc tcc aac atg gac att gac gtg atc cta ggt gga ggc cga aag 1104
   tac atg ttt cgc atg gga acc cca gac cct gag tac cca gat gac tac 1152
   agc caa ggt ggg acc agg ctg gac ggg aag aat ctg gtg cag gaa tgg 1200
   ctg gcg aag cgc cag ggt gcc cgg tat gtg tgg aac cgc act gag ctc 1248
   atg cag gct tcc ctg gac ccg tct gtg acc cat ctc atg ggt ctc ttt 1296
   gag cct gga gac atg aaa tac gag atc cac cga gac tcc aca ctg gac 1344
   ccc tcc ctg atg gag atg aca gag gct gcc ctg cgc ctg ctg agc agg 1392
   aac ccc cgc ggc ttc ttc ctc ttc gtg gag ggt ggt cgc atc gac cat 1440
   ggt cat cat gaa agc agg gct tac cgg gca ctg act gag acg atc atg 1488
   ttc gac gac gcc att gag agg gcg ggc cag ctc acc agc gag gag gac 1536
   acg ctg agc ctc gtc act gcc gac cac tcc cac gtc ttc tcc ttc gga 1584
   ggc tac ccc ctg cga ggg agc tcc atc ttc ggg ctg gcc cct ggc aag 1632
   gcc cgg gac agg aag gcc tac acg gtc ctc cta tac gga aac ggt cca 1680
   ggc tat gtg ctc aag gac ggc gcc cgg ccg gat gtt acc gag agc gag 1728
   agc ggg agc ccc gag tat cgg cag cag tca gca gtg ccc ctg gac gaa 1776
   gag acc cac gca ggc gag gac gtg gcg gtg ttc gcg cgc ggc ccg cag 1824
   gcg cac ctg gtt cac ggc gtg cag gag cag acc ttc ata gcg cac gtc 1872
   atg gcc ttc gcc gcc tgc ctg gag ccc tac acc gcc tgc gac ctg gcg 1920
   ccc ccc gcc ggc acc acc gac gcc gcg cac ccg ggt tac tct aga gtc 1968
   ggg gcg gcc ggc cgc ttc gag cag aca tga 1998